(19) Europäisches Patentamt
European Patent Office
Office européen des brevets

(11) Veröffentlichungsnummer: **0 236 734**
**A2**

# (12) EUROPÄISCHE PATENTANMELDUNG

(21) Anmeldenummer: **87101502.0**

(22) Anmeldetag: **04.02.87**

(51) Int. Cl.³: **C 07 D 233/64**
C 07 D 417/12, C 07 D 401/12
C 07 D 233/84, C 07 C 147/107
A 61 K 31/415, A 61 K 31/425
A 61 K 31/44, A 61 K 31/165

(30) Priorität: **07.02.86 CH 486/86**
**20.08.86 CH 3347/86**

(43) Veröffentlichungstag der Anmeldung:
**16.09.87 Patentblatt 87/38**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Bühlmayer, Peter, Dr.**
**Im Baumgarten 3**
**CH-4144 Arlesheim(CH)**

(72) Erfinder: **Stanton, James Lawrence, Dr.**
**2 Fawn Ridge Road**
**Lebanon New Jersey 08833(US)**

(72) Erfinder: **Fuhrer, Walter, Dr.**
**Im Budler 3**
**CH-4419 Lupsingen(CH)**

(72) Erfinder: **Göschke, Richard, Dr.**
**Felixhäglistrasse 21**
**CH-4103 Bottmingen(CH)**

(72) Erfinder: **Rasetti, Vittorio, Dr.**
**Passwangstrasse 6**
**CH-4059 Basel(CH)**

(72) Erfinder: **Rüeger, Heinrich, Dr.**
**Rohrhagstrasse 18**
**CH-4104 Oberwil(CH)**

(74) Vertreter: **Zumstein, Fritz, Dr. et al,**
**Bräuhausstrasse 4**
**D-8000 München 2(DE)**

## (54) Durch schwefelhaltigen Gruppen substituierte 5-Amino-4-hydroxyvalerylderivate.

(57) Verbindungen der Formel

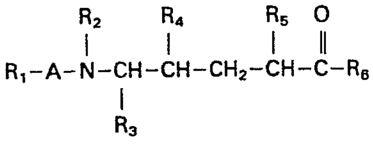

(I),

worin $R_1$ durch eine Thio-, Sulfinyl- oder Sulfonylgruppe substituiertes Acyl, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, gegebenenfalls veräthertes oder verestertes Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, veräthertes oder verestertes Hydroxy, $R_5$ Niederalkyl, gegebenenfalls veräthertes oder verestertes Hydroxyniederalkyl, Cycloalkyl, Cycloalkylniederalkyl, Bicycloalkyl, Bicycloalkylniederalkyl, Tricycloalkyl, Tricycloalkylniederalkyl, Aryl, Arylniederalkyl, gegebenenfalls substituiertes Carbamoyl, gegebenenfalls substituiertes Amino, gegebenenfalls substituiertes Hydroxy oder gegebenenfalls substituiertes Mercapto, Sulfinyl oder Sulfonyl und $R_6$ substituiertes Amino darstellen, und Salze von solchen Verbindungen mit salzbildenden Gruppen hemmen die blutdrucksteigernde Wirkung des Enzyms Renin und können als Antihypertensiva verwendet werden.

EP 0 236 734 A2

0236734

CIBA-GEIGY AG
Basel (Schweiz)

4-15746/1+2/+

# Durch schwefelhaltige Gruppen substituierte 5-Amino-4-hydroxyvalerylderivate

Die Erfindung betrifft Verbindungen der Formel

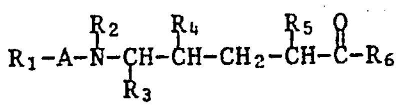

(I),

worin $R_1$ durch eine Thio-, Sulfinyl- oder Sulfonylgruppe substituiertes Acyl mit Ausnahme eines gegebenenfalls N-substituierten Acylrestes einer natürlichen Aminosäure, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, gegebenenfalls veräthertes oder verestertes Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, veräthertes oder verestertes Hydroxy, $R_5$ Niederalkyl, gegebenenfalls veräthertes oder verestertes Hydroxyniederalkyl, Cycloalkyl, Cycloalkylniederalkyl, Bicycloalkyl, Bicycloalkylniederalkyl, Tricycloalkyl, Tricycloalkylniederalkyl, Aryl, Arylniederalkyl, gegebenenfalls substituiertes Carbamoyl, gegebenenfalls substituiertes Amino, gegebenenfalls substituiertes Hydroxy oder gegebenenfalls substituiertes Mercapto, Sulfinyl oder Sulfonyl und $R_6$ substituiertes Amino mit Ausnahme eines von einer α-Aminosäure abgeleiteten Aminorestes darstellen, ferner Salze von solchen Verbindungen mit salzbildenden Gruppen, Verfahren zu ihrer Herstellung, pharmazeutische Präparate mit diesen Verbindungen und

die Verwendung dieser Verbindungen als Arzneimittel oder zur Herstellung von pharmazeutischen Präparaten, sowie Zwischenprodukte zur Herstellung von Verbindungen der Formel I.

In der Beschreibung der vorliegenden Erfindung bedeutet der bei der Definition von Gruppen oder Resten, z.B. Niederalkyl, Niederalkoxy, Niederalkanoyl etc., verwendete Ausdruck "Nieder", dass die so definierten Gruppen oder Reste, falls nicht ausdrücklich anders definiert, bis einschliesslich 7 und bevorzugt bis einschliesslich 4 C-Atome enthalten.

Die durch $R_3$, $R_4$ und $R_5$ substituierten C-Atome können die R-, S- oder R,S-Konfiguration haben. Bevorzugt sind Verbindungen der Formel I, worin die durch $R_3$ und $R_4$ substituierten C-Atome die S-Konfiguration aufweisen.

Die in der Beschreibung der vorliegenden Erfindung verwendeten allgemeinen Ausdrücke und Bezeichnungen haben vorzugsweise die folgenden Bedeutungen:

Acyl $R_1$ hat z.B. bis zu 25 C-Atome und ist in erster Linie eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin.

Bevorzugte Substituenten $R_1$ sind Acylgruppen der Formel

$$R^a - \underset{(O)_m}{\underset{|}{S}} - (CH_2)_n - \underset{\underset{R^b}{|}}{\underset{(CH_2)_q}{\underset{|}{CH}}} - (CH_2)_p - \underset{O}{\underset{\|}{C}} - \qquad \text{(Ia),}$$

worin $R^a$ unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, unsubstituiertes oder substituiertes Hydroxy oder unsubstituiertes oder substituiertes Amino, $R^b$ Wasserstoff, Mono-, Bi- oder Tricycloalkyl, unsubstituiertes oder substituiertes Aryl oder unsubstituiertes oder substituiertes Heteroaryl, m 0, 1 oder 2, n 0, 1 oder 2, p 0, 1 oder 2 und q 0, 1, 2, 3 oder 4 darstellt.

Das Methin-Kohlenstoffatom in der Teilformel Ia und, falls m 1 ist, auch das Schwefelatom können in der R-, S- oder R,S-Konfiguration vorliegen.

Niederalkyl $R^a$ hat vorzugsweise 1 - 7 C-Atome und ist z.B. Methyl, Aethyl, n-Propyl, Isopropyl, n-Butyl oder tert-Butyl, welche durch eine oder mehrere funktionelle Gruppen, beispielsweise Hydroxy, veräthertes Hydroxy, z.B. Niederalkoxy, wie Methoxy oder Aethoxy, oder Phenyloxy, verestertes Hydroxy, z.B. Niederalkanoyloxy, wie Acetoxy, Halogen, z.B. Chlor oder Brom, Hydroxysulfonyloxy, Carboxy, verestertes Carboxy, z.B. Niederalkoxycarbonyl, wie Methoxy- oder Aethoxycarbonyl, amidiertes Carboxy, z.B. Carbamoyl oder Mono- oder Diniederalkylcarbamoyl, wie Methyl- oder Dimethylcarbamoyl, Cyano, Amino, substituiertes Amino, z.B. Mononiederalkylamino, Diniederalkylamino, Acylamino oder substituiertes Amino, worin die Aminogruppe Teil eines fünf- oder sechsgliedrigen Heterocyclus enthaltend ein bis zwei Stickstoffatome und gewünschtenfalls ein Sauerstoff- oder Schwefelatom ist, oder durch Oxo substituiert sein können.

Substituiertes Niederalkyl $R^a$ ist beispielsweise Hydroxyniederalkyl, z.B. 2-Hydroxyäthyl, Niederalkoxyniederalkyl, z.B. Niederalkoxyäthyl, wie 2-Methoxyäthyl, Phenoxyniederalkyl, z.B. 2-Phenoxyäthyl, Niederalkanoyloxyniederalkyl, z.B. Niederalkanoyloxyäthyl, wie 2-Acetoxyäthyl, Halogenniederalkyl, z.B. Halogenäthyl, wie 2-Chlor- oder 2-Bromäthyl, Hydroxysulfonyloxyniederalkyl, z.B. 2-Hydroxysulfonyloxyäthyl, Carboxyniederalkyl, z.B. Carboxymethyl oder 2-Carboxyäthyl, Niederalkoxycarbonylniederalkyl, z.B. Niederalkoxycarbonylmethyl oder Niederalkoxycarbonyläthyl, wie Methoxycarbonylmethyl, 2-Methoxycarbonyläthyl, Aethoxycarbonylmethyl oder 2-Aethoxycarbonyläthyl, Carbamoylniederalkyl, z.B. Carbamoylmethyl oder 2-Carbamoyläthyl, Niederalkylcarbamoylniederalkyl, z.B. Methylcarbamoylmethyl, Diniederalkylcarbamoylniederalkyl, z.B. Dimethylcarbamoylmethyl, Cyanoniederalkyl, z.B. 2-Cyanoäthyl, Aminoniederalkyl, z.B. 2-Aminoäthyl, Niederalkylaminoniederalkyl, z.B. 2-Methylaminoäthyl, Diniederalkylaminoniederalkyl, z.B. 2-Dimethylaminoäthyl, Morpholinoniederalkyl, z.B. 2-Morpholinoäthyl, Piperidinoniederalkyl, z.B. 2-Piperidinoäthyl, Acylaminoniederalkyl, z.B. Niederalkanoylaminoniederalkyl, wie 2-Acetylaminoäthyl, Benzyloxycarbonylaminoniederalkyl, wie 2-Benzyloxycarbonylaminoäthyl, Niederalkoxycarbonylaminoniederalkyl, wie 2-tert-Butoxycarbonylaminoäthyl, oder Oxoniederalkyl, z.B. 2-Oxopropyl oder 2-Oxobutyl.

Niederalkenyl $R^a$ enthält z.B. 2 - 7, insbesondere 2 - 4, C-Atome und ist z.B. Vinyl, Allyl oder 2- oder 3-Butenyl. Niederalkenyl $R^a$ kann durch die gleichen Substituenten substituiert sein wie Niederalkyl, z.B. durch Hydroxy, veräthertes Hydroxy, z.B. Methoxy, verestertes Hydroxy, z.B. Acetoxy, Halogen, z.B. Chlor oder Brom, Carboxy, verestertes Carboxy, z.B. Methoxycarbonyl oder Aethoxycarbonyl, oder amidiertes Carboxy, z.B. Carbamoyl.

Niederalkinyl $R^a$ enthält z.B. 2 - 7, insbesondere 2 - 4, C-Atome und ist beispielsweise Aethinyl, 1-Propinyl oder 2-Propinyl.

Cycloalkyl $R^a$ oder $R^b$ enthält z.B. 3 - 8, insbesondere 3 - 6, C-Atome und ist z.B. Cyclopropyl, Cyclobutyl, Cyclopentyl oder Cyclohexyl.

Bicycloalkyl $R^a$ oder $R^b$ enthält z.B. 5 - 10, insbesondere 6 - 9, C-Atome und ist z.B. Bicyclohexyl, -heptyl, -octyl, -nonyl oder -decyl, z.B. Bicyclo[3.1.0]hex-1-, -2- oder -3-yl, Bicyclo[4.1.0]-hept-1- oder -7-yl, Bicyclo[2.2.1]hept-2-yl, z.B. endo- oder exo-Norbornyl, Bicyclo[3.2.1]oct-2-yl, Bicyclo[3.3.0]oct-3-yl oder Bicyclo[3.3.1]non-9-yl, ferner α- oder β-Decahydronaphthyl.

Tricycloalkyl $R^a$ oder $R^b$ enthält z.B. 8 - 10 C-Atome und ist z.B. Tricyclo[5.2.1.$0^{2,6}$]dec-8-yl oder Adamantyl, wie 1-Adamantyl.

Cycloalkylniederalkyl $R^a$ enthält z.B. 4 - 10, insbesondere 4 - 7, C-Atome und ist beispielsweise Cyclopropylmethyl, Cyclobutylmethyl, Cyclopentylmethyl oder Cyclohexylmethyl.

Die genannten cycloaliphatischen oder cycloaliphatisch-aliphatischen Reste können durch die gleichen Substituenten wie Niederalkyl $R^a$ substituiert sein.

Aryl $R^a$ oder $R^b$ enthält z.B. 6 bis 14 C-Atome und ist beispielsweise Phenyl, Indenyl, z.B. 2- oder 4-Indenyl, 1- oder 2-Naphthyl, Anthryl, z.B. 1- oder 2-Anthryl, Phenanthryl, z.B. 9-Phenanthryl, oder Acenaphthenyl, z.B. 1-Acenaphthenyl. Aryl $R^a$ oder $R^b$ ist beispielsweise durch Niederalkyl, z.B. Methyl, Hydroxy, Niederalkoxy, z.B. Methoxy, Acyloxy, z.B. Niederalkanoyloxy, wie Acetoxy, Amino, Niederalkylamino, z.B. Methylamino, Diniederalkylamino, z.B. Dimethylamino, Acylamino, z.B. tert-Butoxycarbonylamino, oder Halo, z.B. Chlor, Brom oder Iod, substituiert, wobei der Substituent in irgendeiner Stellung des Arylrestes, z.B. in o-, m- oder p-Stellung des Phenylrestes, stehen kann und der Arylrest auch mehrfach mit gleichen oder verschiedenen Substituenten substituiert sein kann.

Arylniederalkyl $R^a$ hat z.B. 7 bis 15 C-Atome und enthält beispielsweise einen unter Niederalkyl $R^a$ genannten unsubstituierten oder substituierten, gegebenenfalls verzweigten Rest und einen unter Aryl $R^a$ oder $R^b$ genannten unsubstituierten oder substituierten Rest. Ein solches Arylniederalkyl ist beispielsweise Benzyl, Niederalkylbenzyl, wie 4-Methylbenzyl, Niederalkoxybenzyl, wie 4-Methoxybenzyl, 2-Phenyläthyl, 2-(p-Hydroxyphenyl)-äthyl, Diphenylmethyl, Di-(4-methoxyphenyl)-methyl, Trityl oder α- oder β-Naphthylmethyl.

Arylniederalkenyl $R^a$ hat z.B. 8 bis 16 C-Atome und enthält beispielsweise einen unter Niederalkenyl $R^a$ genannten unsubstituierten oder substituierten Rest und einen unter Aryl $R^a$ oder $R^b$ genannten unsubstituierten oder substituierten Rest. Ein solches Arylniederalkenyl ist beispielsweise Styryl, 3-Phenylallyl, 2-(α-Naphthyl)-vinyl oder 2-(β-Naphthyl)-vinyl.

Unsubstituiertes oder substituiertes Heteroaryl $R^a$ oder $R^b$ ist mono-, bi- oder tricyclisch und enthält ein bis zwei Stickstoffatome und/oder ein Sauerstoff- oder Schwefelatom. $R^a$ oder $R^b$ ist beispielsweise Pyrrolyl, Furyl, Thienyl, Imidazolyl, Pyrazolyl, Oxazolyl, Thiazolyl, Pyridyl, Pyrazinyl, Pyrimidinyl, Indolyl, Chinolyl, Isochinolyl, Chinoxalinyl, β-Carbolinyl oder ein benz-annelliertes, cyclopenta-, cyclohexa- oder cyclohepta-annelliertes Derivat dieser Reste. Dieser Heterocyclus kann an einem Stickstoffatom durch Oxido, Niederalkyl, z.B. Methyl oder Aethyl, Phenyl oder Phenylniederalkyl, z.B. Benzyl, und/oder an einem oder mehreren Kohlenstoff-Atomen durch Niederalkyl, z.B. Methyl, Phenyl, Phenylniederalkyl, z.B. Benzyl, Halogen, z.B. Chlor, Hydroxy, Niederalkoxy, z.B. Methoxy, Phenylniederalkoxy, z.B. Benzyloxy, oder Oxo substituiert und teilweise gesättigt sein und ist beispielsweise 2-oder 3-Pyrrolyl, Phenyl-pyrrolyl, z.B. 4- oder 5-Phenyl-2-pyrrolyl, 2-Furyl, 2-Thienyl, 4-Imidazolyl, Methyl-imidazolyl, z.B. 1-Methyl-2-, 4- oder 5-imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 5-Indolyl, substituiertes 2-Indolyl, z.B. 1-Methyl-, 5-Methyl-, 5-Methoxy-, 5-Benzyloxy-, 5-Chlor- oder 4,5-Dimethyl-2-indolyl, 1-Benzyl-2- oder 3-indolyl, 4,5,6,7-Tetrahydro-2-indolyl, Cyclohepta[b]-5-pyrrolyl, 2-, 3- oder 4-Chinolyl, 4-Hydroxy-2-chinolyl, 1-, 3- oder 4-Isochinolyl, 1-Oxo-1,2-dihydro-3-isochinolyl, 2-Chinoxalinyl, 2-Benzofuranyl, 2-Benzoxazolyl, 2-Benzthiazolyl, Benz[e]indol-2-yl oder β-Carbolin-3-yl.

Heteroarylniederalkyl $R^a$ enthält z.B. einen unter Niederalkyl $R^a$ genannten unsubstituierten oder substituierten Rest und einen unter Heteroaryl $R^a$ oder $R^b$ genannten unsubstituierten oder substituierten Rest und ist beispielsweise 2- oder 3-Pyrrolylmethyl, 2-, 3- oder 4-Pyridylmethyl, 2-(2-, 3- oder 4-Pyridyl)-äthyl, 4-Imidazolylmethyl, 2-(4-Imidazolyl)-äthyl, 2- oder 3-Indolylmethyl, 2-(3-Indolyl)-äthyl oder 2-Chinolylmethyl.

Hydroxy $R^a$ ist unsubstituiert oder beispielsweise durch Niederalkyl oder Aryl substituiert und ist z.B. Hydroxy, Methoxy, Aethoxy n-Butoxy, Phenoxy, 4-Hydroxyphenoxy oder 3,4-Methylendioxyphenoxy.

Amino $R^a$ ist unsubstituiert, durch eine oder zwei Niederalkylgruppen oder durch Arylniederalkyl, Niederalkanoyl, Niederalkoxycarbonyl oder Arylmethoxycarbonyl substituiert oder Teil eines fünf- oder sechsgliedrigen Heterocyclus enthaltend ein bis zwei Stickstoffatome und gewünschtenfalls ein Sauerstoff- oder Schwefelatom und ist z.B. Amino, Methylamino, Aethylamino, Isopropylamino, n-Butylamino, Dimethylamino, Diethylamino, Benzylamino, Acetylamino, Pivaloylamino, Methoxy-, Aethoxy- oder tert-Butoxycarbonylamino, Benzyloxycarbonylamino, 1-Pyrrolidinyl, 1-Piperidinyl, 1-Methyl-4-Pyridazinyl, 4-Morpholinyl oder 4-Thiomorpholinyl.

A ist ein bivalenter Rest einer α-Aminosäure, beispielsweise einer natürlichen α-Aminosäure mit der L-Konfiguration, wie sie normalerweise in Proteinen vorkommen, eines Homologen einer solchen Aminosäure, z.B. worin die Aminosäureseitenkette um eine oder zwei Methylengruppen verlängert oder verkürzt ist und/oder eine Methylgruppe durch Wasserstoff ersetzt ist, einer substituierten aromatischen α-Aminosäure, z.B. eines substituierten Phenylalanins oder Phenylglycins, worin der Substituent Niederalkyl, z.B. Methyl, Halogen, z.B. Fluor, Chlor, Brom oder Jod, Hydroxy, Niederalkoxy, z.B. Methoxy, Niederalkanoyloxy, z.B. Acetoxy, Amino, Niederalkylamino, z.B. Methylamino, Diniederalkylamino, z.B. Dimethylamino, Niederalkanoylamino, z.B. Acetylamino oder Pivaloylamino, Niederalkoxycarbonylamino, z.B. tert-Butoxycarbonylamino, Arylmethoxycarbonylamino, z.B. Benzyloxycarbonylamino, und/oder Nitro sein kann und ein- oder mehrfach vorkommt, eines benzannellierten Phenylalanins oder Phenylglycins, wie α-Naphthylalanins, oder eines hydrierten Phenylalanins oder Phenylglycins, wie Cyclohexylalanins oder Cyclohexylglycins, einer fünf- oder sechs-gliedrigen cyclischen, benzannellierten α-Aminosäure, z.B. Indolin-2-carbonsäure oder 1,2,3,4-Tetrahydroisochinolin-3-carbonsäure, einer natürlichen oder homologen α-Aminosäure, in der eine Carboxygruppe der Seitenkette in veresterter oder amidierter Form vorliegt, z.B. als Niederalkylestergruppe, wie Methoxycarbonyl oder tert-Butoxycarbonyl, oder als Carbamoyl-, als Niederalkylcarbamoyl-, wie Methylcarbamoyl, oder als Diniederalkylcarbamoylgruppe, wie Dimethylcarbamoyl, in der eine Aminogruppe der Seitenkette in acylierter Form vorliegt, z.B. als Niederalkanoylamino-, wie Acetylamino oder Pivaloylamino, als Niederalkoxycarbonylamino-, wie tert-Butoxycarbonylamino, oder als Arylmethoxycarbonylaminogruppe, wie Benzyloxycarbonylamino, oder in der eine Hydroxygruppe der Seitenkette in veräthertet oder veresterter Form vorliegt, z.B. als Niederalkoxygruppe, wie Methoxy, als Arylniederalkoxygruppe, wie Benzyloxy, oder als Niederalkanoyloxygruppe, wie Acetoxy, oder eines Epimeren einer solchen Aminosäure, d.h. mit der unnatürlichen D-Konfiguration.

Solche Aminosäuren sind beispielsweise Glycin (H-Gly-OH), Alanin (H-Ala-OH), Valin (H-Val-OH), Norvalin (α-Aminovaleriansäure), Leucin, (H-Leu-OH), Isoleucin (H-Ile-OH), Norleucin (α-Aminohexansäure, H-Nle-OH), Serin (H-Ser-OH), Homoserin (α-Amino-γ-hydroxybuttersäure), Threonin (H-Thr-OH), Methionin (H-Met-OH), Cystein (H-Cys-OH), Prolin (H-Pro-OH), trans-3- und trans-4-Hydroxyprolin, Phenylalanin (H-Phe-OH), Tyrosin (H-Tyr-OH), 4-Nitrophenylalanin, 4-Aminophenylalanin, 4-Chlorphenylalanin, β-Phenylserin (β-Hydroxyphenylalanin), Phenylglycin, α-Naphthylalanin, Cyclohexylalanin (H-Cha-OH), Cyclohexylglycin, Tryptophan (H-Trp-OH), Indolin-2-carbonsäure, 1,2,3,4-Tetrahydroisochinolin-3-carbonsäure, Asparaginsäure (H-Asp-OH), Asparagin (H-Asn-OH), Aminomalonsäure, Aminomalonsäure-monoamid, Glutaminsäure (H-Glu-OH), Glutaminsäure-monotert-butylester, Glutamin (H-Gln-OH), $N^{\delta}$-Dimethylglutamin, Histidin (H-His-OH), Arginin (H-Arg-OH), Lysin (H-Lys-OH), $N^{\varepsilon}$-tert-Butoxycarbonyl-lysin, δ-Hydroxylysin, Ornithin (α,δ-Diaminovaleriansäure), $N^{\delta}$-Pivaloyl-ornithin, α,γ-Diaminobuttersäure oder α,ß-Diamino-propionsäure.

Der Aminosäurerest A kann N-terminal zur Erhöhung der Stabilität der Verbindung der Formel I gegen enzymatischen Abbau durch Niederalkyl, z.B. Methyl oder Aethyl, substituiert sein.

A ist vorzugsweise der bivalente Rest von Alanin, Valin, Norvalin, Leucin, Norleucin, Serin, veräthertem Serin, Prolin, Phenylalanin, ß-Phenylserin, α-Naphthylalanin, Cyclohexylalanin, Indolin-2-carbonsäure, 1,2,3,4-Tetrahydroisochinolin-3-carbonsäure, Asparaginsäure, veresterter Asparaginsäure, Asparagin, Aminomalonsäure, Aminomalonsäure-monoamid, Glutaminsäure, veresterter Glutaminsäure, Glutamin, Diniederalkyl-glutamin, Histidin, Lysin, acyliertem Lysin, Ornithin oder acyliertem Ornithin, N-terminal gewünschtenfalls durch Niederalkyl, z.B. Methyl, substituiert. Ganz besonders bevorzugt als Gruppe A ist der bivalente Rest von Histidin, ferner auch von Serin und Alanin.

Niederalkyl $R_2$ oder $R_3$ hat die weiter vorn für Niederalkyl $R^a$ genannten Bedeutungen. Niederalkyl $R_2$ ist vorzugsweise Methyl oder Aethyl. Niederalkyl $R_3$ ist vorzugsweise Isopropyl, Isobutyl, tert-Butyl, Isopentyl, 2-Methylbutyl oder 2-Aethylbutyl.

Hydroxyniederalkyl $R_3$ oder $R_5$ ist vorzugsweise Hydroxymethyl oder Hydroxyäthyl und ist gegebenenfalls durch eine der weiter unten für veräthertes oder verestertes Hydroxy $R_4$ angegebenen Gruppen veräthert oder verestert.

Cycloalkyl $R_3$ oder $R_5$ hat die weiter vorn für Cycloalkyl $R^a$ oder $R^b$ genannten Bedeutungen und ist vorzugsweise Cyclopentyl oder Cyclohexyl.

Cycloalkylniederalkyl $R_3$ und $R_5$ hat die weiter vorn für Cycloalkylniederalkyl $R^a$ genannten Bedeutungen und ist vorzugsweise Cyclohexylmethyl.

Bicycloalkylniederalkyl $R_3$ oder $R_5$ enthält z.B. 6 - 14, insbesondere 7 - 12, C-Atome und ist beispielsweise durch die weiter vorn für Bicycloalkyl $R^a$ oder $R^b$ genannten Reste substituiertes Methyl oder Aethyl, z.B. Bicyclo[2.2.1]hept-2-ylmethyl.

Tricycloalkylniederalkyl $R_3$ oder $R_5$ enthält z.B. 9 - 14, insbesondere 10 - 12, C-Atome und ist beispielsweise durch die weiter vorn für Tricycloalkyl $R^a$ oder $R^b$ genannten Reste substituiertes Methyl oder Aethyl, vorzugsweise 1-Adamantylmethyl.

Aryl $R_3$ oder $R_5$ hat die weiter vorn für Aryl $R^a$ oder $R^b$ genannten Bedeutungen und ist vorzugsweise Phenyl.

Arylniederalkyl $R_3$ oder $R_5$ hat die weiter vorn für Arylniederalkyl $R^a$ genannten Bedeutungen und ist vorzugsweise Benzyl.

Eine verätherte Hydroxygruppe $R_4$ ist vorzugsweise durch solche organischen Reste veräthert, die unter physiologischen Bedingungen abspaltbar sind und nach der Abspaltung in der betreffenden Konzentration pharmakologisch unbedenkliche Spaltprodukte liefern.

Veräthertes Hydroxy $R_4$ ist z.B. Acyloxyniederalkoxy, worin Acyl die Acylgruppe einer gegebenenfalls verzweigten Niederalkancarbonsäure oder der durch gegebenenfalls verzweigtes Niederalkyl monoveresterten Kohlensäure darstellt, z.B. Niederalkanoyloxyniederalkoxy, wie Acetoxymethoxy, 1-Acetoxyäthoxy, Pivaloyloxymethoxy oder 1-Pivaloyloxyäthoxy, oder Niederalkoxycarbonyloxyniederalkoxy, wie Aethoxycarbonyloxymethoxy, 1-Aethoxycarbonyloxyäthoxy, tert-Butoxycarbonyloxymethoxy oder 1-tert-Butoxycarbonyloxyäthoxy.

Veräthertes Hydroxy $R_4$ ist ferner Niederalkoxy, z.B. Methoxy oder Aethoxy, Aryloxy, z.B. Phenoxy, oder Arylniederalkoxy, z.B. Benzyloxy.

Verestertes Hydroxy $R_4$ ist z.B. aliphatisches Acyloxy, z.B. Niederalkanoyloxy, wie Acetoxy oder Pivaloyloxy, cycloaliphatisches Acyloxy, z.B. Cycloalkylcarbonyloxy, wie Cyclohexylcarbonyloxy, oder aromatisches Acyloxy, z.B. Benzoyloxy.

Niederalkyl $R_5$ hat 1-7 C-Atome und ist z.B. Methyl, Aethyl, n-Propyl, Isopropyl, n-Butyl, Isobutyl, tert-Butyl oder Isopentyl. Besonders bevorzugt sind Methyl, Isopropyl, Isobutyl und tert-Butyl.

Bicycloalkyl $R_5$ hat die weiter vorn für Bicycloalkyl $R^a$ oder $R^b$ genannten Bedeutungen und ist vorzugsweise α-Decahydronaphthyl.

Tricycloalkyl $R_5$ hat die weiter vorn für Tricycloalkyl $R^a$ oder $R^b$ genannten Bedeutungen und ist vorzugsweise 1-Adamantyl.

Gegebenenfalls substituiertes Carbamoyl $R_5$ ist unsubstituiert oder durch eine oder zwei Niederalkyl- oder Hydroxyniederalkylgruppen substituiert und ist z.B. Carbamoyl, Methylcarbamoyl, Aethylcarbamoyl, n-Propylcarbamoyl, Isopropylcarbamoyl, n-Butylcarbamoyl, Dimethylcarbamoyl, 2-Hydroxyäthylcarbamoyl oder Di-(2-hydroxyäthyl)-carbamoyl.

Gegebenenfalls substituiertes Amino $R_5$ ist unsubstituiert, durch eine oder zwei Niederalkylgruppen oder durch Arylniederalkyl, Niederalkanoyl, Niederalkoxycarbonyl oder Arylmethoxycarbonyl substituiert oder Teil eines fünf- oder sechgliedrigen Heterocyclus enthaltend ein bis zwei Stickstoffatome und gewünschtenfalls ein Sauerstoff- oder Schwefelatom und ist z.B. Amino, Methylamino, Aethylamino, Isopropylamino, n-Butylamino, Dimethylamino, Diäthylamino, Benzylamino, Acetylamino, Pivaloylamino, Methoxy-, Aethoxy-

oder tert-Butoxycarbonylamino, Benzyloxycarbonylamino, 1-Pyrrolidinyl, 1-Piperidinyl, 1-Methyl-4-Pyridazinyl, 4-Morpholinyl oder 4-Thiomorpholinyl, vorzugsweise Dimethylamino.

Gegebenenfalls substituiertes Hydroxy $R_5$ ist unsubstituiert oder durch eine der weiter vorn für veräthertes oder verestertes Hydroxy $R_4$ genannten Gruppen veräthert oder verestert und ist z.B. Hydroxy, Methoxy, Aethoxy, Acetoxymethoxy, Phenoxy, Benzyloxy, Acetoxy, Pivaloyloxy oder Benzoyloxy.

Gegebenenfalls substituiertes Mercapto $R_5$ ist unsubstituiert oder durch Niederalkyl, z.B. Methyl oder Aethyl, Aryl, z.B. Phenyl, Arylniederalkyl, z.B. Benzyl, Niederalkanoyl, z.B. Acetyl, oder Arylcarbonyl, z.B. Benzoyl, substituiert und ist beispielsweise Mercapto, Methylthio, Aethylthio, Phenylthio, Benzylthio, Acetylthio oder Benzoylthio.

Sulfinyl $R_5$ trägt eine Niederalkylgruppe, z.B. Methyl oder Aethyl, eine Arylgruppe, z.B. Phenyl, oder eine Arylniederalkylgruppe, z.B. Benzyl, und ist beispielsweise Methylsulfinyl, Aethylsulfinyl, Phenylsulfinyl oder Benzylsulfinyl.

Sulfonyl $R_5$ trägt eine Niederalkylgruppe, z.B. Methyl oder Aethyl, eine Arylgruppe, z.B. Phenyl, oder eine Arylniederalkylgruppe, z.B. Benzyl, und ist beispielsweise Methylsulfonyl, Aethylsulfonyl, Phenylsulfonyl oder Benzylsulfonyl, bevorzugt Methylsulfonyl.

Substituiertes Amino $R_6$ ist beispielsweise eine Aminogruppe, welche durch einen oder gegebenenfalls zwei unsubstituierte oder substituierte, gesättigte oder ungesättigte, aliphatische Kohlenwasserstoffreste mit bis einschliesslich 18, bevorzugt bis einschliesslich 10, C-Atomen oder einen unsubstituierten oder substituierten, aromatischen, heteroaromatischen, aromatisch-aliphatischen oder heteroaromatisch-aliphatischen Kohlenwasserstoffrest mit bis zu 18, bevorzugt bis einschliesslich 10, C-Atomen substituiert ist.

Ausgeschlossen als substituiertes Amino $R_6$ ist der Rest einer α-Aminosäure oder deren N-substituierten, veresterten oder amidierten Derivate.

Ein unsubstituierter oder substituierter, gesättigter oder ungesättigter, aliphatischer Kohlenwasserstoffrest, welcher die Aminogruppe $R_6$ substituiert, ist beispielsweise gegebenenfalls substituiertes Alkyl mit bis zu 10 C-Atomen, Niederalkenyl oder Niederalkinyl mit bis einschliesslich 7 C-Atomen, oder Cycloalkylniederalkyl mit 4-10 C-Atomen. Diese Reste können wie Niederalkyl $R^a$ durch eine oder mehrere der weiter vorn genannten funktionellen Gruppen, sowie durch Sulfo, substituiert sein.

Als Substituenten sind Hydroxy, Niederalkoxy, z.B. Methoxy, Niederalkanoyloxy, z.B. Acetoxy, substituiertes oder unsubstituiertes Phenyloxy, z.B. Carbamoylphenyloxy oder Carbamoyl-hydroxy-phenyloxy, Carboxy, verestertes Carboxy, z.B. Niederalkoxycarbonyl, wie Methoxycarbonyl oder tert-Butoxycarbonyl, oder ein physiologisch spaltbares verestertes Carboxy, z.B. 1-(Niederalkanoyloxy)-niederalkoxycarbonyl, wie Acetoxymethoxycarbonyl, Pivaloyloxymethoxycarbonyl oder 1-Propionyloxyäthoxycarbonyl, 1-(Niederalkoxycarbonyloxy)-niederalkoxycarbonyl, wie 1-(Aethoxycarbonyloxy)-äthoxycarbonyl, oder α-Aminoniederalkanoyloxymethoxycarbonyl, wie α-Aminoacetoxymethoxycarbonyl oder (S)-α-Amino-ß-methylbutyryloxymethoxycarbonyl, Carbamoyl, substituiertes oder unsubstituiertes Niederalkylcarbamoyl, z.B. Hydroxyniederalkylcarbamoyl, wie 2-Hydroxyäthylcarbamoyl oder Tris-(hydroxymethyl)-methylcarbamoyl, Amino, Niederalkylamino, z.B. Methylamino, Diniederalkylamino, z.B. Dimethylamino, Niederalkoxycarbonylamino, z.B. tert-Butoxycarbonylamino, Guanidino, über ein Stickstoff-Atom gebundenes, gesättigtes fünf- oder sechsgliedriges, gewünschtenfalls durch Oxo substituiertes Heterocyclyl, z.B. 1-Piperidinyl, 4-Morpholinyl oder 2-Oxo-1-pyrrolidinyl, oder Sulfo bevorzugt.

Ein aromatischer oder aromatisch-aliphatischer Kohlenwasserstoffrest in einer Gruppe $R_6$ hat vorzugsweise die gleichen wie unter Aryl $R^a$ oder $R^b$ oder Arylniederalkyl $R^a$ genannten Bedeutungen und ist beispielsweise Phenyl oder Phenylniederalkyl.

Diese Reste können im Aromaten z.B. durch Niederalkyl, z.B. Methyl oder Aethyl, Hydroxy, veräthertes Hydroxy, z.B. Niederalkoxy, wie Methoxy oder tert-Butoxy, verestertes Hydroxy, z.B. Niederalkanoyloxy, wie Acetoxy, oder Halogen, z.B. Fluor oder Chlor, Carboxy, verestertes Carboxy, z.B. Niederalkoxycarbonyl, wie tert-Butoxycarbonyl, Carbamoyl, Amino, Niederalkylamino, z.B. Methylamino, Diniederalkylamino, z.B. Dimethylamino, acyliertes Amino, z.B. Niederalkoxycarbonylamino, wie tert-Butoxycarbonylamino, sowie durch Nitro substituiert sein.

Niederalkyl in einem Rest Phenylniederalkyl kann durch die gleichen Substituenten wie Alkyl in einem Rest $R_6$ substituiert sein.

Ein heteroaromatischer oder heteroaromatisch-aliphatischer Kohlenwasserstoffrest in einer Gruppe $R_6$ hat vorzugsweise die gleichen wie unter Heteroaryl $R^a$ und $R^b$ oder Heteroarylniederalkyl $R^a$ genannten Bedeutungen und ist beispielsweise Pyridylniederalkyl, z.B. 2-, 3- oder 4-Pyridylmethyl, Imidazolylniederalkyl, z.B. 2-(4-Imidazolyl)-äthyl oder auch 2-(2-[4-Imidazolyl]-äthylamino)-äthyl, oder Indolylniederalkyl, z.B. 3-Indolylmethyl oder 2-(3-Indolyl)-äthyl.

Substituiertes Amino $R_6$ ist vorzugsweise Alkylamino, z.B. Methyl-, Aethyl-, n-Propyl-, Isopropyl-, n-Butyl-, Isobutyl-, tert-Butyl-, n-Pentyl-, Isopentyl-, n-Hexyl-, n-Octyl- oder n-Decylamino, Diniederalkylamino, z.B. Dimethylamino oder Diäthylamino, Hydroxyniederalkylamino, z.B. 2-Hydroxyäthylamino, 1-Hydroxybut-2-ylamino, 5-Hydroxypentylamino oder Tris(hydroxymethyl)-methylamino, Di-(hydroxyniederalkyl)-amino, z.B. Di-(2-hydroxyäthyl)-amino, Niederalkoxyniederalkylamino, z.B. 2-Methoxyäthylamino, Niederalkanoyloxyniederalkylamino, z.B. 2-Acetoxyäthylamino, Phenoxyniederalkylamino oder Phenoxy-hydroxy-niederalkylamino, worin Phenoxy gegebenenfalls durch Niederalkyl, Niederalkoxy, Hydroxy, Carboxy, Niederalkoxycarbonyl oder Carbamoyl substituiert ist, z.B. 2-Phenoxyäthylamino, 2-(3-Carbamoyl-4-hydroxyphenoxy)-äthylamino oder 3-(3-Carbamoylphenoxy)-2-hydroxy-propylamino, Carboxyalkylamino oder Amino-carboxy-alkylamino, worin der Carboxyrest nicht in 1-Stellung des Alkylrests steht, z.B. 4-Carboxy-n-butylamino, 5-Carboxy-n-pentylamino, 5-Amino-5-carboxy-n-pentylamino, 6-Carboxy-n-hexylamino, 7-Carboxy-n-heptylamino oder 8-Carboxy-n-octylamino, ferner Dicarboxymethylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butylamino, 5-tert-Butoxycarbonylamino-5-methoxycarbonyl-n-pentylamino, 7-tert-Butoxycarbonyl-n-heptylamino oder 8-tert-Butoxycarbonyl-n-octylamino, ferner Di-niederalkoxycarbonyl-methylamino, z.B. Di-methoxycarbonyl-methylamino, physiologisch spaltbares verestertes Carboxyalkylamino, worin die Esterfunktion nicht in 1-Stellung des Alkylrests steht, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoylalkylamino oder Hydroxyniederalkylcarbamoylalkylamino, worin der Carbamoylrest nicht in 1-Stellung des Alkylrests steht, z.B. 4-Carbamoyl-n-butylamino, 7-Carbamoyl-n-heptylamino oder 4-(Tris[hydroxymethyl]-methyl)-carbamoyl-n-butylamino, ferner Dicarbamoyl-methylamino, Di-(niederalkylcarbamoyl)-methylamino, z.B. Di-(methylcarbamoyl)-methylamino, Di-(hydroxyniederalkylcarbamoyl)-methylamino, z.B. Di-(2-hydroxyäthylcarbamoyl)-methylamino, oder Bis-(diniederalkylcarbamoyl)-methylamino, z.B. Bis-(dimethylaminocarbamoyl)-methylamino, Aminoniederalkylamino, z.B. 2-Aminoäthylamino oder 4-Aminobutylamino, Niederalkylaminoniederalkylamino, z.B. 2-Methylaminoäthylamino, Diniederalkylaminoniederalkylamino, z.B. 2-Dimethylaminoäthylamino oder 3-Dimethylaminopropylamino, Niederalkoxycarbonylaminoniederalkylamino, z.B. 2-(tert-Butoxycarbonylamino)-äthylamino, Guanidinoniederalkylamino, z.B. 2-Guanidinoäthylamino, über ein Stickstoff-Atom gebundenes, gesättigtes fünf- oder sechsgliedriges Heterocyclylniederalkylamino, z.B. 2-(4-Morpholinyl)-äthylamino, 3-(4-Morpholinyl)-propylamino oder 3-(2-Oxo-1-pyrroli-

dinyl)-propylamino, Niederalkenylamino, z.B. Allylamino oder 2- oder 3-Butenylamino, Niederalkinylamino, z.B. Propargylamino, Cycloalkylniederalkylamino, z.B. Cyclopropylmethylamino oder Cyclohexylmethylamino, Phenylamino oder Phenylniederalkylamino, worin Phenyl gegebenenfalls durch Niederalkyl, z.B. Methyl, Hydroxy, Niederalkoxy, z.B. Methoxy oder tert-Butoxy, Niederalkanoyloxy, z.B. Acetoxy, Halogen, z.B. Fluor oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenylamino, 2-, 3- oder 4-Methylphenylamino, 4-Hydroxyphenylamino, 4-Methoxyphenylamino, 2,3-, 2,4- oder 2,5-Dimethoxyphenylamino, 4-Chlorphenylamino, 2-, 3- oder 4-Carboxyphenylamino, 2-, 3- oder 4-Methoxy- oder tert-Butoxy-carbonylphenylamino, 2-, 3- oder 4-Carbamoylphenylamino, 4-Aminophenylamino, 4-tert-Butoxycarbonylaminophenylamino oder 4-Nitrophenylamino, ferner z.B. Benzylamino, 4-Methylbenzylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenyläthylamino oder 3-Phenylpropylamino, Pyridylniederalkylamino, z.B. 2-, 3- oder 4-Pyridylmethylamino, 2-(2-, 3- oder 4-Pyridyl)-äthylamino oder 3-(2-, 3- oder 4-Pyridyl)-propylamino, Imidazolylniederalkylamino, z.B. 4-Imidazolylmethylamino, 2-(4-Imidazolyl)-äthylamino oder 2-(2-[4-Imidazolyl]-äthylamino)-äthylamino, Indolylniederalkylamino, z.B. 3-Indolylmethylamino oder 2-(3-Indolyl)-äthylamino, oder Sulfoniederalkylamino, z.B. 2-Sulfoäthylamino.

Salze sind in erster Linie die pharmazeutisch verwendbaren, nichttoxischen Salze von Verbindungen der Formel I.

Solche Salze werden beispielsweise von Verbindungen der Formel I mit einer sauren Gruppe, z.B. einer Carboxygruppe, gebildet und sind in erster Linie geeignete Alkalimetall-, z.B. Natrium- oder Kalium-, oder Erdalkalimetallsalze, z.B. Magnesium- oder Calciumsalze, ferner Zinksalze oder Ammoniumsalze, auch solche Salze, welche mit organischen Aminen, wie gegebenenfalls durch Hydroxy substituierten Mono-, Di- oder Trialkylaminen, gebildet werden, z.B. mit Diäthylamin, Di-(2-hydroxyäthyl)-amin, Triäthylamin, N,N-Dimethyl-N-(2-hydroxyäthyl)-amin, Tri-(2-hydroxyäthyl)-amin oder N-Methyl-D-glucamin. Die Verbindungen der Formel I mit einer basischen Gruppe, z.B. einer Aminogruppe, können Säureadditionssalze bilden, z.B. mit anorganischen Säuren, z.B. Salzsäure, Schwefelsäure oder Phosphorsäure, oder mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure oder Isonicotinsäure, ferner mit Aminosäuren, wie z.B. den weiter vorn genannten α-Aminosäuren, sowie mit Methansulfonsäure, Aethansulfonsäure, 2-Hydroxyäthansulfonsäure, Aethan-1,2-disulfonsäure, Benzolsulfonsäure, 4-Methylbenzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden.

Die Verbindungen der vorliegenden Erfindung weisen Enzym-hemmende Wirkungen auf; insbesondere hemmen sie die Wirkung des natürlichen Enzyms Renin. Letzteres gelangt aus den Nieren in das Blut und bewirkt dort die Spaltung von Angiotensinogen unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III

zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Renin-Hemmern wird unter anderem experimentell mittels in vitro-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro Test verwendet: Ein Extrakt von menschlichem Renin aus der Niere (0,5 mGU [Milli-Goldblatt-Einheiten]/ml) wird eine Stunde lang bei 37°C und pH 7,2 in 1-molarer wässriger 2-N-(Tris-hydroxymethyl-methyl)-amino-äthansulfonsäure-Pufferlösung mit 23 µg/ml synthetischem Renin-Substrat, dem Tetradecapeptid H-Asp-Arg-Val-Tyr-Ile-His-Pro-Phe-His-Leu-Leu-Val-Tyr-Ser-OH, inkubiert. Die Menge des gebildeten Angiotensins I wird in einem Radioimmunoassay ermittelt. Die erfindungsgemässen Hemmstoffe werden dem Inkubationsgemisch jeweils in verschiedenen Konzentrationen zugefügt. Als $IC_{50}$ wird diejenige Konzentration des jeweiligen Hemmstoffes bezeichnet, die die Bildung von Angiotensin I um 50 % reduziert. Die Verbindungen der vorliegenden Erfindung zeigen in den in vitro-Systemen Hemmwirkungen bei minimalen Konzentrationen von etwa $10^{-7}$ bis etwa $10^{-10}$ Mol/l.

An salzverarmten Tieren bewirken Renin-Hemmer einen Blutdruckabfall. Das menschliche Renin unterscheidet sich von Renin anderer Spezies. Zur Prüfung von Hemmern des humanen Renins werden Primaten (Marmosets, Callithrix jacchus) verwendet, weil humanes Renin und Primaten-Renin im enzymatisch aktiven Bereich weitgehend homolog sind. Unter anderem wird der folgende in vivo Test benutzt: Die Testverbindungen werden an normotensiven Marmosets beider Geschlechter mit einem Körpergewicht von etwa 300 g, die bei Bewusstsein sind, geprüft. Blutdruck und Herzfrequenz werden mit einem Katheter in der Oberschenkelarterie gemessen. Die endogene Freisetzung von Renin wird durch die intravenöse Injektion von Furosemid (5 mg/kg)

angeregt. 30 Minuten nach der Injektion von Furosemid werden die Testsubstanzen entweder über einen Katheter in der lateralen Schwanzvene durch einmalige Injektion oder durch kontinuierliche Infusion oder peroral direkt in den Magen als Lösung oder Suspension verabreicht und ihre Wirkung auf Blutdruck und Herzfrequenz ausgewertet. Die Verbindungen der vorliegenden Erfindung sind in dem beschriebenen in vivo Test bei Dosen von etwa 0,1 bis etwa 1,0 mg/kg i.v. und bei Dosen von etwa 1,0 bis etwa 10 mg/kg p.o. wirksam.

Die Verbindungen der vorliegenden Erfindung können als Antihypertensiva, ferner zur Behandlung von Herzinsuffizienz verwendet werden.

Die Erfindung betrifft insbesondere Verbindungen der Formel I, worin $R_1$ eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, $R_5$ Niederalkyl mit 2 und mehr C-Atomen, Hydroxyniederalkyl, Cycloalkyl, Cycloalkylniederalkyl, Bicycloalkyl, Bicycloalkylniederalkyl, Tricycloalkyl, Tricycloalkylniederalkyl, Carbamoyl, Niederalkylcarbamoyl, Diniederalkylamino, Aryl oder Arylniederalkyl und $R_6$ eine Aminogruppe, welche durch einen oder gegebenenfalls zwei unsubstituierte oder substituierte, gesättigte oder ungesättigte, aliphatische Kohlenwasserstoffreste mit bis einschliesslich 18, bevorzugt bis einschliesslich 10, C-Atomen oder einen unsubstituierten oder substituierten aromatischen, heteroaromatischen, aromatisch-aliphatischen oder heteroaromatisch-aliphatischen Kohlenwasserstoffrest mit bis einschliesslich 18, bevorzugt bis einschliesslich 10, C-Atomen substituiert ist, mit Ausnahme eines von einer α-Aminosäure abgeleiteten Aminorestes, darstellen, ferner pharmazeutisch annehmbare Salze von solchen Verbindungen mit salzbildenden Gruppen.

Die Erfindung betrifft hauptsächlich Verbindungen der Formel I, worin $R_1$ einen Rest der Formel

$$R^a - \underset{(O)_m}{\underset{\|}{S}} - (CH_2)_n - \underset{R^b}{\underset{|}{\underset{(CH_2)_q}{\underset{|}{CH}}}} - (CH_2)_p - \underset{O}{\underset{\|}{C}} - \qquad (Ia),$$

worin $R^a$ unsubstituiertes oder substituiertes Niederalkyl, z.B. Methyl, Aethyl, Isopropyl, tert-Butyl, 2-Hydroxyäthyl, 2-Methoxyäthyl, 2-Phenoxyäthyl, 2-Acetoxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl, 2-Carbamoyläthyl, 2-Aminoäthyl, 2-Dimethylaminoäthyl, 2-Morpholinoäthyl, 2-Piperidinoäthyl, 2-Benzyloxycarbonylaminoäthyl, 2-tert-Butoxycarbonylaminoäthyl, 2-Oxopropyl oder 2-Oxobutyl, Niederalkenyl, z.B. Vinyl, Allyl oder 2- oder 3-Butenyl, Niederalkinyl, z.B. Aethinyl, 1-Propinyl, oder 2-Propinyl, Cycloalkyl, z.B. Cyclopropyl, Cyclobutyl, Cyclopentyl oder Cyclohexyl, Bicycloalkyl, z.B. Bicyclo[2.2.1]hept-2-yl, Tricycloalkyl, z.B. 1-Adamantyl, Cycloalkylniederalkyl, z.B. Cyclopropylmethyl, Cyclobutylmethyl, Cyclopentylmethyl oder Cyclohexylmethyl, unsubstituiertes oder substituiertes Aryl, z.B. Phenyl, 1- oder 2-Naphthyl, o-, m- oder p-Methylphenyl, o-, m- oder p-Hydroxyphenyl oder o-, m- oder p-Aminophenyl, Arylniederalkyl, z.B. Benzyl, 2-Phenyläthyl oder α- oder β-Naphthylmethyl, Arylniederalkenyl, z.B. Styryl oder 3-Phenylallyl, unsubstituiertes oder substituiertes Heteroaryl, z.B. 2- oder 3-Pyrrolyl, 2-Furyl, 2-Thienyl, 2- oder 4-Imidazolyl, 1-Methyl-2-, 4- oder 5-Imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl, 2-, 3- oder 4-Chinolyl, 1-, 3- oder 4-Isochinolyl oder

2-Benzoxazolyl, Heteroarylniederalkyl, z.B. 2-, 3- oder 4-Pyridylmethyl, 2-(2-, 3- oder 4-Pyridyl)-äthyl, 4-Imidazolylmethyl oder 2-(4-Imidazolyl)-äthyl, Hydroxy, substituiertes Hydroxy, z.B. Niederalkoxy, z.B. Methoxy, Aethoxy oder n-Butoxy, oder Aryloxy, z.B. Phenoxy, 4-Hydroxyphenoxy oder 3,4-Methylendioxyphenoxy, Amino oder substituiertes Amino, z.B. Niederalkylamino, z.B. Methylamino, Aethylamino, Isopropylamino, n- oder tert-Butylamino, Diniederalkylamino, z.B. Dimethylamino oder Diäthylamino, oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl, $R^b$ Wasserstoff, Cycloalkyl, z.B. Cyclopentyl oder Cyclohexyl, Bicycloalkyl, z.B. Bicyclo[2.2.1]hept-2-yl, Tricycloalkyl, z.B. 1-Adamantyl, unsubstituiertes oder substituiertes Aryl, z.B. Phenyl, 1- oder 2-Naphthyl, o-, m- oder p-Methylphenyl, o-, m- oder p-Hydroxyphenyl oder o-, m- oder p-Aminophenyl, oder unsubstituiertes oder substituiertes Heteroaryl, z.B. 2- oder 4-Imidazolyl, 2-, 3- oder 4-Pyridyl, 1-Oxido-2-, 3- oder 4-pyridinio oder 2-, 4- oder 5-Pyrimidinyl, m 0, 1 oder 2, bevorzugt 2, n 0, 1 oder 2, bevorzugt 1, p 0, 1 oder 2, bevorzugt 0, und q 0, 1, 2, 3 oder 4, bevorzugt 1 oder 2, darstellen,

A einen bivalenten Rest einer α-Aminosäure, beispielsweise einer natürlichen α-Aminosäure mit der L-Konfiguration, wie sie normalerweise in Proteinen vorkommen, eines Homologen einer solchen Aminosäure, z.B. worin die Aminosäureseitenkette um eine oder zwei Methylengruppen verlängert oder verkürzt ist und/oder eine Methylgruppe durch Wasserstoff ersetzt ist, einer substituierten aromatischen α-Aminosäure, z.B. eines substituierten Phenylalanins oder Phenylglycins, worin der Substituent Niederalkyl, z.B. Methyl, Halogen, z.B. Fluor, Chlor, Brom oder Jod, Hydroxy, Niederalkoxy, z.B. Methoxy, Niederalkanoyloxy, z.B. Acetoxy, Amino, Niederalkylamino, z.B. Methylamino, Diniederalkylamino, z.B. Dimethylamino, Niederalkanoylamino, z.B. Acetylamino oder Pivaloylamino, Niederalkoxycarbonylamino, z.B. tert-Butoxycarbonylamino, Arylmethoxycarbonylamino, z.B. Benzyloxycarbonylamino, und/oder Nitro sein kann und ein- oder mehrfach vorkommt, eines benzannellierten Phenylala-

nins oder Phenylglycins, wie α-Naphthylalanins, oder eines hydrierten Phenylalanins oder Phenylglycins, wie Cyclohexylalanins oder Cyclohexylglycins, einer fünf- oder sechsgliedrigen cyclischen, benzannellierten α-Aminosäure, z.B. Indolin-2-carbonsäure oder 1,2,3,4-Tetrahydroisochinolin-3-carbonsäure, einer natürlichen oder homologen α-Aminosäure, in der eine Carboxygruppe der Seitenkette in veresterter oder amidierter Form vorliegt, z.B. als Niederalkylestergruppe, wie Methoxycarbonyl oder tert-Butoxycarbonyl, oder als Carbamoyl-, als Niederalkylcarbamoyl-, wie Methylcarbamoyl, oder als Diniederalkylcarbamoylgruppe, wie Dimethylcarbamoyl, in der eine Aminogruppe der Seitenkette in acylierter Form vorliegt, z.B. als Niederalkanoylamino-, wie Acetylamino oder Pivaloylamino, als Niederalkoxycarbonylamino-, wie tert-Butoxycarbonylamino, oder als Arylmethoxycarbonylaminogruppe, wie Benzyloxycarbonylamino, oder in der eine Hydroxygruppe der Seitenkette in verätherter oder veresterter Form vorliegt, z.B. als Niederalkoxygruppe, wie Methoxy, als Arylniederalkoxygruppe, wie Benzyloxy, oder als Niederalkanoyloxygruppe, wie Acetoxy, oder eines Epimeren einer solchen Aminosäure, d.h. mit der unnatürlichen D-Konfiguration, gegebenenfalls am N-Atom durch Niederalkyl, z.B. Methyl, substituiert,

$R_2$ Wasserstoff oder Niederalkyl, z.B. Methyl, $R_3$ Niederalkyl, z.B. Isopropyl oder Isobutyl, Cycloalkyl, z.B. Cyclohexyl, Cycloalkylniederalkyl, z.B. Cyclopentylmethyl, Cyclohexylmethyl, 2-Cyclohexyläthyl oder Cycloheptylmethyl, Tricycloalkylniederalkyl, z.B. 1-Adamantylmethyl, Phenylniederalkyl, z.B. Benzyl, oder Phenyl, $R_4$ Hydroxy, $R_5$ Niederalkyl, z.B. Methyl, Isopropyl, Isobutyl oder tert-Butyl, Cycloalkyl, z.B. Cyclopentyl oder Cyclohexyl, Cycloalkylniederalkyl, z.B. Cyclohexylmethyl, Bicycloalkyl, z.B. α-Decahydronaphthyl, Tricycloalkyl, z.B. 1-Adamantyl, Phenyl, Phenylniederalkyl, z.B. Benzyl, Carbamoyl oder Niederalkylcarbamoyl, z.B. Methylcarbamoyl, Diniederalkylamino, z.B. Dimethylamino, Hydroxy, Niederalkoxy, z.B. Methoxy, Niederalkylthio, z.B. Methylthio, oder Niederalkylsulfonyl, z.B. Methylsulfonyl,

und $R_6$ Alkylamino mit 1 bis 10 C-Atomen, z.B. Methyl-, Aethyl-, n-Propyl-, Isopropyl, n-Butyl-, Isobutyl-, tert-Butyl-, n-Pentyl-, Isopentyl, n-Hexyl-, n-Octyl- oder n-Decylamino, Diniederalkylamino, z.B. Dimethylamino oder Diäthylamino, Hydroxyniederalkylamino, z.B. 2-Hydroxyäthylamino, 1-Hydroxybut-2-ylamino, 5-Hydroxypentylamino oder Tris-(hydroxymethyl)-methylamino, Di-(hydroxyniederalkyl)-amino, z.B. Di-(2-hydroxyäthyl)-amino, Niederalkoxyniederalkylamino, z.B. 2-Methoxyäthylamino, Niederalkanoyloxyniederalkylamino, z.B. 2-Acetoxyäthylamino, Phenoxyniederalkylamino oder Phenoxy-hydroxy-niederalkylamino, worin Phenoxy gegebenenfalls durch Niederalkyl, Niederalkoxy, Hydroxy, Carboxy, Niederalkoxycarbonyl oder Carbamoyl substituiert ist, z.B. 2-Phenoxyäthylamino, 2-(3-Carbamoyl-4-hydroxyphenoxy)-äthylamino oder 3-(3-Carbamoylphenoxy)-2-hydroxy-propylamino, Carboxyalkylamino oder Amino-carboxy-alkylamino, worin der Carboxyrest nicht in 1-Stellung des Alkylrests steht, z.B. 4-Carboxy-n-butyl-, 5-Carboxy-n-pentyl-, 6-Carboxy-n-hexyl-, 7-Carboxy-n-heptyl- oder 8-Carboxy-n-octylamino oder 5-Amino-5-carboxy-n-pentylamino, ferner Dicarboxy-methylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butyl-, 7-tert-Butoxycarbonyl-n-heptyl- oder 8-tert-Butoxycarbonyl-n-octylamino oder 5-tert-Butoxycarbonylamino-5-methoxycarbonyl-n-pentylamino, ferner Diniederalkoxycarbonyl-methylamino, z.B. Di-methoxycarbonyl-methylamino, physiologisch spaltbares verestertes Carboxyalkylamino, worin die Esterfunktion nicht in 1-Stellung des Alkylrests steht, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoyl- oder Hydroxyniederalkylcarbamoylalkylamino, worin der Carbamoylrest nicht in 1-Stellung des Alkylrests steht, z.B. 4-Carbamoyl-n-butylamino, 7-Carbamoyl-n-heptylamino oder 4-(Tris-[hydroxymethyl]-methyl)-carbamoyl-n-butylamino, ferner Dicarbamoyl-methylamino, Di-(niederalkylcarbamoyl)-methylamino, z.B. Di-(methylcarbamoyl)-methylamino, Di-(hydroxyniederalkylcarbamoyl)-methylamino, z.B. Di-(2-hydroxyäthylcarbamoyl)-methylamino, oder Bis-(diniederalkylcarbamoyl)-methylamino, z.B. Bis-(dimethylcarbamoyl)-methylamino, Aminoniederalkylamino, z.B. 2-Aminoäthylamino oder 4-Aminobutylamino, Niederalkylaminoniederalkylamino, z.B. 2-Methylaminoäthylamino, Diniederalkylaminoniederalkylamino, z.B. 2-Dimethylaminoäthylamino oder 3-Dimethylaminopropylamino, Niederalkoxycarbonylaminoniederalkylamino, z.B. 2-(tert-Butoxycarbonylamino)-äthylamino, Guanidinoniederalkylamino, z.B. 2-Guanidino-äthylamino, über ein Stickstoffatom gebundenes, gesättigtes fünf- oder sechsgliedriges Heterocyclylniederalkylamino, z.B. 2-(4-Morpholinyl)-äthylamino, 3-(4-Morpholinyl)-propylamino oder 3-(2-Oxopyrrolidin-1-yl)-propylamino, Niederalkenylamino, z.B. Allylamino oder 2- oder 3-Butenylamino, Niederalkinylamino, z.B. Propargylamino, Cycloalkylniederalkylamino, z.B. Cyclopropylmethylamino oder Cyclohexylmethylamino, Phenylamino oder Phenylniederalkylamino, worin Phenyl gegebenenfalls durch Niederalkyl, z.B. Methyl, Hydroxy, Niederalkoxy, z.B. Methoxy oder tert-Butoxy, Niederalkanoyloxy, z.B. Acetoxy, Halogen, z.B. Fluor oder Chlor, Carboxy, Niederalkoxycarbonyl, z.B. tert-Butoxycarbonyl, Carbamoyl, Amino, Niederalkylamino, z.B. Methylamino, Diniederalkylamino, z.B. Dimethylamino, Acylamino, z.B. tert-Butoxycarbonylamino und/oder durch Nitro mono- oder mehrfach substituiert ist, z.B. Phenyl-, 2-, 3- oder 4-Methylphenyl-, 4-Hydroxyphenyl-, 4-Methoxyphenyl-, 2,3-, 2,4-oder 2,5-Dimethoxyphenyl-, 4-Chlorphenyl-, 2-, 3- oder 4-Carboxyphenyl-, 2-, 3- oder 4-Methoxy- oder tert-Butoxycarbonylphenyl-, 2-, 3- oder 4-Carbamoylphenyl-, 4-Aminophenyl-, 4-tert-Butoxycarbonylaminophenyl- oder 4-Nitrophenylamino, ferner z.B. Benzylamino, 4-Methylbenzylamino, 4-Methoxybenzylamino, 2-, 3- oder 4-Carboxybenzylamino, 2-, 3- oder 4-tert-Butoxycarbonylbenzylamino, 2-, 3- oder 4-Carbamoylbenzylamino, 2-Phenyläthylamino oder 3-Phenylpropylamino, Pyridylniederalkylamino, z.B. 2-, 3- oder 4-Pyridylmethyl-, 2-(2-, 3- oder 4-Pyridyl)-äthyl- oder 3-(2-, 3- oder 4-Pyridyl)-propyl-amino, Imidazolylniederalkylamino, z.B. 4-Imidazolylmethylamino, 2-(4-Imidazolyl)-äthylamino oder 2-(2-[4-Imidazolyl]-äthylamino)-äthylamino, Indolylniederalkylamino, z.B. 3-Indolylmethylamino oder

2-(3-Indolyl)-äthylamino, oder Sulfoniederalkylamino, z.B. 2-Sulfoäthylamino, darstellen, sowie pharmazeutisch annehmbare Salze von diesen Verbindungen mit salzbildenden Gruppen.

Die Erfindung betrifft ganz besonders Verbindungen der Formel I, worin $R_1$ einen Rest der Formel

$$R^a-\underset{(O)_m}{S}-(CH_2)_n-\underset{\underset{R^b}{|}}{\underset{(CH_2)_q}{\underset{|}{CH}}}-(CH_2)_p-\underset{O}{\overset{}{\underset{\|}{C}}}- \qquad (Ia),$$

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, z.B. Methyl, Aethyl, Isopropyl, tert-Butyl, 2-Hydroxyäthyl, 2-Methoxyäthyl, 2-Phenoxyäthyl, 2-Acetoxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl oder 2-Carbamoyläthyl, Cycloalkyl, z.B. Cyclopentyl oder Cyclohexyl, Cycloalkylniederalkyl, z.B. Cyclopropylmethyl, Cyclopentylmethyl oder Cyclohexylmethyl, unsubstituiertes oder durch Niederalkyl, Hydroxy oder Amino substituiertes Aryl, z.B. Phenyl, 1- oder 2-Naphthyl, o-, m- oder p-Methylphenyl, m- oder p-Hydroxyphenyl oder m- oder p-Aminophenyl, Arylniederalkyl, z.B. Benzyl, 2-Phenyläthyl oder α- oder β-Naphthylmethyl, unsubstituiertes oder durch Oxido, Niederalkyl oder Phenyl substituiertes Heteroaryl, z.B. 2- oder 4-Imidazolyl, 1-Methyl-2-, 4- oder 5-imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl oder 2-Benzoxazolyl, Heteroarylniederalkyl, z.B. 2-, 3- oder 4-Pyridylmethyl oder 4-Imidazolylmethyl, Hydroxy, substituiertes Hydroxy, z.B. Niederalkoxy, z.B. Methoxy, Aethoxy oder n-Butoxy, oder Aryloxy, z.B. Phenoxy, 4-Hydroxyphenoxy oder 3,4-Methylendioxyphenoxy, Amino oder substituiertes Amino, z.B. Niederalkylamino, z.B. Methylamino, Aethylamino, Isopropylamino, n- oder tert-Butylamino, Diniederalkylamino, z.B. Dimethylamino oder Diäthylamino, oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl, $R^b$ Wasserstoff, Cycloalkyl, z.B. Cyclohexyl, Aryl, z.B. Phenyl oder 1- oder 2-Naphthyl, oder Heteroaryl, z.B. 4-Imidazolyl oder 2-, 3- oder 4-Pyridyl, m 0, 1 oder 2, n 0, 1 oder 2, p 0, 1 oder 2 und q 0, 1, 2 oder 3 darstellen,

A den bivalenten Rest der Aminosäuren Alanin, Valin, Norvalin, Leucin, Norleucin, Serin, Prolin, Phenylalanin, β-Phenylserin, α-Naphthylalanin, Cyclohexylalanin, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutamin, Diniederalkylglutamin, Histidin, Lysin oder Ornithin, wobei die Carboxygruppe in der Seitenkette von Asparaginsäure oder Glutaminsäure mit einem Niederalkanol, z.B. Methanol oder tert-Butanol, verestert, die Hydroxygruppe in Serin mit Niederalkyl, z.B. Methyl, oder mit Benzyl veräthert, die Aminogruppe in der Seitenkette von Lysin oder Ornithin durch Niederalkanoyl, z.B. Pivaloyl, durch Niederalkoxycarbonyl, z.B. tert-Butoxycarbonyl, oder durch Arylmethoxycarbonyl, z.B. Benzyloxycarbonyl, acyliert und/oder das α-Stickstoffatom der Aminosäuren durch Niederalkyl, z.B. Methyl, substituiert sein kann,

$R_2$ Wasserstoff oder Niederalkyl, z.B. Methyl, $R_3$ Niederalkyl, z.B. Isopropyl oder Isobutyl, Cycloalkylniederalkyl, z.B. Cyclopentylmethyl, Cyclohexylmethyl, 2-Cyclohexyläthyl oder Cycloheptylmethyl, oder Tricycloalkylniederalkyl, z.B. 1-Adamantylmethyl, $R_4$ Hydroxy und $R_5$ Niederalkyl, z.B. Methyl, Isopropyl oder tert-Butyl, Cycloalkyl, z.B. Cyclopentyl oder Cyclohexyl, Cycloalkylniederalkyl, z.B. Cyclohexylmethyl, 1-Adamantyl, Benzyl, Carbamoyl oder Niederalkylcarbamoyl, z.B. Methylcarbamoyl, Diniederalkylamino, z.B. Dimethylamino, Hydroxy, Niederalkoxy, z.B. Methoxy, Niederalkylthio, z.B. Methylthio, oder Niederalkylsulfonyl, z.B. Methylsulfonyl, und

$R_6$ Alkylamino mit 1 bis 10 C-Atomen, z.B. Methyl-, Aethyl-, n-Propyl-, Isopropyl-, n-Butyl-, Isobutyl-, tert-Butyl-, n-Pentyl-, Isopentyl-, n-Hexyl-, n-Octyl- oder n-Decylamino, Diniederalkylamino, z.B. Dimethylamino, Hydroxyniederalkylamino, z.B. 2-Hydroxyäthylamino, 1-Hydroxybut-2-ylamino oder Tris-(hydroxymethyl)-methylamino, Carboxyalkylamino oder Amino-carboxy-alkylamino, worin der Carboxyrest nicht in 1-Stellung des Alkylrests steht, z.B. 4-Carboxy-n-butyl-, 5-Carboxy-n-pentyl-, 6-Carboxy-n-hexyl-, 7-Carboxy-n-heptyl- oder 8-Carboxy-n-octylamino oder 5-Amino-5-carboxy-n-pentylamino, ferner Dicarboxy-methylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butyl-, 7-tert-Butoxycarbonyl-n-heptyl- oder 8-tert-Butoxycarbonyl-n-octylamino oder 5-tert-Butoxycarbonylamino-5-methoxycarbonyl-n-pentylamino, ferner Diniederalkoxycarbonyl-methylamino, z.B. Di-methoxycarbonyl-methylamino, physiologisch spaltbares verestertes Carboxyalkylamino, worin die Esterfunktion nicht in 1-Stellung des Alkylrests steht, z.B. 4-Pivaloyloxymethoxycarbonyl-n-butylamino, 7-(1-Aethoxycarbonyloxyäthoxycarbonyl)-n-heptylamino oder 7-Pivaloyloxymethoxycarbonyl-n-heptylamino, Carbamoyl- oder Hydroxyniederalkylcarbamoylalkylamino, worin der Carbamoylrest nicht in 1-Stellung des Alkylrests steht, z.B. 4-Carbamoyl-n-butylamino, 7-Carbamoyl-n-heptylamino oder 4-(Tris-[hydroxymethyl]-methyl)-carbamoyl-n-butylamino, ferner Dicarbamoyl-methylamino, Di-(niederalkylcarbamoyl)-methylamino, z.B. Di-(methylcarbamoyl)-methylamino, Di-(hydroxyniederalkylcarbamoyl)-methylamino, z.B. Di-(2-hydroxyäthylcarbamoyl)-methylamino, oder Bis-(diniederalkylcarbamoyl)-methylamino, z.B. Bis-(dimethylcarbamoyl)-methylamino, Aminoniederalkylamino, z.B. 2-Aminoäthylamino oder 4-Aminobutylamino, Niederalkylaminoniederalkylamino, z.B. 2-Methylaminoäthylamino, Diniederalkylaminoniederalkylamino, z.B. 2-Dimethylaminoäthylamino, Niederalkoxycarbonylaminoniederalkylamino, z.B. 2-(tert-Butoxycarbonylamino)-äthylamino, Guanidinoniederalkylamino, z.B. 2-Guanidinoäthylamino, Cycloalkylniederalkylamino, z.B. Cyclopropylmethylamino, Benzylamino, Pyridylniederalkylamino, z.B. 2-, 3- oder 4-Pyridylmethylamino, 2-(2-, 3- oder 4-Pyridyl)-äthylamino oder 3-(2-, 3-

oder 4-Pyridyl)-propylamino, Imidazolylniederalkylamino, z.B. 4-Imidazolylmethylamino oder 2-(4-Imidazolyl)-äthylamino, Indolylniederalkylamino, z.B. 3-Indolylmethylamino oder 2-(3-Indolyl)-äthylamino, oder Sulfoniederalkylamino, z.B. 2-Sulfoäthylamino, darstellen, sowie pharmazeutisch annehmbare Salze von diesen Verbindungen mit salzbildenden Gruppen.

Ganz besonders bevorzugt sind Verbindungen der Formel I, worin $R_1$ einen Rest der Formel

$$R^a-\underset{(O)_m}{S}-(CH_2)_n-\underset{\substack{|\\(CH_2)_q\\|\\R^b}}{CH}-(CH_2)_p-\underset{O}{\overset{\|}{C}}- \qquad (Ia),$$

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, z.B. Methyl, Aethyl, Isopropyl, tert-Butyl, 2-Hydroxyäthyl, 2-Methoxyäthyl, 2-Phenoxyäthyl, 2-Acetoxyäthyl, Carboxymethyl, 2-Carboxyäthyl, Methoxycarbonylmethyl, 2-Methoxycarbonyläthyl, Aethoxycarbonylmethyl, 2-Aethoxycarbonyläthyl, Carbamoylmethyl oder 2-Carbamoyläthyl, Cycloalkyl, z.B. Cyclopentyl oder Cyclohexyl, Cycloalkylniederalkyl, z.B. Cyclopropylmethyl, Cyclopentylmethyl oder Cyclohexylmethyl, unsubstituiertes oder durch Niederalkyl, Hydroxy oder Amino substituiertes Aryl, z.B. Phenyl, 1- oder 2-Naphthyl, o-, m- oder p-Methylphenyl, m- oder p-Hydroxyphenyl oder m- oder p-Aminophenyl, Arylniederalkyl, z.B. Benzyl, 2-Phenyläthyl oder α- oder β-Naphthylmethyl, unsubstituiertes oder durch Oxido, Niederalkyl oder Phenyl substituiertes Heteroaryl, z.B. 2- oder 4-Imidazolyl, 1-Methyl-2-, 4- oder 5-imidazolyl, 1,3-Thiazol-2-yl, 2-, 3- oder 4-Pyridyl, 1-Oxido-2-, 3- oder 4-pyridinio, 2-Pyrazinyl, 2-, 4- oder 5-Pyrimidinyl oder 2-Benzoxazolyl, oder Heteroarylniederalkyl, z.B. 2-, 3- oder 4-Pyridylmethyl oder 4-Imidazolylmethyl, Hydroxy, substituiertes Hydroxy, z.B. Niederalkoxy, z.B. Methoxy oder Aethoxy, Amino oder substituiertes Amino, z.B. Niederalkylamino, z.B. Methylamino, Aethylamino, Isopropylamino, n- oder tert-Butylamino, Diniederalkylamino,

z.B. Dimethylamino oder Diäthylamino, oder Amino als Teil eines fünf- oder sechsgliedrigen Ringes enthaltend ein Stickstoffatom und gewünschtenfalls ein Sauerstoffatom, z.B. 1-Pyrrolidinyl, 1-Piperidinyl oder 4-Morpholinyl, $R^b$ Wasserstoff, Cycloalkyl, z.B. Cyclohexyl, Aryl, z.B. Phenyl oder 1- oder 2-Naphthyl, oder Heteroaryl, z.B. 4-Imidazolyl oder 2-, 3- oder 4-Pyridyl, m 0, 1 oder 2, n 0 oder 1, p 0 und q 1 oder 2 darstellen,

A den bivalenten Rest der Aminosäuren Alanin, Serin, Phenylalanin, N-Methyl-phenylalanin, Cyclohexylalanin, Histidin oder N-Methylhistidin, $R_2$ Wasserstoff, $R_3$ Niederalkyl, z.B. Isobutyl, oder Cycloalkylniederalkyl, z.B. Cyclopentylmethyl, Cyclohexylmethyl, 2-Cyclohexyläthyl oder Cycloheptylmethyl, $R_4$ Hydroxy, $R_5$ Niederalkyl, z.B. Isopropyl, Hydroxy, Niederalkoxy, z.B. Methoxy, Niederalkylthio, z.B. Methylthio, oder Niederalkylsulfonyl, z.B. Methylsulfonyl, und

$R_6$ Niederalkylamino mit 1 - 7 C-Atomen, z.B. Methyl-, Aethyl-, n-Propyl-, Isopropyl-, n-Butyl-, Isobutyl-, n-Pentyl- oder Isopentyl-amino, Diniederalkylamino, z.B. Dimethylamino, Hydroxyniederalkylamino, z.B. 2-Hydroxyäthylamino, 1-Hydroxybut-2-ylamino oder 5-Hydroxypentylamino, Carboxyalkylamino oder Amino-Carboxyalkylamino, worin der Carboxyrest nicht in 1-Stellung des Alkylrests steht, z.B. 4-Carboxy-n-butylamino, 7-Carboxy-n-heptylamino oder 8-Carboxy-n-octylamino oder 5-Amino-5-carboxy-n-pentylamino, Niederalkoxycarbonylalkylamino oder Acylamino-niederalkoxycarbonyl-alkylamino, worin der Carbonylrest nicht in 1-Stellung des Alkylrests steht, z.B. 4-tert-Butoxycarbonyl-n-butylamino oder 7-tert-Butoxycarbonyl-n-heptylamino oder 5-tert-Butoxycarbonylamino-5-methoxycarbonyl-n-pentylamino, Aminoniederalkylamino, z.B. 2-Aminoäthylamino oder 4-Aminobutylamino, Diniederalkylaminoniederalkylamino, z.B. 2-Dimethylaminoäthylamino oder 3-Dimethylaminopropylamino, Niederalkoxycarbonylaminoniederalkylamino, z.B. 2-tert-Butoxycarbonylamino-äthylamino, Morpholinoniederalkylamino, z.B. 2-Morpholinoäthylamino, Pyridylniederalkylamino, z.B. 2-Pyridylmethylamino, Imidazolylniederalkylamino, z.B. 2-(4-Imidazolyl)-äthyl-

amino, oder Sulfoniederalkylamino, z.B. 2-Sulfoäthylamino, darstellen, sowie pharmazeutisch annehmbare Salze von diesen Verbindungen mit salzbildenden Gruppen.

In erster Linie betrifft die Erfindung Verbindungen der Formel I, worin $R_1$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia),$$

worin $R^a$ unsubstituiertes oder durch Hydroxy oder Niederalkoxy substituiertes Niederalkyl, z.B. Methyl, Aethyl, Isopropyl, tert-Butyl, 2-Hydroxyäthyl oder 2-Methoxyäthyl, Phenyl, Benzyl oder unsubstituiertes oder durch Oxido oder Niederalkyl substituiertes Heteroaryl mit 1 oder 2 Stickstoffatomen, z.B. 2- oder 4-Imidazolyl, 1-Methyl-2-imidazolyl, 2-, 3- oder 4-Pyridyl, 1-Oxido-2-, 3- oder 4-pyridinio oder 2-Pyrimidinyl, $R^b$ Cyclohexyl oder Phenyl, m 0, 1 oder 2, n 1, p 0 und q 1 oder 2 darstellen, A den bivalenten Rest der Aminosäure Histidin, $R_2$ Wasserstoff, $R_3$ Cyclohexylmethyl, $R_4$ Hydroxy, $R_5$ Isopropyl und $R_6$ Niederalkylamino mit 1-7 C-Atomen, z.B. Methyl-, Aethyl-, n-Propyl-, Isopropyl-, n-Butyl-, Isobutyl-, n-Pentyl- oder Isopentylamino, Dimethylamino oder 2-Hydroxyäthylamino, darstellen, sowie pharmazeutisch annehmbare Salze von diesen Verbindungen.

Ganz besonders betrifft die Erfindung Verbindungen der Formel I, worin $R_1$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia),$$

worin $R^a$ Niederalkyl, z.B. Methyl, Aethyl, Isopropyl oder tert-Butyl, Phenyl, 2-Pyridyl, Hydroxy, Niederalkylamino, z.B. Methylamino oder Isopropylamino, Diniederalkylamino, z.B. Dimethylamino

oder Diäthylamino, oder Pyrrolidino, $R^b$ Phenyl, m 2, n 1, p 0 und q 1 bedeuten, A den bivalenten Rest der Aminosäure Alanin, Serin oder Histidin, $R_2$ Wasserstoff, $R_3$ Cycloalkylniederalkyl, z.B. Cyclopentylmethyl, Cyclohexylmethyl, 2-Cyclohexyläthyl oder Cycloheptylmethyl, $R_4$ Hydroxy, $R_5$ Methyl, Isopropyl, Hydroxy, Methoxy, Methylthio oder Methylsulfonyl und $R_6$ Niederalkylamino mit 1 - 4 C-Atomen, z.B. Methylamino, Aethylamino, n-Propylamino oder n-Butylamino, 5-Amino-5-carboxy-n-pentylamino, 4-Aminobutylamino, 2-(4-Imidazolyl)-äthylamino oder 2-Sulfoäthylamino darstellen und die die Reste $R_3$ und $R_4$ tragenden C-Atome die S-Konfiguration aufweisen, ferner pharmazeutisch annehmbare Salze von diesen Verbindungen.

Die Erfindung betrifft zuallererst die in den Beispielen erwähnten Verbindungen und deren pharmazeutisch annehmbare Salze, insbesondere

die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze,

die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Methyl und $R_6$ Methylamino bedeuten und die die Reste $R_3$ und $R_4$ tragenden C-Atome und das Methin-C-Atom der Teilformel Ia die S-Konfiguration und das den Rest $R_5$ tragende C-Atom die R-Konfiguration aufweisen, und deren pharmazeutisch annehmbare Salze,

die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Alanin, $R_2$ Wasserstoff, $R_3$ Cyclohexylmethyl, $R_4$ Hydroxy, $R_5$ Isopropyl und $R_6$ n-Butylamino

bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze,

die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cycloheptylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze, und

die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ Methylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze.

Verfahren:

Die erfindungsgemässen Verbindungen der Formel I und Salze von solchen Verbindungen mit mindestens einer salzbildenden Gruppe werden nach an sich bekannten Verfahren erhalten, z.B. indem man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

$$R_1 - A - \overset{R_2}{\overset{|}{N}} - \underset{\underset{R_3}{|}}{CH} - \overset{O}{\overset{\|}{C}} - CH_2 - \overset{R_5}{\overset{|}{CH}} - \overset{O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

$$R_1 - A - \overset{R_2}{\overset{|}{N}} - \underset{\underset{R_3}{|}}{CH} - \overset{O}{\overset{\|}{C}} - H \qquad (III),$$

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

$$M - CH_2 - \overset{R_5}{\overset{|}{CH}} - \overset{O}{\overset{\|}{C}} - R_6 \qquad (IV),$$

worin die Substituenten die genannten Bedeutungen haben und M ein Metallradikal bedeutet, umsetzt und das gebildete Additionsprodukt hydrolysiert, oder

d) in einer Verbindung der Formel

$$R_1 - A - \overset{R_2}{\overset{|}{N}} - \underset{\underset{R_3}{|}}{CH} - \overset{X}{\overset{|}{CH}} - CH_2 - \overset{R_5}{\overset{|}{CH}} - \overset{O}{\overset{\|}{C}} - R_6 \qquad (V),$$

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X durch ein den Substituenten $R_4$ in nukleophiler Form einführendes Reagens gegen $R_4$ austauscht, oder

e) in einer Verbindung der Formel

$$R_1 - A - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - CN \qquad (VI),$$

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C=O)R_6$ überführt, oder

f) zur Herstellung einer Verbindung der Formel I, worin $R_4$ freies Hydroxy bedeutet, ein Epoxid der Formel

$$R_1 - A - \overset{R_2}{\overset{|}{N}} - \underset{\underset{R_3}{|}}{CH} - \overset{O}{\widehat{CH - CH}} - \overset{R_5}{\overset{|}{CH}} - \overset{O}{\overset{\|}{C}} - R_6 \qquad (VII),$$

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, oder

g) zur Herstellung einer Verbindung der Formel I, worin $R_1$ einen Rest der Formel

$$R^a - \underset{\underset{(O)_m}{\|}}{S} - (CH_2)_n - \underset{\underset{\underset{\underset{R^b}{|}}{(CH_2)_q}}{|}}{CH} - (CH_2)_p - \underset{\underset{O}{\|}}{C} - \qquad (Ia)$$

und m 0 oder 2, n 1 und p 0 bedeuten, eine Verbindung der Formel $R^a-S(O)_mH$ oder ein Salz davon an eine Verbindung der Formel

$$R^b-(CH_2)_q - \underset{\underset{CH_2}{\|}}{C} - \overset{O}{\overset{\|}{C}} - A - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - \overset{O}{\overset{\|}{C}} - R_6 \qquad (VIII),$$

worin die Substituenten die genannten Bedeutungen haben und freie funktionelle Gruppen gegebenenfalls in geschützter Form vorliegen, addiert, oder

h) zur Herstellung einer Verbindung der Formel I, worin $R_1$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia)$$

und p 0 bedeutet, eine Verbindung der Formel

$$R^a - \underset{(O)_m}{S} - (CH_2)_n - CH_2 - \underset{O}{\underset{\|}{C}} - A - \overset{R_2}{\overset{|}{N}} - \underset{R_3}{\underset{|}{CH}} - \overset{R_4}{\overset{|}{CH}} - CH_2 - \overset{R_5}{\overset{|}{CH}} - \overset{O}{\overset{\|}{C}} - R_6 \qquad (IX),$$

worin die Substituenten die genannten Bedeutungen haben und freie funktionelle Gruppen gegebenenfalls in geschützter Form vorliegen, mit einer den Rest $R^b$-$(CH_2)q$- einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

Die Erfindung betrifft auch die nach irgendeinem der oben genannten Verfahren erhältlichen anderen Verbindungen als Verbindungen der Formel I (Nebenprodukt), sowie Verbindungen der Formel I und ihre Salze, welche nach einem anderen Verfahren als einem der weiter vorn genannten hergestellt werden.

Verfahren a) (Herstellung einer Amidbindung):

Fragmente einer Verbindung der Formel I mit einer endständigen Carboxygruppe, welche mit einem zur Verbindung der Formel I komplementären Fragment unter Bildung einer Amidbindung kondensiert werden können, sind z.B. Verbindungen der Formeln $R_1$-OH, $R_1$-A-OH oder

$$R_1 - A - \overset{R_2}{N} - \underset{R_3}{CH} - \overset{R_4}{CH} - CH_2 - \overset{R_5}{CH} - \overset{O}{\overset{\|}{C}} - OH \;,$$

die von diesen Verbindungen abgeleiteten aktivierten Ester oder reaktionsfähigen Anhydride, ferner reaktionsfähige cyclische Amide. Die reaktionsfähigen Säurederivate können auch in situ gebildet werden.

Aktivierte Ester sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylvinylester (erhältlich z.B. durch Behandeln der entsprechenden Säure mit einem Isoxazoliumreagens; 1,2-Oxazolium- oder Woodward-Methode) oder 1-Niederalkoxyvinylester (erhältlich z.B. durch Behandeln der entsprechenden Säure mit einem Niederalkoxyacetylen; Aethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode) oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol, 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester),

Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u.a. mit Hilfe der Anhydrid- oder Carbodiimid-Methode; Methode der aktivierten Thiolester) oder insbesondere Amino- oder Amidoester (erhältlich z.B. durch Behandeln der entsprechenden Säure mit einer N-Hydroxyamino- bzw. N-Hydroxyamido-Verbindung, z.B. N-Hydroxysuccinimid, N-Hydroxypiperidin, N-Hydroxyphthalimid, N-Hydroxy-5-norbornen- oder norbornan-2,3-dicarbonsäureimid, 1-Hydroxybenztriazol oder 3-Hydroxy-3,4-dihydro-1,2,3-benzotriazin-4-on, z.B. nach der Anhydrid- oder Carbodiimid-Methode; Methode der aktivierten N-Hydroxyester).

Anhydride von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid oder Oxalylchlorid; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin, z.B. 1-Aethoxycarbonyl-2-äthoxy-1,2-dihydrochinolin; Methode der gemischten O-Alkylkohlensäureanhydride), Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenylphosphoramidochloridat erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten

Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimids oder von 1-Diäthylaminopropin; Methode der symmetrischen Anhyride).

Geeignete cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazolen, z.B. 3,5-Dimethylpyrazol (erhältlich z.B. über das Säurehydrazid durch Behandeln mit Acetylaceton; Pyrazolid-Methode).

Zur Verbindung der Formel I komplementäre Fragmente mit einer freien Aminogruppe sind z.B. je nach Bedeutung von $R_6$ ein primäres oder sekundäres Amin, ferner Verbindungen der Formeln

$$H - A - \overset{R_2}{\overset{|}{N}} - \underset{R_3}{\underset{|}{CH}} - \overset{R_4}{\overset{|}{CH}} - CH_2 - \overset{R_5}{\overset{|}{CH}} - \overset{O}{\overset{\|}{C}} - R_6$$

oder $$\overset{R_2}{\overset{|}{HN}} - \underset{R_3}{\underset{|}{CH}} - \overset{R_4}{\overset{|}{CH}} - CH_2 - \overset{R_5}{\overset{|}{CH}} - \overset{O}{\overset{\|}{C}} - R_6 \quad .$$

Die an der Reaktion teilnehmende Aminogruppe in einem zu einer Verbindung der Formel I komplementären Fragment liegt bevorzugt in freier Form vor, insbesondere wenn die damit reagierende Carboxygruppe in reaktionsfähiger Form vorliegt; sie kann aber auch selbst derivatisiert sein, z.B. durch Reaktion mit einem Phosphit, wie Diäthylchlorphosphit, 1,2-Phenylenchlorphosphit, Aethyldichlorphosphit, Aethylenchlorphosphit oder Tetraäthylpyrophosphit. Ein Derivat

eines solchen komplementären Bruchstücks mit einer Aminogruppe ist z.B. auch ein Carbaminsäurehalogenid oder ein Isocyanat, wobei die an der Reaktion teilnehmende Aminogruppe durch Halogencarbonyl, z.B. Chlorcarbonyl, substituiert bzw. als Isocyanatgruppe abgewandelt ist, wobei im letzteren Falle nur Verbindungen der Formel I zugänglich sind, die am Stickstoffatom der durch die Reaktion gebildeten Amidgruppe ein Wasserstoffatom tragen.

Ist das komplementäre Fragment mit einer Aminogruppe ein durch Niederalkyl oder Arylniederalkyl mono- oder disubstituiertes Amin, so stellt auch eine entsprechende Harnstoff-Verbindung ein reaktionsfähiges Derivat dar. Beispielsweise erhält man beim Erhitzen äquimolarer Mengen dieser Harnstoffverbindung und der Komponente mit freier Carboxygruppe entsprechende Verbindungen der Formel I. Ist das komplementäre Fragment Dimethylamin, so ist auch Dimethylformamid ein reaktionsfähiges Derivat.

Funktionelle Gruppen in Ausgangsmaterialien, deren Umsetzung vermieden werden soll, insbesondere Carboxy-, Amino-, Hydroxy-, Mercapto- und Sulfogruppen, können durch geeignete Schutzgruppen geschützt sein, wie sie üblicherweise bei der Synthese von Peptid-Verbindungen, aber auch von Cephalosporinen und Penicillinen verwendet werden. Diese Schutzgruppen können bereits in den Vorstufen vorhanden sein und sollen die betreffenden funktionellen Gruppen gegen unerwünschte Nebenreaktionen wie Acylierungen, Verätherungen, Veresterungen, Oxydationen, Solvolyse etc. schützen. Schutzgruppen können aber auch in den Endstoffen vorhanden sein. Verbindungen der Formel I mit geschützten funktionellen Gruppen können eine höhere metabolische Stabilität aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen, sind beispielsweise in Standardwerken wie in J.F.W. McOmie, "Protective Groups in Organic Chemistry", Plenum Press, London und New York 1973, in Th. W. Greene, "Protective Groups in Organic Synthesis",

Wiley, New York 1981, in "The Peptides"; Band 3 (Herausg. E. Gross und J. Meienhofer), Academic Press, London und New York 1981, sowie in "Methoden der organischen Chemie", Houben-Weyl, 4. Auflage, Bd. 15/I, Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche in 1-Stellung der Niederalkylgruppe verzweigt oder in 1- oder 2-Stellung der Niederalkylgruppe durch geeignete Substituenten substituiert ist.

Eine geschützte Carboxygruppe, welche durch eine Niederalkylgruppe verestert ist, die in 1-Stellung der Niederalkylgruppe verzweigt ist, ist beispielsweise tert-Niederalkoxycarbonyl, z.B. tert-Butoxycarbonyl, oder Arylmethoxycarbonyl mit einem oder zwei Arylresten, worin Aryl unsubstituiertes oder z.B. durch Niederalkyl, z.B. tert-Niederalkyl, wie tert-Butyl, Niederalkoxy, z.B. Methoxy, Hydroxy, Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl.

Eine geschützte Carboxygruppe, welche durch eine Niederalkylgruppe verestert ist, welche in 1- oder 2-Stellung der Niederalkylgruppe durch geeignete Substituenten substituiert ist, ist beispielsweise 1-Niederalkoxyniederalkoxycarbonyl, z.B. Methoxymethoxycarbonyl, 1-Methoxyäthoxycarbonyl oder 1-Aethoxyäthoxycarbonyl, 1-Niederalkylthioniederalkoxycarbonyl, z.B. 1-Methylthiomethoxycarbonyl oder 1-Aethylthioäthoxycarbonyl, Aroylmethoxycarbonyl, z.B. Phenacyloxycarbonyl, 2-Halogenniederalkoxycarbonyl, z.B. 2,2,2-Trichloräthoxycarbonyl, 2-Bromäthoxycarbonyl oder 2-Jodäthoxycarbonyl, sowie 2-Triniederalkylsilylniederalkoxycarbonyl, z.B. 2-Trimethylsilyläthoxycarbonyl.

Eine Carboxygruppe kann auch als organische Silyloxycarbonylgruppe geschützt sein. Eine organische Silyloxycarbonylgruppe ist beispielsweise eine Triniederalkylsilyloxycarbonylgruppe, z.B. Trimethylsilyloxycarbonyl. Das Siliciumatom der Silyloxycarbonylgruppe kann auch durch zwei Niederalkylgruppen, z.B. Methylgruppen, und die Aminogruppe oder die Carboxygruppe eines zweiten Moleküls der Formel I substituiert sein. Verbindungen mit solchen Schutzgruppen lassen sich z.B. mit Dimethylchlorsilan als Silylierungsmittel herstellen.

Eine geschützte Carboxygruppe ist bevorzugt tert-Niederalkoxycarbonyl, z.B. tert-Butoxycarbonyl, Benzyloxycarbonyl, 4-Nitrobenzyloxycarbonyl oder Diphenylmethoxycarbonyl.

Eine Aminogruppe kann z.B. in Form einer Acylamino-, Arylmethylamino-, verätherten Mercaptoamino- oder Silylaminogruppe oder als Azidogruppe geschützt sein.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, z.B. tert-Niederalkoxycarbonyl, wie

tert-Butoxycarbonyl, Arylmethoxycarbonyl mit einem oder zwei Arylresten, die gegebenenfalls, z.B. durch Niederalkyl, z.B. tert-Niederalkyl, wie tert-Butyl, Niederalkoxy, wie Methoxy, Hydroxy, Halogen, wie Chlor, und/oder Nitro, mono- oder polysubstituiertes Phenyl darstellen, z.B. Benzyloxycarbonyl, 4-Nitrobenzyloxycarbonyl, Diphenylmethoxycarbonyl oder Di-(4-methoxyphenyl)-methoxycarbonyl, Aroylmethoxycarbonyl, z.B. Phenacyloxycarbonyl, 2-Halogen-niederalkoxycarbonyl, z.B. 2-Chloräthoxycarbonyl, 2,2,2-Trichloräthoxycarbonyl, 2-Bromäthoxycarbonyl oder 2-Jodäthoxycarbonyl, 2-Triniederalkylsilylniederalkoxycarbonyl, z.B. 2-Trimethlysilyläthoxycarbonyl, oder 2-Triarylsilylniederalkoxycarbonyl, z.B. 2-Triphenylsilyläthoxycarbonyl.

Eine Arylmethylaminogruppe ist z.B. Mono-, Di- oder insbesondere Triphenylmethylamino, z.B. Benzyl-, Diphenylmethyl- oder Tritylamino.

In einer verätherten Mercaptoaminogruppe ist die verätherte Mercaptogruppe in erster Linie substituiertes Arylthio, z.B. 4-Nitrophenylthio.

Eine Silylaminogruppe ist beispielsweise eine Triniederalkylsilylaminogruppe, z.B. Trimethylsilylamino. Das Siliciumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Bevorzugte Aminoschutzgruppen sind Acylreste von Kohlensäurehalbestern, insbesondere tert-Butoxycarbonyl, gegebenenfalls substituiertes Benzyloxycarbonyl, z.B. 4-Nitrobenzyloxycarbonyl, Diphenylmethoxycarbonyl, 2-Halogen-niederalkoxycarbonyl, z.B. 2,2,2-Trichloräthoxycarbonyl, ferner Trityl und Formyl.

Eine Hydroxygruppe kann beispielsweise durch eine durch Halogen, z.B. Chlor, substituierte Niederalkanoylgruppe, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2-Chloräthoxycarbonyl, 2,2,2-Trichloräthoxycarbonyl, 4-Nitrobenzyloxycarbonyl oder Diphenylmethoxycarbonyl. Eine Hydroxygruppe kann ferner durch Triniederalkylsilyl, z.B. Trimethylsilyl oder bevorzugt Dimethyltert-butylsilyl, eine leicht abspaltbare Alkylgruppe, wie tert-Niederalkyl, z.B. tert-Butyl, einen oxa- oder einen thiaaliphatischen oder -cycloaliphatischen Kohlenwasserstoffrest, beispielsweise 1-Niederalkoxyniederalkyl oder 1-Niederalkylthioniederalkyl, z.B. Methoxymethyl, 1-Methoxyäthyl, 1-Aethoxyäthyl, Methylthiomethyl, 1-Methylthioäthyl oder 1-Aethylthioäthyl, oder 2-Oxa- oder 2-Thiacycloalkyl mit 5 - 7 Ringatomen, z.B. 2-Tetrahydrofuryl oder 2-Tetrahydropyranyl, oder ein entsprechendes Thiaanaloges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei benachbarte Hydroxylgruppen können beispielsweise durch eine vorzugsweise substituierte Methylengruppe geschützt sein, z.B. durch Niederalkyliden, z.B. Isopropyliden, Cycloalkyliden, z.B. Cyclohexyliden, oder Benzyliden.

Eine Mercaptogruppe, wie z.B. in Cystein, kann insbesondere durch S-Alkylierung mit gegebenenfalls substituierten Alkylresten, Silylierung, Thioacetalbildung, S-Acylierung oder durch die Bildung asymmetrischer Disulfid-Gruppierungen geschützt sein. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls am Phenylrest, z.B. durch Methoxy, substituiertes Diphenylmethyl, wie 4,4'-Dimethoxydiphenylmethyl, Triphenylmethyl, Trimethylsilyl, Benzylthiomethyl, 2-Tetrahydropyranyl,

Acylaminomethyl, Benzoyl, Benzyloxycarbonyl oder Niederalkylaminocarbonyl, wie Aethylaminocarbonyl, ferner Niederalkylthio, z.B. Methylthio.

Eine Sulfogruppe kann beispielsweise durch Niederalkyl, z.B. Methyl oder Aethyl, durch Phenyl oder als Sulfonamid, beispielsweise als Imidazolid, geschützt sein.

Die Kondensation zur Herstellung der Amidbindung kann in an sich bekannter Weise durchgeführt werden, beispielsweise wie in Standardwerken, wie "Houben-Weyl, Methoden der organischen Chemie", 4. Auflage, Band 15/II, Georg Thieme Verlag, Stuttgart 1974, "The Peptides" (Herausg. E. Gross und J. Meienhofer), Band 1 und 2, Academic Press, London und New York, 1979/1980, oder M. Bodanszky, "Principles of Peptide Synthesis", Springer-Verlag, Berlin 1984, beschrieben.

Die Kondensation kann in Gegenwart eines der üblichen Kondensationsmittel durchgeführt werden. Uebliche Kondensationsmittel sind z.B. Carbodiimide, beispielsweise Diäthyl-, Dipropyl-, N-Aethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Aethyl-5-phenyl-1,2-oxazolium-3'-sulfonat und 2-tert-Butyl-5-methylisoxazoliumperchlorat, oder eine geeignete Acylaminoverbindung, z.B. 2-Aethoxy-1-äthoxycarbonyl-1,2-dihydrochinolin, ferner aktivierte Phosphorsäurederivate, z.B. Diphenylphosphorylazid, Diäthylphosphorylcyanid, Phenyl-N-phenylphosphoramidochloridat, Bis-(2-oxo-3-oxazolidinyl)-phosphinsäurechlorid oder 1-Benzotriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. Aethyldiisopropylamin, oder eine heterocyclische Base, z.B. Pyridin, 4-Dimethylaminopyridin oder bevorzugt N-Methylmorpholin.

Die Kondensation von Säureanhydriden mit Aminen kann z.B. in Gegenwart von anorganischen Carbonaten, z.B. Alkalimetallcarbonaten oder -hydrogencarbonaten, wie Natrium- oder Kaliumcarbonat oder -hydrogencarbonat (üblicherweise zusammen mit einem Sulfat), erfolgen.

Die Kondensation wird vorzugsweise in einem inerten, polaren, aprotischen, vorzugsweise wasserfreien, Lösungsmittel oder Lösungsmittelgemisch durchgeführt, beispielsweise in einem Carbonsäureamid, z.B. Formamid oder Dimethylformamid, einem halogenierten Kohlenwasserstoff, z.B. Methylenchlorid, Tetrachlorkohlenstoff oder Chlorbenzol, einem Keton, z.B. Aceton, cyclischen Aether, z.B. Tetrahydrofuran, einem Ester, z.B. Essigsäureäthylester, oder einem Nitril, z.B. Acetonitril, oder in Mischungen davon, gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre.

Reaktionsfähige Säurederivate können auch in situ gebildet werden. So kann man z.B. N,N'-disubstituierte Amidinoester in situ bilden, indem man das Gemisch des Fragments mit freier Carboxygruppe und des komplementären Fragments mit einer Aminogruppe in Gegenwart eines geeigneten disubstituierten Carbodiimids, z.B. Dicyclohexylcarbodiimid, umsetzt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines disubstituierten Carbodiimids, z.B. Dicyclohexylcarbodiimid, und eines N-Hydroxylamins oder N-Hydroxyamids, z.B. N-Hydroxybenztriazol, N-Hydroxysuccinimid oder N-Hydroxy-norbornan-2,3-dicarbonsäureimid, gegebenenfalls in Anwesenheit einer geeigneten Base, z.B. 4-Dimethylaminopyridin, N-Methylmorpholin oder Aethyldiisopropylamin, umsetzt.

Die Kondensation einer Carbonsäure $R_1$-A-OH mit dem entsprechenden, zur Verbindung der Formel I komplementären Fragment mit einer freien Aminogruppe kann auch auf an sich bekannte Weise mit Hilfe von Enzymen erreicht werden, z.B. wie von H.-D. Jakubke et al. in Angewandte Chemie 97, 79 (1985) beschrieben. Als Enzyme sind beispielsweise Thermolysin, Carboxypeptidase Y, Papain, Chymotrypsin, Trypsin oder Pepsin geeignet. Die Reaktion wird vorzugsweise in Wasser oder in Gemischen von Wasser mit organischen Lösungsmitteln, z.B. mit Niederalkanolen, wie Aethanol, Dimethylformamid, Dimethylsulfoxid, Aethern, wie Tetrahydrofuran, Dioxan oder 1,2-Dimethoxyäthan, Aceton, Acetonitril oder Polyalkoholen, z.B. Aethylenglykol, Di-, Tri- oder Poly-äthylenglykol, aber auch mit nicht mischbaren organischen Lösungsmitteln, z.B. Methylenchlorid oder Essigsäureäthylester, bei einem pH von 5 bis 8, bevorzugt um den Neutralpunkt, bei Temperaturen zwischen 0°C und 50°C durchgeführt. Die Lösungsmittel und die Reaktionsbedingungen werden vorzugsweise so gewählt, dass die gewünschte Verbindung ausfällt oder in die nicht-mischbare organische Phase extrahiert wird und so dem Reaktionsgleichgewicht entzogen wird. Es ist auch möglich, die Kondensation mit auf einem geeigneten Träger immobilisierten Enzymen, wie sie oben genannt sind, in den genannten organischen Lösungsmitteln im Gemisch mit wenig Wasser durchzuführen.

Verfahren b) (Reduktion einer Ketogruppe):

In einem Ausgangsmaterial der Formel II sind funktionelle Gruppen mit Ausnahme der zu reduzierenden Ketogruppe gegebenenfalls durch eine der unter Verfahren a) genannten Schutzgruppen geschützt.

Zur Reduktion der Ketogruppe in einer Verbindung der Formel II eignen sich solche Reduktionsmittel, die unter den Reaktionsbedingungen des Verfahrens eine isolierte Ketogruppe selektiv oder schneller als die in Verbindungen der Formel I vorhandenen Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, ferner Zinkborhydrid, oder geeignete Aluminiumhydride, wie Alkalimetallniederalkoxyaluminiumhydride mit voluminösen Resten, z.B. Lithium-tris-tert-butoxyaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, oder nach Meerwein-Ponndorf-Verley mit Hilfe von Aluminiumalkanolaten, bevorzugt Aluminium-2-propanolat oder -äthanolat, durchgeführt werden.

Die Reduktion kann vorzugsweise mit stöchiometrischen Mengen oder einem sinnvoll bemessenen Ueberschuss des Reduktionsmittels in einem inerten Lösungsmittel bei Temperaturen zwischen -80°C und dem Siedepunkt des Lösungsmittels, vorzugsweise zwischen -20°C und +100°C, wenn nötig unter Schutzgas, z.B. Stickstoff oder Argon, durchgeführt werden. Ein Ueberschuss des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B. Methanol, Aethanol oder Isopropanol; bei Verwendung der anderen Reduktionsmittel die unter Verfahren a) genannten polaren, aprotischen Lösungsmittel, z.B. Tetrahydrofuran.

<u>Verfahren c) (Addition einer metallorganischen Verbindung):</u>

In einem Ausgangsmaterial der Formel III sind funktionelle Gruppen mit Ausnahme der Aldehydgruppe gegebenenfalls durch die unter Verfahren a) genannten Schutzgruppen geschützt. Ebenso sind vorhandene funktionelle Gruppen in einer Verbindung der Formel IV geschützt.

In einer Verbindung der Formel IV ist ein Metallradikal -M beispielsweise -Li oder -MgHal, z.B. -MgCl, -MgBr oder -MgJ.

Die Umsetzung einer Verbindung der Formel III mit einer Verbindung der Formel IV erfolgt in üblicher Weise in einem wasserfreien, inerten, aprotischen Lösungsmittel, z.B. in einem Aether, wie Diäthyläther oder Tetrahydrofuran, oder einem Kohlenwasserstoff, wie Benzol oder Toluol, oder Gemischen davon, gegebenenfalls unter Kühlen, insbesondere nach Beginn der Reaktion, z.B. bis etwa -30°C, oder unter Erwärmen, z.B. bis zur Siedetemperatur des Reaktionsgemisches, gegebenenfalls in einer Inertgas-, z.B. Stickstoffatmosphäre. Eine bevorzugte Ausführungsform des Verfahrens ist die Umsetzung des Aldehyds der Formel III mit einem Ueberschuss der Lithiumverbindung der Formel IV.

Die Hydrolyse des Additionsprodukts erfolgt mit $H^+$-Ionen liefernden Lösungsmitteln, z.B. Wasser (Eis-Wasser-Gemisch) oder verdünnten, wässrigen Säuren, z.B. verdünnten Mineralsäuren, wie verdünnter, wässriger Schwefelsäure, oder verdünnten organischen Säuren, z.B. verdünnter, wässriger Essigsäure.

Die Umsetzung einer Verbindung der Formel III kann auch mit einer *in situ* hergestellten Verbindung der Formel IV erfolgen, welche man z.B. aus dem entsprechenden Halogenid, z.B. Chlorid, durch Umsetzung mit einem Metallierungsmittel, z.B. Magnesium, Lithium oder tert-Butyllithium, erhält.

Verfahren d) (nukleophile Substitution):

In einem Ausgangsmaterial der Formel V sind funktionelle Gruppen gegebenenfalls durch die unter Verfahren a) genannten Schutzgruppen geschützt.

In einer Verbindung der Formel V ist die nukleofuge Abgangsgruppe X insbesondere mit einer starken anorganischen oder organischen Säure verestertes Hydroxy, wie mit einer Mineralsäure, z.B. Halogenwasserstoffsäure, wie Chlor-, Brom- oder Jodwasserstoffsäure, Schwefelsäure oder Halogenschwefelsäure, z.B. Fluorschwefelsäure, mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, oder mit Stickstoffwasserstoffsäure verestertes Hydroxy.

Ein den Substituenten $R_4$ in nukleophiler Form einführendes Reagens ist abhängig von der Bedeutung von $R_4$ eine Hydroxid-haltige Base, z.B. Natrium- oder Kaliumhydroxid ($R_4$ = OH), ein Alkohol, z.B. Methanol oder Aethanol ($R_4$ = veräthertes Hydroxy) oder das Salz einer Carbonsäure, z.B. Silberacetat ($R_4$ = verestertes Hydroxy).

Vorzugsweise werden die Reaktionsbedingungen so gewählt, dass die Reaktion im wesentlichen als nukleophile Substitution zweiter Ordnung ($S_N2$) abläuft. Beispielsweise kann man eine Verbindung der Formel V, worin X für eine Abgangsgruppe mit hoher Polarisierbarkeit der Elektronenhülle, z.B. für Jod, steht, in einem polaren aprotischen Lösungsmittel, z.B. Aceton, Acetonitril, Nitromethan, Dimethylsulfoxid oder Dimethylformamid, mit dem Silbersalz einer Carbonsäure, z.B. Silberacetat, umsetzen. Die Reaktion mit einer Hydroxidhaltigen Base wird vorzugsweise in Wasser, dem gegebenenfalls als Lösungsvermittler ein organisches Lösungsmittel, z.B. Aethanol, Tetrahydrofuran oder Aceton, beigemischt ist, und die Reaktion mit einem Alkohol in einem Ueberschuss dieses Alkohols, gegebenenfalls in Gegenwart eines der obengenannten polaren aprotischen Lösungsmittel, durchgeführt. Die Substitutionsreaktion wird gegebenenfalls bei erniedrigter oder erhöhter Temperatur, z.B. in einem Temperaturbereich von etwa -40°C bis etwa +100°C, bevorzugt von etwa -10°C bis etwa +50°C, und gegebenenfalls unter Inertgas-, z.B. Stickstoffatmosphäre, durchgeführt.

Verfahren e) (Ueberführung einer Cyanogruppe in eine Amidgruppe):

In einem Ausgangsmaterial der Formel VI sind funktionelle Gruppen gegebenenfalls durch die unter a) genannten Schutzgruppen geschützt.

Die Ueberführung einer Verbindung der Formel VI in eine Verbindung der Formel I kann durch eine Ritter-Reaktion oder über Carbonsäureesterimid-Salze erfolgen.

Bei der Ritter-Reaktion setzt man die Nitrile in Gegenwart einer starken Säure, beispielsweise 85-90%iger Schwefelsäure, oder auch Polyphosphorsäure, Fluorwasserstoff, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol, meist ohne Lösungsmittel oder beispielsweise in Eisessig.

In einer Variante der Ritter-Reaktion wird ein Nitril der Formel VI mit einem Olefin und Quecksilber(II)-nitrat umgesetzt und die Organoquecksilberverbindung anschliessend mit Natriumborhydrid zu einer N-substituierten Verbindung der Formel I reduziert.

Durch säurekatalysierte, bevorzugt Chlorwasserstoff-katalysierte, Anlagerung von Alkoholen an die Nitrile der Formel VI erhält man Carbonsäureesterimide, die durch thermische Umlagerung bei Temperaturen oberhalb von etwa 80°C Amide der Formel I ergeben.

Verfahren f) (Reduktion des Epoxids):

In einem Ausgangsmaterial der Formel VII sind funktionelle Gruppen gegebenenfalls durch die unter Verfahren a) genannten Schutzgruppen geschützt.

Es können solche Reduktionsmittel verwendet werden, die unter den Reaktionsbedingungen des Verfahrens die Epoxygruppe selektiv oder schneller als die vorhandenen Amidgruppen reduzieren und das Epoxid so öffnen, dass ein genügend und möglichst grosser Anteil der Reaktionsprodukte die neugebildete Hydroxygruppe in der der Formel I entsprechenden Position trägt. Beispiele für solche selektive Reduktionsmittel sind Lithiumborhydrid oder Natriumcyanborhydrid/Bortrifluoridätherat. Mit dem letztgenannten Reagens lässt sich die Reaktion z.B. so durchführen, dass man zu 1 Mol der Verbindung der Formel VII und einem Ueberschuss, z.B. 1,4-3 Mol, Natriumcyanborhydrid in Tetrahydrofuran bei erhöhter Temperatur, z.B. unter Rückfluss, eine Lösung von Bortrifluoridätherat, $BF_3 \cdot O(C_2H_5)_2$, in Tetrahydrofuran so zugibt, dass der pH-Wert der Reaktionslösung in der Nähe des Umschlagpunktes des ebenfalls zugegebenen Indikators Bromkresolgrün gehalten wird. Die Reduktion mit Lithiumborhydrid wird vorzugsweise in einem Aether, z.B. Tetrahydrofuran, 1,2-Dimethoxyäthan oder Diäthylenglykoldimethyläther, bei Temperaturen zwischen Raumtemperatur und Rückflusstemperatur durchgeführt.

Verfahren g) (Addition an ein Acrylamid)

Eine Verbindung der Formel $R^a-S(O)_mH$ ist entweder ein Thiol der Formel $R^a-SH$ oder eine Sulfinsäure der Formel $R^a-SO_2H$.

In einem Ausgangsmaterial der Formel VIII sind funktionelle Gruppen gegebenenfalls durch die unter Verfahren a) genannten Schutzgruppen geschützt. Ebenso sind vorhandene funktionelle Gruppen in der Verbindung der Formel $R^a-S(O)_mH$ gegebenenfalls geschützt.

Geeignete Salze der Verbindung der Formel $R^a-S(O)_mH$ sind beispielsweise Alkalimetallsalze, z.B. Natrium- oder Kaliumsalze.

Die Addition einer Verbindung der Formel $R^a-S(O)_mH$ oder eines geeigneten Salzes davon an eine Verbindung der Formel VIII erfolgt in üblicher Weise in einem inerten, polaren Lösungsmittel, z.B. in einem polaren Aether, z.B. Tetrahydrofuran, Dioxan oder Dimethoxyäthan, einem Niederalkanol, z.B. Methanol, Aethanol oder Isopropanol, oder einem dipolar aprotischen Lösungsmittel, z.B. Dimethylformamid, Dimethylacetamid, Dimethylsulfoxid, Hexamethylphosphorsäuretriamid, N-Methylpyrrolidon oder Acetonitril, gegebenenfalls auch in Gemischen der genannten Lösungsmittel untereinander oder mit Wasser, bei Temperaturen zwischen ca. -30°C und dem Siedepunkt des jeweiligen Lösungsmittel, z.B. zwischen 0° und +80°C, beispielsweise um 50°C.

Anstelle einer Sulfinsäure $R^a-SO_2H$ werden vorzugsweise deren Salze eingesetzt, beispielsweise das Natrium- oder Kaliumsalz.

Ein Salz eines Thiols der Formel $R^a-SH$ kann auch in situ gebildet werden, beispielsweise durch Zugabe einer geeigneten Base, z.B. Alkalimetallhydroxid, wie Natrium- oder Kaliumhydroxid, oder Alkalimetallhydrid, z.B. Natriumhydrid, wobei allerdings nur wasserfreie Lösungsmittel verwendet werden können. Es ist auch möglich, die Additionsreaktion mit einem freien Thiol der Formel $R^a-SH$ in Gegenwart einer organischen Base, z.B. eines tertiären Amins, z.B. Triäthylamin, N-Methylmorpholin, Dimethylanilin, Diazabicyclo-[5.4.0]undec-7-en oder Diazabicyclo[4.3.0]non-5-en, zu führen.

Verfahren h) (Alkylierung im Acylrest)

In einem Ausgangsmaterial der Formel IX sind funktionelle Gruppen gegebenenfalls durch die unter Verfahren a) genannten Schutzgruppen geschützt.

Eine den Rest $R^b-(CH_2)q-$ einführende Verbindung ist beispielsweise das entsprechende Halogenid, z.B. Chlorid, Bromid oder Iodid, oder ein reaktiver Ester des entsprechenden Alkohols, z.B. ein Sulfonsäureester, wie der Methansulfonsäureester oder p-Toluolsulfonsäureester.

Zur Alkylierung wird die Verbindung der Formel IX vorzugsweise nach den üblichen Methoden mit einer starken, nicht-nukleophilen Base in das entsprechende Anion übergeführt, beispielsweise mit dem Lithium-oder Kaliumsalz eines sterisch gehinderten sekundären Amins, z.B. mit Lithiumdiisopropylamid, Lithiumcyclohexylisopropylamid, Lithium-2,2,6,6-tetramethylpiperidid, Lithium- oder Kalium-bis(trimethylsilyl)-amid oder dergleichen. Die Deprotonierung mit der Base wird vorzugsweise bei tiefen Temperaturen, z.B. zwischen -100°C und -50°C in einem inerten polaren Lösungsmittel, z.B. in einem polaren Aether, z.B. Tetrahydrofuran, Dioxan oder Dimethoxyäthan, gegebenenfalls gemischt mit einem Kohlenwasserstoff, z.B. Hexan oder Toluol, und/oder Hexamethylphosphorsäuretriamid oder N,N'-Dimethyl-N,N'-propylenharnstoff, durchgeführt. Enthält die Verbindung der Formel IX noch weitere, leichter deprotonierbare Methylen- oder Methingruppen, z.B. solche neben der Sulfinyl- oder Sulfonylgruppe, so werden vorzugsweise zwei oder mehr Aequivalente der Base eingesetzt, um das entsprechende Di- oder Polyanion zu erhalten.

Die dermassen deprotonierte Verbindung der Formel IX wird mit dem den Rest $R^b-(CH_2)q-$ einführenden Alkylierungsmittel bevorzugt in situ bei tiefen Temperaturen, z.B. bei -78° bis -30°C, und nachfolgendem Aufwärmen auf Raumtemperatur oder leicht erhöhter Temperatur, z.B. auf 50°C, im gleichen Lösungsmittel oder Lösungsmittelgemisch umgesetzt.

Ist in einer Verbindung der Formel IX n = 0, so kann die Alkylierung unter wesentlich milderen Bedingungen durchgeführt werden, beispielsweise in einem der oben genannten Lösungsmittel oder anderen polaren Lösungsmitteln, z.B. Dimethylsulfoxid, Dimethylformamid oder Acetonitril, mit dem den Rest $R^b(CH_2)q-$ einführenden Alkylierungsmittel bei Temperaturen zwischen -30°C und etwa Raumtemperatur und einem tertiären Amin, z.B. Triäthylamin, N-Methylmorpholin, Diazabicyclo[5.4.0]undec-7-en oder Diazabicyclo[4.3.0]non-5-en, oder einer unlöslichen anorganischen Base, z.B. Kaliumcarbonat oder Natriumhydrid, oder einem Alkoholat, z.B. Kalium-tert-butanolat. Geeignet ist auch die Alkylierung unter Phasentransfer-Bedingungen, d.h. in einem zweiphasigen Gemisch aus wässriger Base, z.B. Natronlauge, und einem nicht mischbaren organischen Lösungsmittel, z.B. Methylenchlorid oder Toluol, und einem Phasentransfer-Katalysator, z.B. einem Ammonium- oder Phosphoniumsalz.

Verfahren i) (Nachoperationen):

In einer erhältlichen Verbindung der Formel I, worin $R_1$, A, $R_2$, $R_3$, $R_4$, $R_5$ und $R_6$ die genannten Bedeutungen haben, kann man eine Carboxamidgruppe substituieren, eine in freier oder in reaktionsfähiger Form vorliegende Carboxygruppe verestern bzw. eine veresterte Carboxygruppe in eine Carboxy- oder eine Carboxamidgruppe überführen.

Die Substitution einer Carboxamidgruppe oder einer anderen Aminogruppe erfolgt z.B. durch Alkylierung.

Geeignete Mittel zur Alkylierung einer Carboxamidgruppe in einer Verbindung der Formel I sind z.B. Diazoverbindungen, z.B. Diazomethan. Man kann Diazomethan in einem inerten Lösungsmittel zersetzen, wobei das gebildete freie Methylen mit der Carboxamidgruppe in der Verbindung der Formel I reagiert. Die Zersetzung von Diazomethan erfolgt vorzugsweise katalytisch, z.B. in Gegenwart eines Edelmetalls in fein verteilter Form, z.B. Kupfer, oder eines Edelmetallsalzes, z.B. Kupfer(I)-chlorid oder Kupfer(II)-sulfat.

Weitere Alkylierungsmittel sind die in der Deutschen Offenlegungsschrift 2 331 133 genannten Alkylierungsmittel, z.B. Alkylhalogenide, Sulfonsäureester, Meerweinsalze oder 1-substituierte 3-Aryltriazene. welche man unter den dort genannten Reaktionsbedingungen mit einer Verbindung der Formel I mit einer Carboxamidgruppe umsetzen kann.

Zur Veresterung einer Carboxygruppe in einer Verbindung der Formel I kann man die freie Säure verwenden oder die freie Säure in eines der unter Verfahren a) genannten reaktionsfähigen Derivate überführen und mit einem Alkohol umsetzen, oder man kann die freie Säure oder

ein reaktionsfähiges Salz, z.B. das Cäsiumsalz, mit einem reaktionsfähigen Derivat eines Alkohols umsetzen. Beispielsweise kann man das Cäsiumsalz einer Carbonsäure mit dem Halogenid eines Alkohols umsetzen.

Die Veresterung einer Carboxygruppe kann mit den für die Substitution der Carboxamidgruppe oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Zur Ueberführung einer veresterten Carboxygruppe in einer Verbindung der Formel I in eine freie Carboxygruppe kann eine der unter Verfahren a), Abspaltung der Carboxyschutzgruppen, beschriebenen Methoden oder gewünschtenfalls eine alkalische Verseifung nach den im Organikum, 15. Auflage, VEB Deutscher Verlag der Wissenschaften, Berlin (Ost) 1976, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für solche Umsetzungen genannten Reaktionsbedingungen erfolgen.

In einer erhältlichen Verbindung der Formel I, worin die Substituenten die genannten Bedeutungen haben und mindestens eine freie Hydroxygruppe vorhanden ist und die übrigen funktionellen Gruppen gegebenenfalls in geschützter Form vorliegen, kann man die freie Hydroxygruppe, z.B. die Hydroxygruppe $R_4$, veräthern oder verestern.

Die Verätherung dieser Hydroxygruppe kann mit den oben genannten Alkylierungsmitteln und unter den gleichen Reaktionsbedingungen erfolgen, z.B. mit Diazomethan, Alkylhalogeniden, Sulfonsäureestern, Meerweinsalzen, 1-substituierten 3-Aryltriazenen etc.

Die Veresterung der freien Hydroxygruppe kann mit den üblichen Acylierungsmitteln und den üblichen im Organikum angegebenen Reaktionsbedingungen erfolgen, z.B. mit Essigsäureanhydrid.

Die genannten Alkylierungsreaktionen, Verätherungen, Veresterungen etc. können statt im Endstoff auch in einem Ausgangsmaterial entsprechend durchgeführt werden.

In einer erhältlichen Verbindung der Formel I kann man eine Thiogruppe zu einer Sulfinyl- oder Sulfonylgruppe oder eine Sulfinylgruppe zu einer Sulfonylgruppe oxidieren.

Die Oxidation zur Sulfonylgruppe kann mit den meisten der üblichen Oxidationsmittel durchgeführt werden. Bevorzugt verwendet man solche Oxidationsmittel, die die Thiogruppe oder Sulfinylgruppe selektiv in Gegenwart anderer funktioneller Gruppen der Verbindung der Formel I, z.B. der Amidfunktion und der Hydroxygruppe, oxidieren, beispielsweise aromatische oder aliphatische Peroxycarbonsäuren, z.B. Perbenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffe, z.B. Methylenchlorid oder Chloroform, Aether, Essigester oder dergleichen, bei Temperaturen zwischen -78°C und Raumtemperatur, z.B. zwischen -20°C und +10°C, bevorzugt um 0°C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxomonosulfat in Niederalkanol/Wasser-Mischungen, z.B. Methanol-Wasser oder Aethanol-Wasser, oder in wässriger Essigsäure bei Temperaturen zwischen -70°C und +30°C, z.B. zwischen -20°C und Raumtemperatur, ferner Natriummetaperjodat in Methanol oder Methanol-Wasser-Gemischen bei Temperaturen zwischen 0°C und 50°C, z.B. um Raumtemperatur.

Für die Oxidation der Thiogruppe zur Sulfinylgruppe werden selektive Oxidationsmittel in äquimolaren Mengen oder nur geringem Ueberschuss unter kontrollierten Reaktionsbedingungen verwendet, um eine Ueberoxidation zur Sulfonylgruppe zu vermeiden. Geeignet sind beispielsweise Natriummetaperiodat in Methanol oder Methanol-Wasser-Gemischen bei Temperaturen zwischen -15°C und Raumtemperatur, z.B. um 0°C, m-Chlorperbenzoesäure in Methylenchlorid, Chloroform oder Essigester bei Temperaturen zwischen -78°C und 10°C, bevorzugt zwischen -30°C und 0°C, ferner tert-Butylhypochlorit in Niederalkanolen, z.B. Methanol, oder Wasserstoffperoxid in Aceton oder Essigsäure bei Temperaturen um 0°C, oder das oben genannte Kaliumperoxomonosulfat bei tiefen Temperaturen.

In einer erhältlichen Verbindung der Formel I mit einer Sulfinylgruppe kann man diese Gruppe zu einer Thiogruppe reduzieren. Bevorzugt sind selektive Reduktionsmittel, die andere funktionelle Gruppen der Verbindung der Formel I, z.B. die Amidfunktion, unverändert lassen. Beispiele für solche selektive Reduktionsmittel sind Dichlorboran, das vorzugsweise in Tetrahydrofuran oder Dimethoxyäthan bei Temperaturen zwischen -30°C und +10°C eingesetzt wird, Triphenylphosphin in siedendem Tetrachlorkohlenstoff, Trichlorsilan oder Hexachlordisilan, Eisenpentacarbonyl, ferner Natriumhydrogensulfit in wässrig-alkoholischen Lösungsmitteln, z.B. Wasser-Methanol, Wasser-Aethanol oder auch Wasser-Tetrahydrofuran, bei Temperaturen zwischen -10°C und +50°C, ferner Natriumborhydrid in Gegenwart von Kobalt(II)chlorid oder auch Wasserstoff in Gegenwart von katalytischen Mengen Palladium, z.B. Palladium/Kohle in siedendem Aethanol.

Gewünschtenfalls kann in einer erhältlichen Verbindung der Formel I eine Sulfonylgruppe zu einer Thiogruppe reduziert werden, beispielsweise mit Diisobutylaluminiumhydrid in Aether oder Tetrahydrofuran.

In einer erhältlichen Verbindung der Formel I mit einer Sulfonamidgruppe kann diese in der für Carboxamidgruppen beschriebenen Art und Weise alkyliert oder mit Säure oder Alkali zu einer Sulfogruppe

hydrolysiert werden. Beispielsweise kann eine Sulfenamidgruppe mit einem der unter der Oxidation von Thiogruppe zu Sulfonylgruppe genannten Reagentien, z.B. Kaliumperoxomonosulfat, zum Sulfonamid oxidiert und gleich in situ hydrolysiert werden. Eine Sulfonsäureestergruppe kann ebenfalls durch Säure oder Base, beispielsweise wie oben für die Hydrolyse einer Carbonsäureestergruppe beschrieben, in eine Sulfogruppe übergeführt werden.

In einer erhältlichen Verbindung der Formel I mit einer Sulfogruppe kann man diese auf bekannte Art und Weise in eine Sulfonsäureester- oder Sulfonamidgruppe überführen, beispielsweise durch Umwandlung in eine Sulfonsäurehalogenidgruppe und Reaktion mit einem Alkohol, Phenol oder Amin. Eine Sulfonsäureestergruppe wird analog der Carbonsäureestergruppe mit einem Amin in die entsprechende Sulfonamidgruppe umgewandelt.

In einer erhältlichen Verbindung der Formel I, worin eine oder mehrere funktionelle Gruppen geschützt sind, können diese Gruppen, z.B. Carboxy-, Amino-, Hydroxy-, Mercapto- und/oder Sulfogruppen, in an sich bekannter Weise, mittels Solvolyse, insbesondere Hydrolyse, gegebenenfalls enzymatischer Hydrolyse, Alkoholyse oder Acidolyse, oder mittels Reduktion, insbesondere Hydrogenolyse, oder chemischer Reduktion, gegebenenfalls stufenweise oder gleichzeitig, freigesetzt werden. Die Abspaltung der Schutzgruppen ist in den weiter vorn im Abschnitt "Schutzgruppen" genannten Standardwerken beschrieben.

Beispielsweise kann man geschütztes Carboxy, z.B. tert-Niederalkoxycarbonyl, in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl, durch Behandeln mit einer geeigneten Säure, z.B. Ameisensäure oder Trifluoressigsäure, gegebenenfalls unter Zugabe einer nukleophilen Verbindung, z.B. Phenol oder Anisol, in freies Carboxy überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines

Palladiumkatalysators, freigesetzt werden. Ferner kann man geeignet substituiertes Benzyloxycarbonyl, wie 4-Nitrobenzyloxycarbonyl, auch durch Reduktion, z.B. durch Behandeln mit einem Alkalimetall-dithionit, z.B. Natrium-dithionit, mit einem reduzierenden Metall, z.B. Zink, oder einem reduzierenden Metallsalz, wie einem Chrom-IIsalz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlormandelsäure oder Weinsäure, oder eines Alkohols oder Thiols, wobei man vorzugsweise Wasser zugibt, in freies Carboxy überführen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Aroylmethoxycarbonyl in freies Carboxy umwandeln. Aroylmethoxycarbonyl kann ebenfalls durch Behandeln mit einem nukleophilen, vorzugsweise salzbildenden Reagens, wie Natriumthiophenolat oder Natriumjodid, gespalten werden. 2-Triniederalkylsilylniederalkoxycarbonyl kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyäthers ("Kronenäther"), oder mit einem Fluorid einer organischen quaternären Base, wie Tetraniederalkylammoniumfluorid oder Triniederalkylarylammoniumfluorid, z.B. Tetraäthylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy übergeführt werden. Mit einer organischen Silylgruppe, wie Triniederalkylsilyl, z.B. Trimethylsilyl, verestertes Carboxy kann in üblicher Weise solvolytisch, z.B. durch Behandeln mit Wasser, einem Alkohol oder Säure, oder ausserdem einem Fluorid, wie oben beschrieben, freigesetzt werden. Verestertes Carboxy kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Jodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wässriger Essigsäure, gespalten werden. Aroylmethoxycarbonylamino kann auch durch Behandeln mit einem nukleophilen, vorzugsweise salzbildenden Reagens, wie Natriumthiophenolat, und 4-Nitrobenzyloxycarbonylamino auch durch Behandeln mit einem Alkalimetall-, z.B. Natrium-dithionit, gespalten werden. Gegebenenfalls substituiertes Diphenylmethoxycarbonylamino, tert-Niederalkoxycarbonylamino oder 2-Triniederalkylsilylniederalkoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschliessende Solvolyse, wie Alkoholyse oder Hydrolyse, des entstandenen Substitutionsprodukts freigesetzt werden. Eine durch 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 30°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert-Niederalkyl oder durch einen 2-oxa- oder 2-thia-aliphatischen oder -cycloaliphatischen Kohlenwasserstoffrest geschützte Gruppe durch Acidolyse, z.B. durch Behandeln mit einer Mineralsäure oder einer starken Carbonsäure, z.B. Trifluoressigsäure, freigesetzt. Eine Silylgruppe, z.B. eine Trimethylsilyl- oder tert-Butyldimethylsilylgruppe, wird ebenfalls durch Acidolyse, z.B. durch Mineralsäure, bevorzugt Fluorwasserstoffsäure, oder eine starke Carbonsäure abgespalten. 2-Halogenniederalkoxycarbonyl wird durch die obengenannten Reduktionsmittel, z.B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Hydrolyse, z.B. in Gegenwart einer Mineralsäure oder einer starken organischen Säure, freigesetzt werden.

Eine als Sulfonsäureester oder Sulfonamid geschützte Sulfogruppe wird beispielsweise durch saure Hydrolyse, z.B. in Gegenwart von Mineralsäure, oder bevorzugt durch basische Hydrolyse, z.B. mit Alkalimetallhydroxid oder Alkalimetallcarbonat, beispielsweise Natriumcarbonat, freigesetzt.

Salze von Verbindungen der Formel I mit salzbildenden Gruppen können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Aethylhexansäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche z.B. eine freie Carboxygruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen übergeführt werden: Metall- und Ammoniumsalze z.B. durch Behandeln mit geeigneten Säuren, Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

An einzelnen Chiralitätszentren in einer Verbindung der Formel I, z.B. dem CH-$R_4$-C-Atom, kann die Konfiguration gezielt umgekehrt werden. Beispielsweise kann man die Konfiguration am CH-$R_4$-C-Atom durch nukleophile Substitution zweiter Ordnung gemäss Verfahren d) nach Ueberführung der Gruppe $R_4$ in eine nucleofuge Abgangsgruppe X und Reaktion mit einem den gleichen Substituenten $R_4$ einführenden Reagens umkehren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Pharmazeutische Präparate:

Die pharmakologisch verwendbaren Verbindungen der vorliegenden Erfindung können z.B. zur Herstellung von pharmazeutischen Präparaten verwendet werden, welche eine wirksame Menge des Wirkstoffs zusammen oder im Gemisch mit einer signifikanten Menge von anorganischen oder organischen, festen oder flüssigen, pharmazeutisch verwendbaren Trägerstoffen enthalten.

Bei den erfindungsgemässen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, Alter und dem individuellen Zustand, der zu behandelnden Krankheit sowie von der Applikationsweise ab.

Die an Warmblüter, z.B. Menschen von etwa 70 kg Körpergewicht, zu verabreichenden Dosismengen liegen zwischen etwa 3 mg und etwa 3 g, vorzugsweise zwischen etwa 10 mg und etwa 1 g, z.B. bei ungefähr 300 mg pro Person und Tag, verteilt auf vorzugsweise 1 bis 3 Einzeldosen, die z.B. gleich gross sein können. Ueblicherweise erhalten Kinder die halbe Dosis von Erwachsenen.

Die neuen pharmazeutischen Präparate enthalten von etwa 1 % bis etwa 95 %, vorzugsweise von etwa 20 % bis etwa 90 % des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffs, daneben auch Suspensionen, und zwar insbesondere isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder

Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8 - 22, besonders 12 - 22, Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren wie z.B. Oelsäure, Elaidinsäure, Erucasäure, Brasidinsäure oder Linolsäure, enthalten. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Aethanol, Propanol, Butanol oder Pentanol oder deren Isomere, vor allem aber Glycol oder Glyzerin. Als Fettsäureester sind daher beispielsweise zu nennen: Aethyloleat, Isopropylmyristat, Isopropylpalmitat, "Labrafil M 2735" (Polyoxyäthylenglyzerintrioleat der Firma Gattefossé, Paris), "Myglyol 812" (Triglyzerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl und vor allem Erdnussöl.

Die Herstellung der Injektionspräparate erfolgt in üblicher Weise unter sterilen Bedingungen, ebenso das Abfüllen in Ampullen oder Vialen sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch

bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Aethylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Ausgangsmaterialien:

Neue Ausgangsmaterialien und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den als bevorzugt aufgeführten Verbindungen gelangt.

Die Ausgangsmaterialien zur Durchführung des Verfahrens a) können nach an sich bekannten Verfahren hergestellt werden, z.B. aus den betreffenden Aminosäuren durch Kondensation analog dem vorstehend beschriebenen Verfahren a). Beispielsweise kann man eine Verbindung der Formel

$$H_2N - \underset{R_3}{CH} - \overset{OH}{CH} - CH_2 - \overset{R_5}{CH} - \overset{O}{\overset{\|}{C}} - R_6 \qquad (X)$$

analog dem in der Europäischen Patentanmeldung EP 143 746 beschriebenen Verfahren herstellen.

Verbindungen der Formel II werden beispielsweise hergestellt, indem man eine Carbonsäure der Formel

$$R_1 - A - \overset{R_2}{N} - \underset{R_3}{CH} - \overset{O}{\overset{\|}{C}} - OH \qquad (XI)$$

oder ein geeignetes funktionelles Derivat davon, worin die Substituenten die genannten Bedeutungen haben und freie funktionelle Gruppen mit Ausnahme der gegebenenfalls abgewandelten Carboxygruppe gegebenenfalls in geschützter Form vorliegen, mit einer Organometallverbindung der Formel IV, worin $R_5$ und $R_6$ die genannten Bedeutungen haben und M ein Metallradikal, z.B. -Li oder -MgHal, wie -MgCl, -MgBr oder -MgJ bedeutet, umsetzt und das gebildete Additionsprodukt solvolysiert.

Geeignete funktionelle Derivate einer Carbonsäure der Formel XI sind beispielsweise das entsprechende Lithiumsalz der Carbonsäure, ein Carbonsäurehalogenid, z.B. Carbonsäurechlorid, ein Anhydrid, z.B. das symmetrische Carbonsäureanhydrid oder ein gemischtes Carbonsäureanhydrid mit einer sterisch gehinderten Carbonsäure, z.B. mit Pivalinsäure, oder ein Thioester, z.B. 2-Pyridylthioester.

Die Umsetzung einer Carbonsäure der Formel XI oder eines geeigneten funktionellen Derivats davon mit einer Verbindung der Formel IV erfolgt in der üblichen Weise, z.B. nach den in Verfahren c) angegebenen Reaktionsbedingungen, gegebenenfalls jedoch unter Kühlung, z.B. bei Temperaturen von ca. -50°C bis ca. 0°C. In einer bevorzugten Ausführungsform des Verfahrens wird ein 2-Pyridylthioester der Carbonsäure der Formel XI mit einer Brommagnesium-Verbindung der Formel IV umgesetzt.

Verbindungen der Formel III können nach an sich bekannten Verfahren hergestellt werden, beispielsweise indem man in einer Verbindung der Formel XI, worin die Substituenten die genannten Bedeutungen haben und freie funktionelle Gruppen gegebenenfalls in geschützter Form vorliegen, die Carboxygruppe nach an sich bekannten Methoden, beispielsweise über den entsprechenden Methyl- oder Aethylester, über ein Imidazolid oder über ein N-Methoxy-N-methylamid, zur Aldehyd-Funktion reduziert.

Verbindungen der Formel IV können beispielsweise durch Umsetzung eines bekannten oder mit an sich bekannten Methoden herstellbaren Halogenids der Formel

$$Hal - CH_2 - \overset{R_5}{\overset{|}{CH}} - \overset{O}{\overset{\|}{C}} - R_6 \qquad (XII),$$

z.B. des Chlorids, mit einem Metallierungsmittel, z.B. Magnesium, Lithium oder tert-Butyllithium, hergestellt werden.

Verbindungen der Formel V werden beispielsweise hergestellt, indem man einen Aldehyd der Formel III mit einer Organometallverbindung der Formel IV gemäss Verfahren c) umsetzt und die gebildete Hydroxy-Verbindung der Formel I, gegebenenfalls nach Trennung der Isomeren, mit einer der Definition von X entsprechenden starken organischen oder anorganischen Säure verestert.

Nitrile der Formel VI werden beispielsweise hergestellt, indem man eine Verbindung der Formel

$$R_1 - A - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - Hal \qquad (XIII),$$

worin die Substituenten die genannten Bedeutungen haben, mit einem Salz der Cyanwasserstoffsäure umsetzt.

Geeignete Salze der Cyanwasserstoffsäure sollten im gewählten inerten Lösungsmittel genügend löslich sein, damit eine Umsetzung erfolgen kann. Solche Salze sind z.B. Ammoniumcyanid, Alkalimetall- oder Erdalkalimetallcyanide, z.B. Natrium- oder Kaliumcyanid, oder Uebergangsmetallcyanide, z.B. Kupfercyanid. Letztere eignen sich aufgrund ihrer im Vergleich zu den Alkalimetallcyaniden geringeren Basizität.

Je nach der Art des eingesetzten Cyanids und des Lösungsmittels stellt sich ein Gleichgewicht zwischen der isomeren Nitril- und der Isonitrilform ein. Die Nitrilform wird bevorzugt gebildet, wenn z.B. die Umsetzung mit solchen Metallcyaniden erfolgt, deren Metallkationen ein niedrigeres Atomgewicht als das von Kupfer besitzen.

Geeignete inerte Lösungsmittel sind vor allem polare, aprotische Lösungsmittel, beispielsweise Carbonsäureamide, z.B. Dimethylformamid oder Dimethylacetamid, Nitrile, z.B. Acetonitril oder Propionitril, oder Diniederalkylsulfoxide, z.B. Dimethylsulfoxid.

Die Umsetzung erfolgt bei Raumtemperatur, bei erniedrigter oder bei erhöhter Temperatur, z.B. in einem Temperaturbereich von etwa -40°C bis etwa +100°C, bevorzugt von etwa -10°C bis etwa +50°C und gewünschtenfalls in einer Inertgas-, z.B. Stickstoffatmosphäre.

Epoxide der Formel VII werden z.B. hergestellt, indem man eine Verbindung der Formel

$$Z_1HN - \underset{R_3}{CH} - CHO \qquad (XIV),$$

worin $Z_1$ eine Aminoschutzgruppe ist, mit einer Phosphoranylidenverbindung der Formel

$$(R_c)_3P = CH - \overset{R_5}{CH} - \overset{O}{\overset{\|}{C}} - Z_2 \qquad (XV),$$

worin $R_c$ einen gegebenenfalls substituierten Kohlenwasserstoffrest darstellt, $R_5$ die genannte Bedeutung hat und $Z_2$ eine Carboxyschutzgruppe ist, umsetzt und eine erhältliche Verbindung der Formel

$$Z_1HN - \underset{R_3}{CH} - CH = CH - \overset{R_5}{CH} - \overset{O}{\overset{\|}{C}} - Z_2 \qquad (XVI)$$

mit einem die Peroxygruppe enthaltenden Oxidationsmittel in ein Epoxid überführt und in einer erhältlichen Verbindung in beliebiger Reihenfolge der Reaktionsschritte die Schutzgruppen $Z_1$ und $Z_2$ abspaltet und durch die Gruppen $R_1$-A- und $R_6$ ersetzt.

$R_c$ ist bevorzugt Phenyl. Die Umsetzung einer Verbindung der Formel XIV mit einer Phosphoranylidenverbindung der Formel XV erfolgt unter den für Wittig-Reaktionen bekannten und z.B. im Organikum beschriebenen Reaktionsbedingungen. Das dabei erhältliche Olefin der Formel XVI wird gegebenenfalls in situ mit dem Oxidationsmittel, z.B. Peressigsäure oder m-Chlorperbenzoesäure, umgesetzt. Die Abspaltung der Schutzgruppen $Z_1$ und $Z_2$ und die Einführung der Gruppen $R_1$-A- bzw. $R_6$ ist weiter vorn unter Verfahren a) beschrieben.

Verbindungen der Formel VIII werden beispielsweise hergestellt, indem man eine Acrylsäure der Formel

$$R^b - (CH_2)_q - \underset{\underset{CH_2}{\|}}{C} - COOH \qquad (XVII)$$

0236734

oder ein geeignetes funktionelles Derivat davon mit einer Verbindung der Formel

$$H - A - \overset{R_2}{\overset{|}{N}} - \underset{R_3}{\underset{|}{CH}} - \overset{R_4}{\overset{|}{CH}} - CH_2 - \overset{R_5}{\overset{|}{CH}} - \overset{O}{\overset{\|}{C}} - R_6 \qquad (XVIII)$$

gemäss Verfahren a) umsetzt. Die Verbindung der Formel XVIII wird ebenfalls gemäss Verfahren a) aus einer an der Aminogruppe geschützten Aminosäure der Formel H-A-OH und einer Verbindung der Formel X und anschliessender Abspaltung der Schutzgruppe hergestellt, gegebenenfalls vor oder nach Einführung der Reste $R_2$ und $R_4$.

Die folgenden Beispiele dienen zur Illustration der Erfindung, schränken deren Umfang jedoch in keiner Weise ein.

Temperaturen werden in Celsiusgraden angegeben. Die $R_f$-Werte werden auf Kieselgeldünnschichtplatten in folgenden Lösungsmittelsystemen ermittelt:

| | | |
|---|---|---|
| A | Essigester-n-Hexan | 1:1 |
| B | Essigester-n-Hexan | 1:2 |
| C | Essigester-n-Hexan | 1:4 |
| D | Essigester-n-Hexan | 1:5 |
| E | Essigester-n-Hexan | 1:6 |
| F | Essigester-n-Hexan | 1:9 |
| G | Essigester-n-Hexan | 1:19 |
| H | Methylenchlorid-Methanol | 19:1 |
| I | Methylenchlorid-Methanol | 9:1 |
| J | Methylenchlorid-Methanol | 4:1 |
| K | Methylenchlorid-Methanol-Wasser | 300:10:1 |
| L | Methylenchlorid-Aether | 4:1 |
| M | Methylenchlorid-Methanol-Ammoniak konz. | 400:10:1 |
| N | Methylenchlorid-Methanol-Ammoniak konz. | 200:10:1 |
| O | Methylenchlorid-Methanol-Ammoniak konz. | 100:10:1 |
| P | Methylenchlorid-Methanol-Ammoniak konz. | 90:10:1 |
| Q | Methylenchlorid-Methanol-Ammoniak konz. | 80:10:1 |

| | | |
|---|---|---|
| R | Methylenchlorid-Methanol-Ammoniak konz. | 40:10:1 |
| S | Methylenchlorid-Methanol-Ammoniak konz. | 1000:50:1 |
| T | Methylenchlorid-Methanol-Ammoniak konz. | 850:50:1 |
| U | Methylenchlorid-Methanol-Ammoniak konz. | 700:50:1 |
| V | Methylenchlorid-Methanol-Ammoniak konz. | 500:50:1 |
| W | Methylenchlorid-Methanol-Ammoniak konz. | 350:50:1 |
| X | Methylenchlorid-Methanol-Ammoniak konz. | 300:50:1 |
| Y | Methylenchlorid-Methanol-Wasser-Eisessig | 150:54:10:1 |
| Z | Methylenchlorid-Methanol-Ammoniak konz. | 300:10:1 |
| AA | Methylenchlorid-Methanol-Ammoniak konz. | 65:10:1 |
| BB | Methylenchlorid-Methanol-Ammoniak konz. | 60:10:1 |
| CC | Methylenchlorid-Methanol-Ammoniak konz. | 50:10:1 |
| DD | Methylenchlorid-Methanol-Wasser | 5: 3:1 |
| EE | Methylenchlorid-Methanol-Wasser | 14: 6:1 |

Beispielsweise bedeutet die Abkürzung "$R_f$(A)", dass der $R_f$-Wert im System A ermittelt wurde. Das Mengenverhältnis der Lösungsmittel zueinander ist in Volumenanteilen angegeben.

Die gleichen Abkürzungen werden für die Bezeichnung der Fliessmittel-Systeme bei der Flash-Chromatographie und der Mitteldruckchromatographie verwendet.

Abkürzungen für Aminosäuren und Aminosäurederivate:

| | |
|---|---|
| H-Ala-OH | L-Alanin |
| H-Cha-OH | L-Cyclohexylalanin |
| H-Gly-OH | Glycin |
| H-His-OH | L-Histidin |
| H-Ser-OH | L-Serin |
| H-Val-OH | L-Valin |

Die Werte für Proton-Kernresonanzspektroskopie ($^1$H-NMR) werden in ppm (parts per million) bezogen auf Tetramethylsilan als internen Standard angegeben. s = Singulett, d = Dublett, t = Triplett, q = Quartett, m = Multiplett, dxd = Doppeldublett.

m/e: Molekülion der bezeichneten Masse bei der Massenspektrometrie.

Das Fragment mit der Bezeichnung -Cha$\overset{c}{-}$Val- bedeutet das zweiwertige Radikal von (2S,4S,5S)-5-Amino-6-cyclohexyl-4-hydroxy-2-isopropyl-hexansäure und hat die Formel

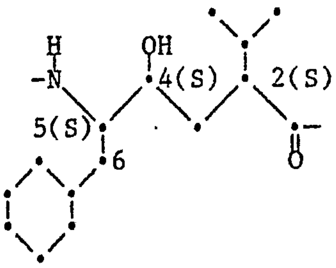

Das Fragment mit der Bezeichnung -Cha$\overset{cx}{-}$Val- leitet sich vom Fragment -Cha$\overset{c}{-}$Val- durch Ueberbrückung von NH und OH durch eine Isopropyliden-Gruppe ab und hat die Formel

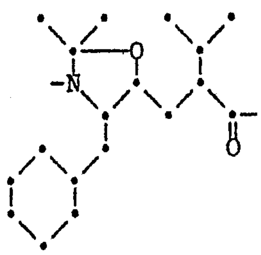

Das Fragment mit der Bezeichnung Gly($R_3$)$\overset{c}{-}$Gly($R_5$) bedeutet das zweiwertige Radikal von 2-$R_5$-4(S)-hydroxy-5-amino-5-$R_3$-pentansäure mit (R)- oder (S)-Konfiguration an C-Atom 2 oder 5 und hat die Formel

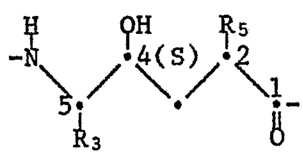

Das Fragment mit der Bezeichnung -Gly($R_3$)$\overset{cx}{-}$Gly($R_5$)- leitet sich vom Fragment -Gly($R_3$)$\overset{c}{-}$Gly($R_5$)- durch Ueberbrückung von NH und OH durch eine Isopropyliden-Gruppe ab.

Das Fragment mit der Bezeichnung -(S)-Gly($R_3$)$\overset{c}{-}$Val- bedeutet demzufolge das zweiwertige Radikal von 2(S)-Isopropyl-4(S)-hydroxy-5(S)-amino-5-$R_3$-pentansäure.

Weitere Abkürzungen:

| | | |
|---|---|---|
| abs. | = | absolut (wasserfrei) |
| Ac | = | Acetyl |
| BOC | = | tert-Butoxycarbonyl |
| DCCI | = | Dicyclohexylcarbodiimid |
| DCH | = | Dicyclohexylharnstoff |
| DMF | = | Dimethylformamid |
| DMSO | = | Dimethylsulfoxid |
| HOBt | = | 1-Hydroxybenzotriazol |
| Min. | = | Minute(n) |
| Sdp. | = | Siedepunkt |
| Smp. | = | Schmelzpunkt |
| Std. | = | Stunde(n) |
| THF | = | Tetrahydrofuran |
| Z | = | Benzyloxycarbonyl |

Beispiel 1: N-(2(R,S)-Benzyl-3-tert-butylsulfonyl-propionyl)-His-Cha$\overset{c}{-}$Val-methylamid

Eine Mischung aus 50 mg H-His-Cha$\overset{c}{-}$Val-methylamid, 37 mg 2-Benzyl-3-tert-butylsulfonyl-propionsäure, 20 mg HOBt, 32 mg DCCI und 3 ml DMF wird 50 Std. bei Raumtemperatur gerührt. Der kristallisierte DCH wird abfiltriert und das Filtrat eingedampft. Das Rohprodukt wird durch Mitteldruckchromatographie (1 Lobar®-Säule Grösse B, Laufmittel N) gereinigt. Die die Titelverbindung enthaltenden Fraktionen werden vereinigt und aus tert-Butanol lyophilisiert. $R_f$(Q) = 0,26 und 0,30 (2 Diastereomere).

Die Ausgangsmaterialien werden folgendermassen hergestellt:

a) bis k) H-His-Cha$\overset{c}{-}$Val-methylamid

a) 2(S)-Benzyloxycarbonylamino-3-cyclohexyl-propionsäureäthylester: 243 g 2(S)-Benzyloxycarbonylamino-3-cyclohexyl-propionsäure (Herstellung: Helvetica Chimica Acta 57, 2131(1974)) werden in 600 ml Toluol und 900 ml Aethanol vorgelegt. Das Reaktionsgemisch wird auf 0° gekühlt und 88,3 g Thionylchlorid innert 30 Min. zugetropft. Die Kühlung wird entfernt und die Mischung während 18 Std. gerührt. Das Reaktionsgemisch wird filtriert und das Filtrat eingeengt. Der Rückstand wird mittels Flash-Chromatographie (2 kg Kieselgel 60, 40-63 µm, Laufmittel F) aufgetrennt. Die produkthaltigen Fraktionen werden vereinigt, eingedampft und im Hochvakuum getrocknet. Man erhält die Titelverbindung als leicht gelbliches Oel. $R_f$ (F) = 0,2; $R_f$ (B) = 0,52.

b) 2(S)-Benzyloxycarbonylamino-3-cyclohexyl-propanal: 116,1 g 2(S)-Benzyloxycarbonylamino-3-cyclohexyl-propionsäureäthylester werden in 2,2 l Toluol vorgelegt und auf -65° abgekühlt. 836 ml Diisobutylaluminiumhydrid werden innert 30 Min. tropfenweise bei -65° zugegeben und das Gemisch 20 Min. nachgerührt. Dann werden bei -65° 84,2 ml Methanol innert 10 Min. zugetropft, anschliessend 825 ml wässrige Kalium-natrium-tartrat-Lösung ohne Kühlung. Das Reaktionsgemisch wird auf 3 l Kalium-natrium-tartrat-Lösung/Eis ausgetragen und mit 5 l Aether extrahiert. Die Aetherphase wird mit 2 l Wasser gewaschen, dann sofort in eine Lösung bestehend aus 106 g Semicarbazid-Hydrochlorid und 156,5 g Natriumacetat in 620 ml Wasser und 620 ml Aethanol gegossen. Das Reaktionsgemisch wird 1 Std. bei Raumtemperatur nachgerührt, dann im Scheidetrichter abgetrennt und die Wasserphase mit 2 x 1,5 l Aether extrahiert. Die organische Phase wird über Magnesiumsulfat getrocknet und eingedampft. Das Rohprodukt wird mittels Flash-Chromatographie gereinigt (2 kg Kieselgel 60, 40-63 µm, Laufmittel A). Durch Eindampfen der vereinigten, produkthaltigen Fraktionen erhält man das Semicarbazon der Titelverbindung, $R_f$ (I) = 0,51. 130 g dieses Semicarbazons werden in 1 l THF gelöst, mit 282 ml 37 % Formaldehydlösung und dann bei 10° mit 143 ml 0,5N HCl versetzt. Das Reaktionsgemisch wird 2 Std. bei Raumtemperatur gerührt, filtriert und das Filtrat mit 0,5 l Wasser, 0,5 l $NaHCO_3$ und 0,5 l Wasser gewaschen. Die Wasserphasen werden mit

600 ml Aether extrahiert. Die Aetherphasen werden über Magnesiumsulfat getrocknet und eingedampft. Der Rückstand wird mit 100 ml Toluol versetzt und eingedampft, wobei die Titelverbindung erhalten wird. Diese wird sofort weiterverarbeitet.

c) <u>(1(S)-Benzyloxycarbonylamino-2-cyclohexyl-äthyl)-oxiran:</u> 18,9 g Natriumhydriddispersion (55 % in Oel) werden in einem trockenen Sulfierkolben unter Argon durch dreimaliges Aufrühren in 50 ml Petroläther (Sdp. 40-60°) und anschliessendes Abdekantieren des Lösungsmittels vom Oel befreit. Nach Trocknen im Hochvakuum wird ein graues Pulver erhalten, das in 500 ml THF vorgelegt und mit 55,6 g Trimethylsulfoxoniumiodid versetzt wird, wobei die Temperatur auf ca. 40° steigt. Die graue Suspension wird am Rückfluss 1 Std. gekocht und anschliessend innerhalb von 50 Min. bei -70° mit einer Lösung von 108,6 g 2(S)-Benzyloxycarbonylamino-3-cyclohexyl-propanal in 250 ml THF versetzt. Die gelbe Suspension wird 2 Std. bei 0° gerührt. Die gelblich-trübe Lösung wird auf 500 g Eis gegossen. Die wässrige Lösung wird mit 2,5 l Aether extrahiert, die organische Phase mit Wasser gewaschen und nach Trocknen über Natriumsulfat eingedampft. Der ölige Rückstand wird mittels Flash-Chromatographie (2,5 kg Kieselgel 60, 40-63 µm, Laufmittel C) aufgetrennt. Die produkthaltigen Fraktionen werden vereinigt, eingedampft und im Hochvakuum getrocknet. Man erhält die Titelverbindung (Diastereomerengemisch, ca. 4:1) als leicht gelbliches Oel. $R_f(K) = 0,71$; $R_f(C) = 0,16$.

d) <u>3(S)-Benzyloxycarbonylamino-4-cyclohexyl-1-jod-butan-2(R,S)-ol:</u> 42,3 g (1(S)-Benzyloxycarbonylamino-2-cyclohexyl-äthyl)-oxiran werden in 200 ml Acetonitril aufgenommen und die erhaltene Lösung auf 0° abgekühlt. Nach Zugabe von 20,9 g Natriumjodid werden während 30 Min. tropfenweise bei 0° 17,7 ml Trimethylchlorsilan zugegeben. Das Gemisch wird 40 Min. bei 0 - 3° gerührt und anschliessend auf 700 ml eiskaltes Wasser gegossen. Das wässrige Gemisch wird mit Aether extrahiert und die organische Phase mit 750 ml 5 %-iger wässriger Natriumthiosulfatlösung und 750 ml gesättigter, wässriger

Natriumchloridlösung gewaschen. Nach Trocknen über Natriumsulfat und Eindampfen erhält man ein öliges Gemisch der Titelverbindung, die direkt weiterverarbeitet wird.

e) <u>3-Benzyloxycarbonyl-4(S)-cyclohexylmethyl-2,2-dimethyl-5(R)-jod-methyl-1,3-oxazolidin:</u> 49,3 g der Verbindung Beispiel 1d) und 1,07 g p-Toluolsulfonsäuremonohydrat werden in 140 ml 2,2-Dimethoxypropan und 450 ml Methylenchlorid 3 Std. lang bei Raumtemperatur gerührt. Das Gemisch wird zwischen 1 l Methylenchlorid und 500 ml gesättigter, wässriger Natriumhydrogencarbonatlösung ausgeschüttelt. Die organische Phase wird mit Wasser gewaschen, über Natriumsulfat getrocknet und eingedampft. Das Rohprodukt wird mittels Flash-Chromatographie gereinigt (3 kg Kieselgel 60, 40-63 µm, Laufmittel E). Durch Eindampfen der vereinigten, produkthaltigen Fraktionen erhält man die Titelverbindung als leicht gelbliches Oel. $R_f$ (C) = 0,55; $R_f$ (E) = 0,46.

f) <u>2(R,S)-(3-Benzyloxycarbonyl-4(S)-cyclohexylmethyl-2,2-dimethyl-1,3-oxazolidinyl-5(S)-methyl)-3-methyl-buttersäuremethylester:</u> 14,3 ml Diisopropylamin werden in 200 ml absolutem Tetrahydrofuran unter Argon gelöst und auf 0° abgekühlt. Anschliessend wird bei 0 - 5° das Gemisch 20 Min. lang tropfenweise mit 65,8 ml einer 1,6M Lösung von n-Butyllithium in Hexan versetzt und 20 Min. gerührt. Dann werden bei -70° bis -75° 13,3 ml Isovaleriansäuremethylester zugetropft und die Mischung 1,5 Std. bei -75° gerührt. Bei -60° bis -75° werden unter Rühren 320 ml Hexamethylphosphorsäuretriamid zugetropft. Die entstandene Suspension wird 10 Min. lang gerührt und schliesslich bei -70° bis -75° in 5 Min. tropfenweise mit einer Lösung von 43,4 g der Verbindung Beispiel 1e) in 110 ml Tetrahydrofuran versetzt. Das Reaktionsgemisch wird bei Raumtemperatur während 2,5 Std. gerührt und schliesslich auf ein Gemisch von 1 l gesättigter, wässriger Ammoniumchloridlösung und 500 g Eis gegossen. Die wässrige Phase wird mit 2 l Essigester extrahiert, die organische Phase mit Wasser gewaschen und über Natriumsulfat getrocknet.

Nach Eindampfen erhält man das Diastereomerengemisch der Titelverbindung als gelbes Oel. $R_f$ (C) = 0,36; $R_f$ (F) = 0,21 (Werte für die weniger polare Komponente).

g) 2(R,S)-(3-Benzyloxycarbonyl-4(S)-cyclohexylmethyl-2,2-dimethyl-1,3-oxazolidinyl-5(S)-methyl)-3-methyl-buttersäure: 16,5 g Kalium-tert-butylat werden in 250 ml Aether bei ca. 5° mit 1,77 ml Wasser versetzt. Die weisse Suspension wird noch 10 Min. im Eisbad gerührt und darauf mit 35,8 g der Verbindung Beispiel 1f) (Diastereomerengemisch) in 250 ml Aether versetzt, wobei die Temperatur unterhalb 10° gehalten wird. Das Reaktionsgemisch wird nun während 18 Std. bei Raumtemperatur gerührt und schliesslich auf 500 ml gesättigte, wässrige Ammoniumchloridlösung gegossen. Die wässrige Phase wird mit Essigester extrahiert und die organische Phase mit gesättigter, wässriger Natriumchloridlösung gewaschen, über Natriumsulfat getrocknet und eingedampft. Das ölige Rohprodukt wird durch Flash-Chromatographie aufgetrennt (2,5 kg Kieselgel 60, 40 - 63 µm, Laufmittel C). Z-Cha$\overset{cx}{-}$Val-OH, die weniger polare Komponente der Titelverbindung mit der gewünschten Konfiguration des an die Isopropylgruppe gebundenen C-Atoms (S-Konfiguration) wird als gelbes Oel erhalten. $R_f$ (K) = 0,20; $R_f$ (L) = 0,35.

h) Z-Cha$\overset{cx}{-}$Val-methylamid: Eine Mischung aus 311,9 mg Z-Cha$\overset{cx}{-}$Val-OH, 6,4 ml DMF, 138,2 mg HOBt und 186,1 mg DCCI wird 24 Std. bei 0° stehen gelassen. Die Mischung wird mit einem Ueberschuss an Methylamin versetzt und 2 Std. bei 0° und 2 Std. bei Raumtemperatur gerührt. Der kristallisierte DCH wird abfiltriert, das Filtrat eingeengt und am Hochvakuum getrocknet. Aus dem Rückstand wird durch Flash-Chromatographie (Fliessmittel-System A) die Titelverbindung als farbloses Oel erhalten. $R_f$ (A) = 0,45.

i) H-Cha$\overset{c}{-}$Val-methylamid: 243 mg Z-Cha$\overset{cx}{-}$Val-methylamid werden in 10 ml Methanol-Wasser 9:1 in Gegenwart von 50 mg Palladium-Kohle (10 % Pd) bei Normaldruck und Raumtemperatur bis zur Sättigung hydriert. Das Reaktionsgemisch wird filtriert und das Filtrat mit

10 ml Wasser bei Raumtemperatur gerührt. Nach dem Abdampfen des Lösungsmittels wird die Titelverbindung als farbloses Oel erhalten. $R_f$ (W) = 0,11; $R_f$ (Y) = 0,31.

j) Z-His-Cha$\overset{c}{-}$Val-methylamid: Eine Mischung aus 150,8 mg H-Cha$\overset{c}{-}$Val-methylamid, 6 ml DMF, 81,1 mg HOBt, 153,3 mg Z-His-OH und 144,2 mg DCCI wird 48 Std. bei Raumtemperatur gerührt. Der DCH wird abfiltriert, das Filtrat eingeengt und am Hochvakuum getrocknet. Der Rückstand wird durch Flash-Chromatographie aufgetrennt (145 g Kieselgel 60, 40 - 63 µm, Laufmittel U). Durch Eindampfen der vereinigten, produkthaltigen Fraktionen erhält man die Titelverbindung. $R_f$ (W) = 0,35; $R_f$ (Y) = 0,65.

k) H-His-Cha$\overset{c}{-}$Val-methylamid: 130 mg Z-His-Cha$\overset{c}{-}$Val-methylamid werden in 5 ml Methanol-Wasser 9:1 in Gegenwart von 20 mg Palladium-Kohle (10 % Pd) bei Normaldruck und Raumtemperatur bis zur Sättigung hydriert. Das Reaktionsgemisch wird filtriert und das Filtrat mit 5 ml Wasser bei Raumtemperatur gerührt. Nach dem Abdampfen des Lösungsmittels wird die Titelverbindung als farbloses Oel erhalten. $R_f$ (R) = 0,38.

l) bis o) 2-Benzyl-3-tert-butylsulfonyl-propionsäure

l) α-Benzylacrylsäure-äthylester: 20 g Benzylmalonsäurediäthylester in 40 ml Aethanol werden bei Raumtemperatur mit 4,0 g KOH in 50 ml Aethanol versetzt, über Nacht bei Raumtemperatur gerührt, eingedampft, mit 7,1 ml Wasser versetzt und im Eisbad mit 6,3 ml konz. Salzsäure angesäuert. Es wird zwischen Wasser und Aether verteilt, die organische Phase getrocknet und der Aether abdestilliert. Der Rückstand wird mit 12,9 ml Pyridin, 0,61 g Piperidin und 1,78 g Paraformaldehyd versetzt. Das Gemisch wird im Oelbad (130°) während 90 Min. erhitzt, abgekühlt, mit 220 ml Wasser versetzt und dreimal mit 75 ml n-Hexan extrahiert. Die vereinigten organischen Phasen werden mit Wasser, 1N HCl, Wasser, ges. $NaHCO_3$-Lösung und Sole

gewaschen. Die Titelverbindung wird durch Destillation gewonnen. $^1$H-NMR (DMSO-$d_6$): 1,2 ppm (t, 3H); 3,6 (d, 2H); 4,1 (q, 2H); 5,6 (m, 1H); 6,15 (m, 1H); 7,25 (m, 5H).

m) <u>2-Benzyl-3-tert-butylthio-propionsäure-äthylester:</u> 4,0 g α-Benzylacrylsäure-äthylester werden in 40 ml THF gelöst und bei Raumtemperatur mit 2,39 ml tert-Butylmercaptan und 459 mg Natriumhydrid-Dispersion (55 % in Oel) umgesetzt. Die Mischung wird 5 Std. bei Raumtemperatur gerührt, auf 1 N Salzsäure gegossen und mit Essigester extrahiert. Die Extrakte werden getrocknet und eingedampft. Der Rückstand wird durch Flash-Chromatographie an 200 g Kieselgel 60 (Laufmittel G) gereinigt. Farbloses Oel, $^1$H-NMR (DMSO-$d_6$): 1,1 ppm (t, 3H); 1,2 (s, 9H); 2,4-3,0 (m, 5H); 4,05 (q, 2H); 7,2 (s, 5H).

n) <u>2-Benzyl-3-tert-butylsulfonyl-propionsäure-äthylester:</u> 0,5 g 2-Benzyl-3-tert-butylthio-propionsäure-äthylester werden in 8 ml Methanol gelöst, unter Eiskühlung mit 1,63 g Oxone® (Kaliumperoxomonosulfat, 50 % $KHSO_5$, Ventron GmbH, Karlsruhe) in 7 ml Wasser versetzt und über Nacht bei Raumtemperatur gerührt. Die Lösung wird mit Wasser verdünnt und mit Methylenchlorid extrahiert, die Extrakte getrocknet und eingedampft. $^1$H-NMR (DMSO-$d_6$): 1,0 ppm (t, 3H); 1,3 (s, 9H); 2,8-3,5 (m, 5H); 3,95 (q, 2H); 7,15-7,3 (m, 5H).

o) <u>2-Benzyl-3-tert-butylsulfonyl-propionsäure:</u> 550 mg 2-Benzyl-3-tert-butylsulfonyl-propionsäure-äthylester werden in 8 ml THF gelöst und dann mit 5 ml Wasser und 0,88 ml 2 N Kalilauge umgesetzt. Die Mischung wird über Nacht bei Raumtemperatur gerührt, mit 0,88 ml 2 N Salzsäure neutralisiert und eingedampft. Der Rückstand wird durch Flash-Chromatographie an 30 g Kieselgel 60 (Laufmittel H) gereinigt. Gelbes Oel; m/e 284; $^1$H-NMR (DMSO-$d_6$): 1,27 ppm (s, 9H); 2,73-3,1 (m, 4H); 3,2-3,5 (m, 1H); 7,2-7,4 (m, 5H); 12,5 (s, 1H).

Beispiel 2: N-(2(R,S)-Benzyl-3-tert-butylsulfonyl-propionyl)-His-Cha$\underline{c}$Val-n-butylamid

Analog Beispiel 1 wird die Titelverbindung ausgehend von 50 mg H-His-Cha$\underline{c}$Val-n-butylamid, 34 mg 2-Benzyl-3-tert-butylsulfonyl-propionsäure, 18 mg HOBt und 29 mg DCCI hergestellt und durch Flash-Chromatographie an 30 g Kieselgel 60 (Laufmittel M) gereinigt. $R_f$(O) = 0,30 und 0,35 (2 Diastereomere).

Das Ausgangsmaterial wird folgendermassen hergestellt:

a) H-His-Cha$\underline{c}$Val-n-butylamid wird durch Hydrierung von 1,6 g Z-His-Cha$\underline{c}$Val-n-butylamid in Gegenwart von 200 mg Palladium-Kohle (10 %) analog Beispiel 1k) erhalten. $R_f$ (W) = 0,05; $R_f$ (Y) = 0,16.

b) Z-His-Cha$\underline{c}$Val-n-butylamid wird ausgehend von 1,75 g Z-His-OH, 1,97 g H-Cha$\underline{c}$Val-n-butylamid, 930 mg HOBt und 1,62 g DCCI analog Beispiel 1j) erhalten und durch Flash-Chromatographie mit Lösungsmittel-System W gereinigt. Smp. 208-210°C. $R_f$ (W) = 0,49; $R_f$ (Y) = 0,62.

c) H-Cha$\underline{c}$Val-n-butylamid wird durch Hydrierung von 4,2 g Z-Cha$\underline{cx}$Val-n-butylamid in Gegenwart von 500 mg Palladium-Kohle (10 %) analog Beispiel 1i) erhalten. $R_f$ (W) = 0,25.

d) Z-Cha$\underline{cx}$Val-n-butylamid wird ausgehend von 4,01 g Z-Cha$\underline{cx}$Val-OH, 2,68 g n-Butylamin, 1,80 g HOBt und 2,41 g DCCI analog Beispiel 1h) erhalten und durch Flash-Chromatographie mit Lösungsmittel-System C gereinigt. $R_f$ (A) = 0,61.

Beispiel 3: N-(2(R,S)-Benzyl-3-tert-butylsulfinyl-propionyl)-His-Cha$\underline{c}$Val-n-butylamid

Analog Beispiel 1 wird die Titelverbindung ausgehend von 50 mg H-His-Cha$\underline{c}$Val-n-butylamid, 32 mg 2-Benzyl-3-tert-butylsulfinyl-propionsäure, 18 mg HOBt und 29 mg DCCI hergestellt und durch Mittel-

druckchromatographie (1 Lobar®-Säule Grösse B, Laufmittel N) gereinigt. $R_f$(Q) = 0,35 und 0,425 (mindestens 2 von 4 möglichen Diastereomeren).

Das Ausgangsmaterial wird folgendermassen hergestellt:

a) 2-Benzyl-3-tert-butylsulfinyl-propionsäure: 3,2 g 2-Benzyl-3-tert-butylsulfinyl-propionsäure-äthylester werden in 30 ml Methanol gelöst und mit 30 ml Wasser und 10,8 ml 1 N Natronlauge versetzt. Die Mischung wird 16 Stunden bei Raumtemperatur gerührt, mit 10,8 ml 1 N Salzsäure neutralisiert und am Vakuum eingeengt. Der Rückstand wird durch Flash-Chromatographie an 150 g Kieselgel 60 (Laufmittel J) gereinigt. $R_f$(J) = 0,38.

b) 2-Benzyl-3-tert-butylsulfinyl-propionsäure-äthylester: 4,48 g 2-Benzyl-3-tert-butylthio-propionsäure-äthylester werden bei -78° mit einer Lösung von 3,67 g m-Chlorperbenzoesäure in 40 ml Methylenchlorid versetzt. Die Reaktionslösung wird 2 Stunden bei -78° und 17 Stunden bei Raumtemperatur gerührt, dann mit wässriger $NaHCO_3$-Lösung und Wasser gewaschen, getrocknet und am Vakuum eingeengt. Der Rückstand wird durch Flash-Chromatographie an 150 g Kieselgel 60 (Laufmittel A) gereinigt. $^1$H-NMR (DMSO-$d_6$): 1,0-1,2 ppm (m, 15H); 2,6-3,2 (m, 5H); 4,0 (m, 2H); 7,2-7,4 (m, 5H) (2 Diastereomere).

Beispiel 4: N-(2(R,S)-Benzyl-3-tert-butylthio-propionyl)-His-Cha$\overset{c}{-}$Val-n-butylamid

Analog Beispiel 1 wird die Titelverbindung ausgehend von 50 mg H-His-Cha$\overset{c}{-}$Val-n-butylamid, 30 mg 2-Benzyl-3-tert-butylthio-propionsäure, 18 mg HOBt und 29 mg DCCI hergestellt und durch Flash-Chromatographie an 30 g Kieselgel 60 (Laufmittel M) gereinigt. $R_f$(O) = 0,36.

Das Ausgangsmaterial wird folgendermassen hergestellt:

a) 2-Benzyl-3-tert-butylthio-propionsäure: 0,5 g 2-Benzyl-3-tert-butylthio-propionsäure-äthylester werden in 5 ml THF gelöst und mit 3,2 ml Wasser und 0,9 ml 2 N Kalilauge versetzt. Die Mischung wird über Nacht bei Raumtemperatur gerührt, mit 0,9 ml 2 N Salzsäure neutralisiert und eingedampft. Der Rückstand wird durch Flash-Chromatographie an 30 g Kieselgel 60 (Laufmittel H) gereinigt. Gelbes Oel, $^1$H-NMR (DMSO-$d_6$): 1,23 ppm (s, 9H); 2,55-2,9 (m, 5H); 7,15-7,3 (m, 5H); 12,4 (s, 1H).

Beispiel 5: N-(2(R,S)-Benzyl-3-methylsulfonyl-propionyl)-His-Cha$^C$Val-methylamid

Analog Beispiel 1 wird die Titelverbindung ausgehend von 50 mg H-His-Cha$^C$Val-methylamid, 32 mg 2-Benzyl-3-methylsulfonyl-propionsäure, 20 mg HOBt und 32 mg DCCI hergestellt und durch Mitteldruck-Chromatographie (1 Lobar®-Säule Grösse B, Laufmittel P) gereinigt. $R_f$(Q) = 0,14 und 0,23 (2 Diastereomere).

Das Ausgangsmaterial wird folgendermassen hergestellt:

a) 2-Benzyl-3-methylsulfonyl-propionsäure: 1,74 g 2-Benzyl-3-methylsulfonyl-propionsäure-äthylester werden in 35 ml 4 N Salzsäure während 2 Stunden unter Rückfluss erhitzt. Die abgekühlte Lösung wird mit Essigester extrahiert, die Extrakte getrocknet und eingeengt und der Rückstand durch Mitteldruckchromatographie (1 Lobar®-Säule Grösse B, Laufmittel A) gereinigt. $^1$H-NMR (DMSO-$d_6$): 2,95 ppm (s, 3H); 2,8-3,8 (m, 5H); 7,2 (s, 5H).

b) 2-Benzyl-3-methylsulfonyl-propionsäure-äthylester: 2,198 g 2-Benzyl-3-methylthio-propionsäure-äthylester werden in 20 ml Methanol gelöst, im Eisbad mit 8,4 g Oxone® (Kaliumperoxomonosulfat, 50 % $KHSO_5$, Ventron) in 35 ml Wasser versetzt und bei Raumtemperatur über Nacht gerührt. Die Mischung wird mit Wasser verdünnt und mit Essigester extrahiert, die Extrakte getrocknet und eingedampft und der Rückstand durch Mitteldruckchromatographie (1 Lobar®-Säule Grösse B, Laufmittel C) gereinigt. $^1$H-NMR (DMSO-$d_6$): 1,0 ppm (t, 3H); 3,0 (s, 3H); 3,05-3,6 (m, 5H); 4,0 (q, 2H); 7,2 (s, 5H).

c) 2-Benzyl-3-methylthio-propionsäure-äthylester: 2,0 g α-Benzylacrylsäure-äthylester werden in 20 ml Aethanol gelöst, im Eisbad mit 1,474 g Natriumthiomethylat versetzt und während 45 Minuten bei 0°C gerührt. Das Reaktiongemisch wird in eine Lösung von 18 g Natriumdihydrogenphosphat in Wasser gegossen und mit Aether extrahiert. Die Extrakte werden getrocknet und eingedampft, wobei die Titelverbindung zurückbleibt. $^1$H-NMR (DMSO-$d_6$): 1,65 ppm (t, 3H); 2,1 (s, 3H); 2,4-3,0 (m, 5H); 4,0 (q, 2H); 7,2 (s, 5H).

Beispiel 6: N-(2(R,S)-Benzyl-3-methylsulfonyl-propionyl)-His-Cha$\overset{C}{-}$Val-n-butylamid

Analog Beispiel 1 wird die Titelverbindung ausgehend von 50 mg H-His-Cha$\overset{C}{-}$Val-n-butylamid, 29 mg 2-Benzyl-3-methylsulfonyl-propionsäure, 18 mg HOBt und 29 mg DCCI hergestellt und durch Mitteldruckchromatographie (1 Lobar®-Säule Grösse B, Laufmittel N) gereinigt. $R_f$(Q) = 0,37 und 0,44 (2 Diastereomere).

Beispiel 7: N-(2(R)- und 2(S)-Methylsulfonyl-4-phenyl-butyryl)-His-Cha$\overset{C}{-}$Val-n-butylamid

Analog Beispiel 1 werden die Titelverbindungen ausgehend von 46 mg H-His-Cha$\overset{C}{-}$Val-n-butylamid, 46 mg 2-Methylsulfonyl-4-phenylbuttersäure-dicyclohexylammoniumsalz, 17 mg HOBt und 27 mg DCCI hergestellt und durch Flash-Chromatographie an 110 g Kieselgel 60 (Laufmittel T) aufgetrennt. Diastereomer I: $R_f$(V) = 0,23. Diastereomer II: $R_f$(V) = 0,125.

Das Ausgangsmaterial wird folgendermassen hergestellt:

a) 2-Methylsulfonyl-4-phenylbuttersäure-dicyclohexylammoniumsalz: 2,09 g 2-Methylsulfonyl-4-phenylbuttersäure-äthylester werden bei Raumtemperatur in 11,59 ml 1 N Natronlauge und 11,59 ml Aethanol gelöst und über Nacht gerührt. Die Lösung wird mit 11,59 ml 1 N Salzsäure neutralisiert, eingedampft, in wenig Acetonitril aufgenommen und aufgekocht, dann filtriert. Das Filtrat wird mit 1,54 ml Dicyclohexylamin versetzt, das Kristallisat abfiltriert und aus Acetonitril/Diisopropyläther umkristallisiert. Smp. 184 - 185°.

b) 2-Methylsulfonyl-4-phenylbuttersäure-äthylester: Zu 1,745 g Natriumhydrid-Dispersion (55 % in Oel) in 40 ml DMF werden bei -30° innert 30 Min. 6,648 g Methylsulfonyl-essigsäureäthylester zugetropft. Die Lösung wird 15 Min. nachgerührt, dann erhitzt und unter Rückfluss innert 150 Min. 7,4 g 2-Phenyläthylbromid in 10 ml DMF zugetropft. Die Reaktionsmischung wird 20 Min. nachgerührt, mit 12 ml Eisessig angesäuert und eingedampft. Der Rückstand wird in Essigester aufgenommen, mit Wasser gewaschen, über Magnesiumsulfat getrocknet, eingedampft und durch Flash-Chromatographie an 700 g Kieselgel 60 (Laufmittel D) gereinigt. $^1$H-NMR (DMSO-$d_6$): 1,35 ppm (t, 3H); 2,6-2,9 (m, 2H); 3,75 (dxd, 1H); 4,25 (q, 2H).

Beispiel 8: N-(2(R,S)-Methylsulfonyl-3-phenyl-propionyl)-His-Cha$^C$Val-n-butylamid

Analog Beispiel 1 wird die Titelverbindung ausgehend von 46 mg H-His-Cha$^C$Val-n-butylamid, 45 mg 2-Methylsulfonyl-3-phenylpropionsäure-dicyclohexylammoniumsalz, 17 mg HOBt und 27 mg DCCI hergestellt und durch Flash-Chromatographie an 50 g Kieselgel 60 (Laufmittel U) gereinigt. $R_f$(X) = 0,46.

Das Ausgangsmaterial wird folgendermassen hergestellt:

a) 2-Methylsulfonyl-3-phenylpropionsäure-dicyclohexylammoniumsalz: 3,6 g 2-Methylsulfonyl-3-phenylpropionsäure-äthylester werden in 21 ml Aethanol und 21 ml 1 N Natronlauge über Nacht bei Raumtemperatur gerührt. Das verfestigte Reaktionsgemisch wird mit 21 ml 1 N Salzsäure neutralisiert, eingedampft, in Acetonitril aufgekocht und filtriert. Das Filtrat wird mit 2,8 ml Dicyclohexylamin versetzt, das Kristallisat abfiltriert und aus Acetonitril/Diisopropyläther umkristallisiert. Smp. 187 - 188°.

0236734

b) 2-Methylsulfonyl-3-phenylpropionsäure-äthylester: Zu 1,745 g Natriumhydrid-Dispersion (55 % in Oel) in 40 ml DMF werden bei -25° innert 25 Min. 6,648 g Methylsulfonyl-essigsäureäthylester in 20 ml DMF zugetropft. Die Lösung wird 30 Min. bei -25° nachgerührt, dann 6,84 g Benzylbromid in 40 ml DMF zugetropft. Die Reaktionsmischung wird 1 Std. bei Raumtemperatur nachgerührt, mit 12 ml Eisessig angesäuert, über Nacht stehen gelassen und eingedampft. Der Rückstand wird in Essigester aufgenommen, mit Wasser gewaschen, getrocknet, eingedampft und durch Flash-Chromatographie an 700 g Kieselgel 60 (Laufmittel E) gereinigt. $^1$H-NMR (DMSO-$d_6$): 1,1 ppm (t, 3H); 3,05 (s, 3H); 3,1-3,6 (m, 2H); 3,9-4,3 (m, 3H); 7,25 (m, 5H).

Beispiel 9: N-(2(S)-Benzyl-3-tert-butylsulfonyl-propionyl)-His-Cha$\overset{c}{-}$Val-n-butylamid

Analog Beispiel 2 wird die Titelverbindung ausgehend von 50 mg H-His-Cha$\overset{c}{-}$Val-n-butylamid, 34 mg 2(S)-Benzyl-3-tert-butylsulfonyl-propionsäure, 18 mg HOBt und 29 mg DCCI hergestellt und durch Flash-Chromatographie an 30 g Kieselgel 60 (Laufmittel M) gereinigt. $R_f$(N) = 0,09; $R_f$(Y) = 0,57; $R_f$(O) = 0,35.

Das Ausgangsmaterial wird folgendermassen hergestellt:

a) 2(S)-Benzyl-3-tert-butylsulfonyl-propionsäure: 5,00 g 2(S)-Benzyl-3-tert-butylsulfonyl-propionsäure-1'(S)-benzyl-2'-hydroxy-äthylamid werden in 25 ml Eisessig und 75 ml 6N HCl während 5 Std. bei 90° gehalten. Das Reaktionsgemisch wird eingeengt und mit Methylenchlorid extrahiert. Die organische Phase wird mit 1N HCl gewaschen, eingeengt und an Kieselgel mit Laufmittel M chromatographiert. Die reine Titelverbindung wird aus Essigester/Hexan umkristallisiert, Smp. 99 - 101°. $R_f$(A) = 0,16; $[\alpha]_D^{22}$= 10,9° (c = 0,91 in $CH_2Cl_2$); $^1$H-NMR und DC-Laufverhalten identisch mit Verbindung Beispiel 1 o).

Die reine Titelverbindung kann auch durch fraktionierte Kristallisation der (+)-Dehydroabietylammoniumsalze der racemischen Säure in Isopropanol und Spaltung des diastereomerenreinen Kristallisats erhalten werden.

b) 2(R)- und 2(S)-Benzyl-3-tert-butylsulfonyl-propionsäure-1'(S)-benzyl-2'-hydroxy-äthylamid: Analog Beispiel 1 werden ausgehend von 12,5 g racemischer 2-Benzyl-3-tert-butylsulfonyl-propionsäure (Beispiel 1 o), 7,32 g 2(S)-Amino-3-phenyl-propanol (L-Phenylalaninol), 7,41 g HOBt und 11,80 g DCCI in 400 ml DMF die Amide hergestellt. Der auskristallisierte DCH wird abfiltriert, das Filtrat eingeengt und der Rückstand durch Flash-Chromatographie an Kieselgel mit Laufmittel A getrennt.

Unpolares 2(S)-Amid: $R_f(C)$ = 0,21; $[\alpha]_D^{22}$ = + 0,4° (c = 1,0 in $CH_3OH$). $^1H$-NMR (DMSO-$d_6$): 1,22 (s, 9H); 2,58 - 2,67 (m, 1H); 2,75 - 2,95 (m, 4H); 3,05 - 3,17 (m, 2H); 3,17 - 3,35 (m, 2H); 3,85 - 4,63 (t, 1H, OH); 7,11 - 7,32 (m, 10 H); 7,95 (d, 1H, NH).

Polares 2(R)-Amid: $R_f(C)$ = 0,11; $[\alpha]_D^{22}$ = -47,4° (c = 1,0 in $CH_3OH$). $^1H$-NMR (DMSO-$d_6$): 1,20 (s, 9H); 2,45 - 2,58 (m, 2H); 2,62 - 2,97 (m, 3H); 3,03 - 3,13 (m, 1H); 3,20 - 3,31 (m, 1H); 3,35 - 3,48 (m, 2H); 3,88 (m, 1H); 4,66 (t, 1H, OH); 7,05 - 7,28 (m, 10H); 8,05 (d, 1H, NH).

Beispiel 10: N-(2(R)-Benzyl-3-tert-butylsulfonyl-propionyl)-His-Cha$\overset{c}{-}$Val-n-butylamid

Analog Beispiel 2 wird die Titelverbindung ausgehend von 50 mg H-His-Cha$\overset{c}{-}$Val-n-butylamid, 34 mg 2(R)-Benzyl-3-tert-butylsulfonyl-propionsäure, 18 mg HOBt und 29 mg DCCI hergestellt und durch Flash-Chromatographie an 30 g Kieselgel (Laufmittel M) gereinigt. $R_f(N)$ = 0,11; $R_f(Y)$ = 0,61; $R_f(O)$ = 0,30.

Das Ausgangsmaterial wird folgendermassen hergestellt:

a) 2(R)-Benzyl-3-tert-butylsulfonyl-propionsäure wird analog Beispiel 9a) durch Hydrolyse des entsprechenden (polaren) 1'(S)-Benzyl-2'-hydroxy-äthylamids (Beispiel 9b) erhalten. $[\alpha]_D^{22}$ = -8,6° (c = 1,01 in $CH_3OH$); $^1H$-NMR und DC-Laufverhalten identisch mit Verbindungen Beispiel 9a) und 1o).

Beispiel 11: N-(2(R,S)-Benzyl-3-isopropylsulfonyl-propionyl)-His-Cha$\overset{c}{-}$Val-methylamid

Analog Beispiel 1 wird aus 56 mg 2(R,S)-Benzyl-3-isopropylsulfonyl-propionsäure, 80 mg H-His-Cha$\overset{c}{-}$Val-methylamid, 32 mg HOBt und 51 mg DCCI die Titelverbindung erhalten und durch Flash-Chromatographie mit Laufmittel N gereinigt. $R_f$ (Q) = 0,4 und 0,35 (2 Diastereomere).

Das Ausgangsmaterial 2-Benzyl-3-isopropylsulfonyl-propionsäure wird analog Beispiel 1 m), n) und o) aus α-Benzylacrylsäure-äthylester und Isopropylmercaptan hergestellt.

Beispiel 12: N-(3-Aethylsulfonyl-2(R,S)-benzyl-propionyl)-His-Cha$\overset{c}{-}$Val-methylamid

Analog Beispiel 1 wird aus 67 mg 3-Aethylsulfonyl-2(R,S)-benzyl-propionsäure, 100 mg H-His-Cha$\overset{c}{-}$Val-methylamid, 40 mg HOBt und 63 mg DCCI die Titelverbindung erhalten und durch Flash-Chromatographie mit Laufmittel N gereinigt. $R_f$ (Q) = 0,26 und 0,20 (2 Diastereomere).

Das Ausgangsmaterial 3-Aethylsulfonyl-2-benzyl-propionsäure wird analog Beispiel 1m), n) und o) aus α-Benzylacrylsäure-äthylester und Aethylmercaptan hergestellt.

Beispiel 13: N-(2(R,S)-Benzyl-3-[2-pyridyl]sulfonyl-propionyl)-His-Cha$\overset{c}{-}$Val-methylamid

Analog Beispiel 1 wird aus 41,8 mg 2(R,S)-Benzyl-3-(2-pyridyl)sulfonyl-propionsäure, 50 mg H-His-Cha$\overset{c}{-}$Val-methylamid, 19,9 mg HOBt und 31,8 mg DCCI die Titelverbindung erhalten und durch Flash-Chromatographie mit Laufmittel O gereinigt. $R_f$ (R) = 0,56.

Das Ausgangsmaterial wird folgendermassen hergestellt:

a) 2-Benzyl-3-(2-pyridyl)sulfonyl-propionsäure: 233 mg 2-Benzyl-3-(2-pyridyl)sulfonyl-propionsäure-äthylester werden in 4 ml 4N HCl 2 Std. am Rückfluss gekocht. Das Reaktionsgemisch wird mit Wasser verdünnt und mit Essigester extrahiert. Die organischen Phasen werden mit Wasser gewaschen, getrocknet und eingedampft. Der Rückstand wird an Kieselgel mit Laufmittel H chromatographiert und ergibt weisse Kristalle der Titelverbindung mit $R_f(I) = 0,3$.

b) 2-Benzyl-3-(2-pyridyl)sulfonyl-propionsäure-äthylester: Eine Lösung von 239 mg 2-Benzyl-3-(2-pyridyl)thio-propionsäure-äthylester in 2 ml Methanol wird bei Raumtemperatur mit einer Lösung von 724 mg Oxone® (Kaliumperoxomonosulfat, 50 % $KHSO_5$, Ventron) in 3,5 ml Wasser versetzt und 20 Std. bei dieser Temperatur gerührt. Danach wird das Reaktionsgemisch zweimal mit Essigester extrahiert. Die organischen Phasen werden mit Wasser gewaschen, über Natriumsulfat getrocknet und eingedampft. Durch Chromatographie des Rückstandes auf Kieselgel mit Laufmittel A erhält man die Titelverbindung als farbloses Oel. $R_f(A) = 0,57$.

c) 2-Benzyl-3-(2-pyridyl)thio-propionsäure-äthylester: Eine Lösung von 1 g α-Benzylacrylsäureäthylester und 643 mg 2-Mercaptopyridin in 5 ml abs. Aethanol wird bei 0° unter Rühren mit 106 mg Triäthylamin versetzt. Das Reaktionsgemisch wird 5 Tage bei Raumtemperatur gerührt und dann am Vakuum eingedampft. Durch Flash-Chromatographie des Rückstandes auf Kieselgel mit Laufmittel G erhält man die Titelverbindung als farbloses Oel. $R_f(C) = 0,44$.

Beispiel 14: N-(2(R,S)-Benzyl-3-dimethylaminosulfonyl-propionyl)-His-Cha$^{C}$Val-methylamid

Analog Beispiel 1 wird aus 21,8 mg 2(R,S)-Benzyl-3-dimethylamino-sulfonyl-propionsäure, 36,3 mg H-His-Cha$^{C}$Val-methylamid, 13,1 mg HOBt und 21 mg DCCI die Titelverbindung erhalten und durch Flash-Chromatographie mit Laufmittel Z gereinigt. $R_f(Q) = 0,42$.

Das Ausgangsmaterial wird folgendermassen hergestellt:

a) 2-Benzyl-3-dimethylaminosulfonyl-propionsäure: Ein Gemisch aus 299 mg 2-Benzyl-3-dimethylaminosulfonyl-propionsäureäthylester, 1 ml Methanol, 2 ml Wasser und 0,42 ml 1N NaOH wird 16 Std. bei Raumtemperatur gerührt, danach mit 0,42 ml 1N HCl neutralisiert und mit Essigester extrahiert. Die organischen Extrakte werden mit Wasser gewaschen, getrocknet, eingedampft und durch Chromatographie an Kieselgel mit Laufmittel I gereinigt. $R_f$(J) = 0,48.

b) 2-Benzyl-3-dimethylaminosulfonyl-propionsäureäthylester: Eine Lösung von 359 mg 2-Benzyl-3-chlorsulfonyl-propionsäureäthylester in 2 ml Methylenchlorid wird auf -10° gekühlt und mit 0,464 ml einer 5,6 M Lösung von Dimethylamin in Aethanol versetzt. Die Lösung wird 15 Min. bei -15° gerührt, mit 2N HCl vermischt und mit Methylenchlorid extrahiert. Die Extrakte werden mit Wasser gewaschen, mit Magnesiumsulfat getrocknet und eingedampft. Der Rückstand wird über Kieselgel mit Laufmittel C chromatographiert. $R_f$(A) = 0,43.

c) 2-Benzyl-3-chlorsulfonyl-propionsäureäthylester: In eine Suspension von 333 mg 3-Acetylthio-2-benzyl-propionsäureäthylester in 15 ml Wasser wird bei Raumtemperatur während 30 Min. ein Chlorgasstrom eingeleitet. Danach wird das Reaktionsgemisch mit Stickstoff gespült und mit Methylenchlorid ausgezogen. Die organische Phase wird mit Sole gewaschen, mit Magnesiumsulfat getrocknet und eingedampft. Die rohe Titelverbindung wird direkt weiterverarbeitet. $R_f$(C) = 0,36.

d) 3-Acetylthio-2-benzyl-propionsäureäthylester: Ein Gemisch aus 576 mg α-Benzylacrylsäureäthylester und 0,259 ml Thioessigsäure wird 20 Std. bei 70° gerührt und dann im Vakuum eingedampft. Der Rückstand wird durch Flash-Chromatographie mit Laufmittel F gereinigt. $R_f$(C) = 0,35.

Beispiel 15: N-(2(R,S)-Benzyl-3-diäthylaminosulfonyl-propionyl)-His-Cha$\overset{c}{-}$Val-methylamid

Analog Beispiel 1 wird aus 94 mg 2(R,S)-Benzyl-3-diäthylaminosulfonyl-propionsäure, 75 mg H-His-Cha$\overset{c}{-}$Val-methylamid, 49 mg HOBt und 84 mg DCCI die Titelverbindung erhalten und durch Flash-Chromatographie im System Z gereinigt. $R_f(Q) = 0,4$.

Das Ausgangsmaterial 2-Benzyl-3-diäthylaminosulfonyl-propionsäure wird analog Beispiel 14a) und b) hergestellt.

Beispiel 16: N-(2(R,S)-Benzyl-3-isopropylaminosulfonyl-propionyl)-His-Cha$\overset{c}{-}$Val-methylamid

Analog Beispiel 1 wird aus 13 mg 2(R,S)-Benzyl-3-isopropylaminosulfonyl-propionsäure, 21,2 mg H-His-Cha$\overset{c}{-}$Val-methylamid, 7,7 mg HOBt und 12,2 mg DCCI die Titelverbindung erhalten und durch Flash-Chromatographie mit Laufmittel O gereinigt. $R_f(Q) = 0,46$ und 0,35 (2 Diastereomere).

Das Ausgangsmaterial 2-Benzyl-3-isopropylaminosulfonyl-propionsäure wird analog Beispiel 14a) und b) hergestellt.

Beispiel 17: N-(3-Phenyl-2(R,S)-[2-thiazolinylthio]-propionyl)-His-Cha$\overset{c}{-}$Val-methylamid

Analog Beispiel 1 wird aus 15 mg 2(R,S)-(2-Thiazolinylthio)-hydrozimtsäure, 23,6 mg H-His-Cha$\overset{c}{-}$Val-methylamid, 8,5 mg HOBt und 15 mg DCCI die Titelverbindung erhalten und durch Flash-Chromatographie mit Laufmittel W gereinigt. $R_f(Q) = 0,48$.

Das Ausgangsmaterial wird folgendermassen hergestellt:

a) 2-(2-Thiazolinylthio)-hydrozimtsäure: Ein Gemisch aus 250 mg 2-(2-Thiazolinylthio)-hydrozimtsäure-äthylester, 36 mg Lithiumhydroxid-monohydrat und 5 ml THF/Wasser 9:1 wird 5 Tage bei Raumtemperatur gerührt und dann zur Trockne eingedampft. Der Rückstand wird in Wasser aufgenommen, mit Essigester gewaschen, mit 0,5 N HCl

auf pH 5 gestellt und mit Essigester extrahiert. Die Extrakte werden mit Wasser gewaschen, mit Natriumsulfat getrocknet, eingedampft und über Kieselgel mit Laufmittel R chromatographiert. $R_f$(R) = 0,22.

b) 2-(2-Thiazolinylthio)-hydrozimtsäure-äthylester: Eine Lösung von 1,8 g 2-Mercapto-1,3-thiazolin und 2,6 g 2-Brom-hydrozimtsäure-äthylester in 15 ml THF wird tropfenweise mit 1,4 ml Triäthylamin versetzt und 6 Std. unter Rückfluss gerührt. Das Reaktionsgemisch wird filtriert, das Filtrat eingedampft und der Rückstand zwischen Essigester und Wasser verteilt. Die organische Phase wird mit Wasser gewaschen, getrocknet, eingedampft und durch Chromatographie an Kieselgel mit Laufmittel R gereinigt. $R_f$(W) = 0,67.

Beispiel 18: N-(2(R,S)-Benzyl-3-tert-butylsulfonyl-propionyl)-His-Cha$\stackrel{c}{-}$Val-2-(4-imidazolyl)-äthylamid

Analog Beispiel 1 wird aus 38,6 mg 2(R,S)-Benzyl-3-tert-butylsulfonyl-propionsäure, 61,9 mg H-His-Cha$\stackrel{c}{-}$Val-2-(4-imidazolyl)-äthylamid, 20,8 mg HOBt und 33,1 mg DCCI die Titelverbindung erhalten und durch Flash-Chromatographie mit Laufmittel O gereinigt.
$R_f$(CC) = 0,42 und 0,32 (2 Diasteroemere).

Das Ausgangsmaterial wird folgendermassen hergestellt:

a) H-His-Cha$\stackrel{c}{-}$Val-2-(4-imidazolyl)-äthylamid: Ein Gemisch aus $N^{\alpha}$, $N^{Im}$-Ditrityl-His-Cha$\stackrel{c}{-}$Val-2-(4-imidazolyl)-äthylamid und 2 ml Trifluoressigsäure wird 30 Min. bei Raumtemperatur gerührt, am Wasserstrahlvakuum bei 40° eingeengt, mit 2 ml eines Gemisches aus Methylenchlorid/Methanol/$NH_3$ konz. 5:3:1 versetzt und nochmals am Vakuum eingedampft. Aus dem Rückstand erhält man die Titelverbindung durch Chromatographie mit Laufmittel CC. $R_f$(R) = 0,155.

b) $N^{\alpha}$,$N^{Im}$-Ditrityl-His-Cha$\stackrel{c}{-}$Val-2-(4-imidazolyl)-äthylamid: Analog Beispiel 1j) wird die Titelverbindung aus 155 mg $N^{\alpha}$,$N^{Im}$-Ditrityl-histidin, 63 mg H-Cha$\stackrel{c}{-}$Val-2-(4-imidazolyl)-äthylamid, 37 mg HOBt und 57 mg DCCI erhalten und durch Chromatographie mit Laufmittel N gereinigt. $R_f$(Q) = 0,82.

c) H-Cha$^{c}$Val-2-(4-imidazolyl)-äthylamid: Analog Beispiel 1i) wird die Titelverbindung durch Hydrierung von 103 mg Z-Cha$^{cx}$Val-2-(4-imidazolyl)-äthylamid mit 50 mg Pd/C in 20 ml Methanol/Wasser 19:1 bei Normaldruck und Raumtemperatur erhalten. Farbloses Oel, $R_f$(BB) = 0,25.

d) Z-Cha$^{cx}$Val-2-(4-imidazolyl)-äthylamid: Analog Beispiel 1h) wird aus 100 mg Z-Cha$^{cx}$Val-OH, 45,4 mg Histamin-dihydrochlorid, 60 mg DCCI, 37,8 mg HOBt und 63,8 mg Diisopropyläthylamin die Titelverbindung erhalten und durch Chromatographie mit Laufmittel AA gereinigt. $R_f$(BB) = 0,62.

Beispiel 19: N-(2(R,S)-Benzyl-3-tert-butylsulfonyl-propionyl)-His-Cha$^{c}$Val-((S)-5-tert-butoxycarbonylamino-5-methoxycarbonyl-pentyl)-amid

Analog Beispiel 1 wird aus 56,9 mg 2-Benzyl-3-tert-butylsulfonyl-propionsäure, 118,3 mg H-His-Cha$^{c}$Val-((S)-5-tert-butoxycarbonylamino-5-methoxycarbonyl-pentyl)-amid, 30,6 mg HOBt und 49,4 mg DCCI in 3,3 ml DMF die Titelverbindung erhalten und durch Flash-Chromatographie mit Lösungsmittel-System W gereinigt. $R_f$(X) = 0,67; $R_f$(U) = 0,09.

Die Herstellung des Ausgangsmaterials H-His-Cha$^{c}$Val-((S)-5-tert-butoxycarbonylamino-5-methoxycarbonyl-pentyl)-amid ist in der Europäischen Patentanmeldung EP 184 560 beschrieben.

Beispiel 20: N-(2(R,S)-Benzyl-3-tert-butylsulfonyl-propionyl)-His-Cha$^{c}$Val-((S)-5-amino-5-carboxy-pentyl)-amid

75,6 mg N-(2(R,S)-Benzyl-3-tert-butylsulfonyl-propionyl)-His-Cha$^{c}$Val-((S)-5-tert-butoxycarbonylamino-5-methoxycarbonyl-pentyl)-amid und 1,25 ml 0,1 N NaOH werden in 1,25 ml Methanol bei Raumtemperatur 16 Std. gerührt. Das Reaktionsgemisch wird eingedampft, mit 1,5 ml Trifluoressigsäure versetzt und 20 Min. bei Raumtemperatur stehen gelassen. Die Trifluoressigsäure wird abgedampft und das Rohprodukt durch Flash-Chromatographie (90 g

Kieselgel 40 - 63 µm, Laufmittel DD) gereinigt. Der Rückstand wird aus tert-Butanol lyophilisiert, wobei die Titelverbindung als weisses Pulver zurückbleibt. $R_f$ (DD) = 0,70; $R_f$(Y) = 0,24.

Beispiel 21: N-(2(S)-Benzyl-3-tert-butylsulfonyl-propionyl)-Ala-Cha$\stackrel{C}{-}$Val-n-butylamid

Analog Beispiel 2 wird die Titelverbindung ausgehend von 105 mg H-Ala-Cha$\stackrel{C}{-}$Val-n-butylamid, 98 mg 2(S)-Benzyl-3-tert-butylsulfonyl-propionsäure (Beispiel 9a), 53 mg HOBt und 82 mg DCCI hergestellt und durch Flash-Chromatographie an 65 g Kieselgel (Laufmittel N) gereinigt. $R_f$(P) = 0,62.

Die Ausgangsmaterialien werden folgendermassen hergestellt:

a) H-Ala-Cha$\stackrel{C}{-}$Val-n-butylamid wird durch Hydrierung von 135 mg Z-Ala-Cha$\stackrel{C}{-}$Val-n-butylamid in Gegenwart von 50 mg Palladium-Kohle (10 %) analog Beispiel 1k) erhalten. $R_f$(O) = 0,33.

b) Z-Ala-Cha$\stackrel{C}{-}$Val-n-butylamid wird ausgehend von 64 mg Z-Ala-OH, 62 mg H-Cha$\stackrel{C}{-}$Val-n-butylamid (Beispiel 2c), 38 mg HOBt und 59 mg DCCI analog Beispiel 1j) erhalten und durch Flash-Chromatographie mit Lösungsmittelsystem N gereinigt. $R_f$(N) = 0,22.

Beispiel 22: N-(2(S)-Benzyl-3-tert-butylsulfonyl-propionyl)-Ala-Cha$\stackrel{C}{-}$Val-methylamid

Analog Beispiel 1 wird die Titelverbindung ausgehend von 90 mg H-Ala-Cha$\stackrel{C}{-}$Val-methylamid, 93 mg 2(S)-Benzyl-3-tert-butylsulfonyl-propionsäure, 46 mg HOBt und 78 mg DCCI hergestellt und durch Flash-Chromatographie an 30 g Kieselgel 60 (Laufmittel N) gereinigt. $R_f$(N) = 0,14; $R_f$(P) = 0,48.

Das Ausgangsmaterial wird folgendermassen hergestellt:

a) H-Ala-Cha$\stackrel{C}{-}$Val-methylamid wird durch Hydrierung von 390 mg Z-Ala-Cha$\stackrel{C}{-}$Val-methylamid in Gegenwart von 40 mg Palladium-Kohle (10 %) analog Beispiel 1k) erhalten. $R_f$(P) = 0,38; $R_f$(N) = 0,15.

b) Z-Ala-Cha$\stackrel{c}{-}$Val-methylamid wird ausgehend von 326 mg Z-Ala-OH, 380 mg H-Cha$\stackrel{c}{-}$Val-methylamid (Beispiel 1i), 225 mg HOBt und 358 mg DCCI analog Beispiel 1j) erhalten und durch Flash-Chromatographie mit Lösungsmittelsystem N gereinigt. $R_f$(N) = 0,19.

Beispiel 23: N-(2(S)-Benzyl-3-tert-butylsulfonyl-propionyl)-His-(S)-Gly(2-cyclohexyläthyl)$\stackrel{c}{-}$Val-n-butylamid

Eine Mischung aus 76 mg H-His-(S)-Gly(2-cyclohexyläthyl)$\stackrel{c}{-}$Val-n-butylamid, 50 mg 2-(S)-Benzyl-3-tert-butylsulfonyl-propionsäure, 27 mg HOBt, 43 mg DCCI und 2,4 ml DMF wird 8 Std. bei 0° und anschliessend 12 Std. bei Raumtemperatur gerührt. Der kristalline DCH wird abfiltriert und das Filtrat eingedampft. Der Rückstand wird in einem Gemisch von Methanol/Wasser/Eisessig 94:3:3 während 60 Min. bei 60° gerührt und anschliessend eingeengt. Der Rückstand wird mittels Flash-Chromatographie gereinigt (90 g Kieselgel Si 60, 40 - 63 µm, System U). Die die Titelverbindung enthaltenden Fraktionen werden vereinigt und aus tert-Butanol lyophilisiert. $R_f$(W) = 0,34.

Das Ausgangsmaterial wird folgendermassen hergestellt:

a) H-His-(S)-Gly(2-cyclohexyläthyl)$\stackrel{c}{-}$Val-n-butylamid wird analog Beispiel 2a) bis d) und 1a) bis g) aus 2(S)-Amino-4-cyclohexyl-buttersäure hergestellt. $R_f$(R) = 0,58.

b) 2(S)-Amino-4-cyclohexyl-buttersäure: 896 mg L-Homophenylalanin (2(S)-Amino-4-phenyl-buttersäure) werden in 25 ml Wasser und 5,5 ml 1N HCl in Gegenwart von 18 mg Nishimura-Katalysator (Rhodiumoxid/Platinoxid) hydriert. Das Reaktionsgemisch wird warm filtriert. Aus dem Filtrat kristallisiert nach Zugabe von 5,5 ml 1N NaOH die Titelverbindung als weisse Kristalle. Smp. 265 - 266°; $R_f$(R) = 0,09.

Beispiel 24: N-(2(S)-Benzyl-3-tert-butylsulfonyl-propionyl)-His-(S)-Gly(cyclopentylmethyl)$^{C}$Val-n-butylamid

Eine Mischung aus 72 mg H-His-(S)-Gly(cyclopentylmethyl)$^{C}$Val-n-butylamid, 50 mg 2(S)-Benzyl-3-tert-butylsulfonyl-propionsäure, 27 mg HOBt, 43 mg DCCI und 2,4 ml DMF wird 8 Std. bei 0° und anschliessend 12 Std. bei Raumtemperatur gerührt. Der kristalline DCH wird abfiltriert und das Filtrat eingedampft. Der Rückstand wird in einem Gemisch von Methanol/Wasser/Eisessig 94:3:3 während 60 Min. bei 60° gerührt und anschliessend eingeengt. Der Rückstand wird mittels Flash-Chromatographie aufgetrennt (100 g Kieselgel Si 60, 40 - 63 µm, System U). Die die Titelverbindung enthaltenden Fraktionen werden vereinigt und aus tert-Butanol lyophilisiert.
$R_f(W) = 0,31$; $R_f(Y) = 0,64$.

Das Ausgangsmaterial wird folgendermassen hergestellt:

a) H-His-(S)-Gly(cyclopentylmethyl)$^{C}$Val-n-butylamid wird analog Beispiel 2a) bis d) und 1a) bis g) aus 2-Amino-3-cyclopentyl-propionsäure (P.R.Pal, Ch.G.Skinner, R.L.Dennis und W.Shive, J.Am.Chem. Soc.78, 5116 (1956)) erhalten. $R_f(R) = 0,42$.

Beispiel 25: N-(2(S)-Benzyl-3-tert-butylsulfonyl-propionyl)-His-(S)-Gly(cycloheptylmethyl)$^{C}$Val-n-butylamid

Eine Mischung aus 76 mg H-His-(S)-Gly(cycloheptylmethyl)$^{C}$Val-n-butylamid, 50 mg 2(S)-Benzyl-3-tert-butylsulfonyl-propionsäure, 27 mg HOBt, 43 mg DCCI und 2,4 ml DMF wird 8 Std. bei 0° und anschliessend 12 Std. bei Raumtemperatur gerührt. Der kristalline DCH wird abfiltriert und das Filtrat eingedampft. Der Rückstand wird in einem Gemisch von Methanol/Wasser/Eisessig 94:3:3 während 60 Min. bei 60° gerührt und anschliessend eingeengt. Der Rückstand wird mittels Flash-Chromatographie aufgetrennt (100 g Kieselgel Si 60, 40 - 63 µm, System U). Die die Titelverbindung enthaltenden Fraktionen werden vereinigt und aus tert-Butanol lyophilisiert.
$R_f(W) = 0,58$.

Das Ausgangsmaterial wird folgendermassen hergestellt:

a) <u>H-His-(S)-Gly(cycloheptylmethyl)$\overset{c}{-}$Val-n-butylamid</u> wird analog Beispiel 2a) bis d) und 1a) bis g) aus 2-Amino-3-cycloheptyl-propionsäure hergestellt, $R_f(R) = 0,60$. Die als Ausgangsmaterial verwendete Aminosäure kann analog der entsprechenden Cyclopentylsubstituierten Aminosäure synthetisiert und durch fraktionierte Kristallisation diastereomerer Salze oder chromatographische Trennung diastereomerer Amide in die Enantiomeren getrennt werden.

<u>Beispiel 26:</u> <u>N-(2(R,S)-Benzyl-3-tert-butylsulfonyl-propionyl)-His-Cha$\overset{c}{-}$Gly($SCH_3$)-n-butylamid</u>

Analog Beispiel 1 wird die Titelverbindung ausgehend von 140 mg H-His-Cha$\overset{c}{-}$Gly($SCH_3$)-n-butylamid, 128 mg 2-Benzyl-3-tert-butyl-sulfonyl-propionsäure, 72 mg HOBt und 92 mg DCCI hergestellt und durch Flash-Chromatographie an 30 g Kieselgel 60 (Laufmittel N) gereinigt. $R_f(I) = 0,32$ und 0,28 (2 Diastereomerenpaare).

Das Ausgangsmaterial wird folgendermassen hergestellt:

a) <u>H-His-Cha$\overset{c}{-}$Gly($SCH_3$)-n-butylamid:</u> Eine Mischung aus 132 mg H-Cha$\overset{c}{-}$Gly($SCH_3$)-n-butylamid, 260 mg 1-Benzotriazolyloxy-tris(dimethylamino)-phosphonium-hexafluorophosphat, 384 mg $N^{\alpha}$,$N^{Im}$-Ditrityl-histidin, 3 ml DMF und 0,1 ml Triäthylamin wird 14 Std. bei Raumtemperatur gerührt. Das Reaktionsgemisch wird eingeengt und der Rückstand durch Flash-Chromatographie (25 g Kieselgel 60, Laufmittel A) gereinigt. Anschliessend wird das $N^{\alpha}$,$N^{Im}$-Ditrityl-His-Cha$\overset{c}{-}$Gly($SCH_3$)-n-butylamid mit 3 ml 90 % Trifluoressigsäure versetzt und für 30 Min. bei Raumtemperatur gerührt. Das Reaktionsgemisch wird eingeengt und der Rückstand zwischen wässriger Kaliumcarbonatlösung und Methylenchlorid verteilt. Die vereinigten organischen Extrakte werden mit Sole gewaschen und über Natriumsulfat getrocknet. Nach Eindampfen erhält man das Diastereomerengemisch der Titelverbindung als weissen Schaum. $R_f(Y) = 0,18$.

b) H-Cha$^{c}$Gly($SCH_3$)-n-butylamid: Eine Mischung aus 240 mg Z-Cha$^{cx}$Gly($SCH_3$)-n-butylamid, 200 mg 4-Methylmercapto-phenol und 4 ml Trifluoressigsäure wird für 30 Std. bei Raumtemperatur gerührt. Das Reaktionsgemisch wird eingeengt, der Rückstand bei 0 - 5° mit 0,2 N wässriger NaOH auf pH 9 gestellt und anschliessend mit Methylenchlorid mehrmals extrahiert. Die vereinigten Extrakte werden getrocknet und eingedampft. Der Rückstand wird durch Flash-Chromatographie an 25 g Kieselgel 60 (Laufmittel O) gereinigt. $R_f$(P) = 0,08 (2 Diastereomere).

c) Z-Cha$^{cx}$Gly($SCH_3$)-n-butylamid wird ausgehend von 950 mg Z-Cha$^{cx}$Gly($SCH_3$)-OH, 460 mg HOBt, 440 mg n-Butylamin und 700 mg DCCI analog Beispiel 2d) erhalten und durch Flash-Chromatographie mit Lösungsmittel-System C gereinigt, $R_f$(A) = 0,56 und 0,50 (2 Diastereomere).

d) Z-Cha$^{cx}$Gly($SCH_3$)-OH: Eine Mischung aus 790 mg Z-Cha$^{cx}$Gly-($SCH_3$)-methylester, 5 ml 2N wässriger NaOH und 10 ml 1,4-Dioxan wird 3 Std. bei Raumtemperatur gerührt. Das Reaktionsgemisch wird mit 5 ml 2N wässriger HCl neutralisiert und anschliessend lyophilisiert. Aus dem Rückstand wird durch Flash-Chromatographie (Fliessmittel-System A) die Titelverbindung erhalten. $R_f$(A) 0,15 und 0,10 (2 Diastereomere).

e) 2(R,S)-(3-Benzyloxycarbonyl-4(S)-cyclohexylmethyl-2,2-dimethyl-1,3-oxazolidinyl-5(S)-methyl)-2-methylthio-essigsäuremethylester, Z-Cha$^{cx}$Gly($SCH_3$)-methylester: 0,85 ml Diisopropylamin werden in 15 ml abs. THF und 10 ml Hexamethylphosphorsäuretriamid unter Stickstoff gelöst und auf -78° abgekühlt. Anschliessend wird das Gemisch mit 4 ml einer 1,5 M Lösung von n-Butyllithium in Hexan versetzt und 30 Min. gerührt. Dann werden bei -70° 0,65 ml Methylthioessigsäure-methylester zugetropft und 1/2 Std. bei -75° gerührt. Das Reaktionsgemisch wird mit einer Lösung von 1,3 g der Verbindung Beispiel 1e) in 5 ml Tetrahydrofuran und 5 ml Hexamethylphosphorsäuretriamid versetzt und innerhalb 2 Std. auf 0° erwärmt. Nach Zugabe von 20 ml gesättigter, wässriger Ammoniumchloridlösung

wird das Produkt mit Aether extrahiert. Die vereinigten organischen Phasen werden mit Sole gewaschen, mit Natriumsulfat getrocknet und eingedampft. Das Rohprodukt wird durch Flash-Chromatographie gereinigt (60 g Kieselgel 60, 40 - 63 µm, Laufmittel G). Durch Eindampfen der vereinigten, produkthaltigen Fraktionen erhält man die beiden Diastereomeren der Titelverbindung als leicht gelbliche Oele. $R_f$(C) = 0,35 und 0,32.

Beispiel 27: N-(2(R,S)-Benzyl-3-tert-butylsulfonyl-propionyl)-His-Cha$\overset{c}{-}$Gly($SO_2CH_3$)-n-butylamid

35 mg N-(2(R,S)-Benzyl-3-tert-butylsulfonyl-propionyl)-His-Cha$\overset{c}{-}$Gly($SCH_3$)-n-butylamid werden in 2 ml Methanol und 2 ml 0,1 N $H_2SO_4$ gelöst, mit 55 mg Oxone® (Kaliumperoxomonosulfat, 50 % $KHSO_5$, Ventron) versetzt und bei Raumtemperatur über Nacht gerührt. Die Mischung wird mit gesättigter Natriumbicarbonatlösung verdünnt und mit Methylenchlorid extrahiert und die Extrakte mit Natriumsulfat getrocknet und eingeengt, wobei ein Diastereomerengemisch der Titelverbindung zurückbleibt. $R_f$(I) = 0,18.

Beispiel 28: N-(2(R,S)-Benzyl-3-tert-butylsulfonyl-propionyl)-His-Cha$\overset{c}{-}$Gly($OCH_3$)-methylamid

Analog Beispiel 1 wird die Titelverbindung ausgehend von 102 mg H-His-Cha$\overset{c}{-}$Gly($OCH_3$)-methylamid, 78 mg 2-Benzyl-3-tert-butylsulfonyl-propionsäure, 42 mg HOBt und 67 mg DCCI hergestellt und durch Flash-Chromatographie an 30 g Kieselgel 60 (Laufmittel O) gereinigt. $R_f$(O) = 0,24 und 0,20 (2 Diastereomerenpaare).

a) H-His-Cha$\overset{c}{-}$Gly($OCH_3$)-methylamid wird durch Hydrierung von 130 mg Z-His-Cha$\overset{c}{-}$Gly($OCH_3$)-methylamid in Gegenwart von 50 mg Palladium-Kohle (10 %) analog Beispiel 1k) erhalten. $R_f$(R) = 0,32 (Diastereomerengemisch).

b) Z-His-Cha$\overset{c}{-}$Gly($OCH_3$)-methylamid wird ausgehend von 125 mg Z-His-OH, 107 mg H-Cha$\overset{c}{-}$Gly($OCH_3$)-methylamid, 66 mg HOBt und 106 mg DCCI analog Beispiel 1j) erhalten und durch Flash-Chromatographie mit Lösungsmittel-System O gereinigt. $R_f$(Q) = 0,26.

c) H-Cha$\stackrel{c}{-}$Gly($OCH_3$)-methylamid wird durch Hydrierung von 184 mg Z-Cha$\stackrel{cx}{-}$Gly($OCH_3$)-methylamid in Gegenwart von 50 mg Palladium-Kohle (10 %) analog Beispiel 1i) erhalten. $R_f$(R) = 0,35.

d) Z-Cha$\stackrel{cx}{-}$Gly($OCH_3$)-methylamid: 258 mg Z-Cha$\stackrel{cx}{-}$Gly($OCH_3$)-äthylester werden in 2 ml Methanol gelöst und mit 2 ml 33 % äthanolischer Methylaminlösung versetzt. Die Mischung wird für 20 Std. in einem Bombenrohr auf 120 - 130°C erhitzt und dann eingeengt. Der Rückstand wird durch Flash-Chromatographie an 50 g Kieselgel 60 (Laufmittel A) gereinigt. Gelbes Oel, $R_f$(A) = 0,16.

e) Z-Cha$\stackrel{cx}{-}$Gly($OCH_3$)-äthylester wird ausgehend von 601 mg Methoxyessigsäure-äthylester, 0,72 ml Diisopropylamin, 3,2 ml einer 1,6 M Lösung von n-Butyllithium in Hexan und 2 g der Verbindung Beispiel 1e) analog Beispiel 26 e) erhalten und durch Flash-Chromatographie mit Lösungsmittel-System G gereinigt. $R_f$(C) = 0,36 und 0,32 (Diastereomerengemisch).

Beispiel 29: N-(2(R,S)-Benzyl-3-[2-pyridylthio]-propionyl)-His-Cha$\stackrel{c}{-}$Val-n-butylamid

Analog Beispiel 1 wird aus 85 mg 2-Benzyl-3-(2-pyridylthio)-propionsäure, 115 mg H-His-Cha$\stackrel{c}{-}$Val-n-butylamid, 50 mg HOBt und 102 mg DCCI die Titelverbindung erhalten und durch Flash-Chromatographie mit Laufmittel V gereinigt. $R_f$(X) = 0,30 und 0,20 (2 Diastereomere).

Das Ausgangsmaterial wird folgendermassen hergestellt:

a) 2-Benzyl-3-(2-pyridylthio)-propionsäure: 903 mg 2-Benzyl-3-(2-pyridylthio)-propionsäure-äthylester (Beispiel 13c) werden in 10 ml 4N HCl 10 Std. am Rückfluss gekocht. Das Reaktionsgemisch wird mit Methylenchlorid/Methanol 10:1 extrahiert. Die Extrakte werden mit $NaHCO_3$-Lösung auf pH 6 gestellt und anschliessend mit Sole gewaschen, mit $Na_2SO_4$ getrocknet und eingedampft. Der Rückstand wird aus Dioxan/Wasser lyophilisiert. $R_f$(I) = 0,2.

Beispiel 30: N-(2(R)- und 2(S)-Benzyl-3-[1-methyl-2-imidazolylthio]-propionyl)-His-Cha$\overset{c}{-}$Val-n-butylamid

Analog Beispiel 1 wird aus 277 mg 2-Benzyl-3-[1-methyl-2-imidazolylthio]-propionsäure, 560 mg H-His-Cha$\overset{c}{-}$Val-n-butylamid, 175 mg HOBt und 310 mg DCCI die Titelverbindung erhalten und durch Flash-Chromatographie mit Laufmittel O in die beiden Diastereomere aufgetrennt. Diastereomer I: $R_f$(I) = 0,26. Diastereomer II: $R_f$(I) = 0,19.

Das Ausgangsmaterial 2-Benzyl-3-[1-methyl-2-imidazolylthio]-propionsäure wird analog Beispiel 29a) und 1m) aus α-Benzylacrylsäure-äthylester und 2-Mercapto-1-methyl-imidazol hergestellt.

Beispiel 31: N-(2(R,S)-Benzyl-3-phenylsulfonylpropionyl)-His-Cha$\overset{c}{-}$Val-n-butylamid

Analog Beispiel 1 wird aus 150 mg H-His-Cha$\overset{c}{-}$Val-n-butylamid, 151 mg 2-Benzyl-3-phenylsulfonyl-propionsäure, 58 mg HOBt und 103 mg DCCI die Titelverbindung erhalten und durch Flash-Chromatographie gereinigt (Laufmittel H). $R_f$(I) = 0,33 und 0,28 (2 Diastereomere).

Das Ausgangsmaterial 2-Benzyl-3-phenylsulfonyl-propionsäure wird analog Beispiel 1m), n) und o) aus α-Benzylacrylsäure-äthylester und Thiophenol hergestellt.

Beispiel 32: N-(2(R,S)-Benzyl-3-pyrrolidinosulfonylpropionyl)-His-Cha$\overset{c}{-}$Val-n-butylamid

Analog Beispiel 1 wird aus 175 mg 2-Benzyl-3-pyrrolidinosulfonyl-propionsäure, 139 mg H-His-Cha$\overset{c}{-}$Val-n-butylamid, 70 mg HOBt und 120 mg DCCI die Titelverbindung erhalten und durch Flash-Chromatographie mit Laufmittel N gereinigt. $R_f$(I) = 0,24 und 0,19 (2 Diastereomere).

Das Ausgangsmaterial 2-Benzyl-3-pyrrolidinosulfonyl-propionsäure wird analog Beispiel 14a) und b) hergestellt.

Beispiel 33: N-(3-Aethylthio-2(R,S)-benzyl-propionyl)-His-Cha$\overset{C}{-}$Val-n-butylamid

Analog Beispiel 1 wird aus 134 mg 3-Aethylthio-2-benzyl-propionsäure, 185 mg H-His-Cha$\overset{C}{-}$Val-n-butylamid, 70 mg HOBt und 132 mg DCCI die Titelverbindung erhalten und durch Flash-Chromatographie mit Laufmittel H gereinigt. $R_f$(I) = 0,20 und 0,15 (2 Diastereomere).

Das Ausgangsmaterial 3-Aethylthio-2-benzyl-propionsäure wird analog Beispiel 4a) und 1m) aus α-Benzylacrylsäure-äthylester und Aethylmercaptan hergestellt.

Beispiel 34: N-(3-Aethylsulfonyl-2(R)- und 2(S)-benzyl-propionyl)-His-Cha$\overset{C}{-}$Val-n-butylamid

Eine Mischung aus 81 mg N-(3-Aethylthio-2(R,S)-benzyl-propionyl)-His-Cha$\overset{C}{-}$Val-n-butylamid (Beispiel 33), 61 mg 3-Chlorperbenzoesäure und 5 ml Chloroform wird 20 Std. bei 25°C gerührt. Das Reaktionsgemisch wird mit konzentrierter $NaHCO_3$-Lösung basisch gestellt und anschliessend mit 50 ml Chloroform extrahiert. Die organische Phase wird mit 10 ml Sole gewaschen, mit $Na_2SO_4$ getrocknet, filtriert und eingedampft. Der Rückstand wird mittels Flash-Chromatographie (20 g Kieselgel 60, Laufmittel N) getrennt. Die produkthaltigen Fraktionen der beiden Diastereomere werden vereinigt, eingeengt und anschliessend aus Dioxan/Wasser 9:1 lyophilisiert. Diastereomer I: $R_f$(Q) = 0,38. Diastereomer II: $R_f$(Q) = 0,43.

Beispiel 35: N-(2(S)-Benzyl-3-tert-butylsulfonyl-propionyl)-Ser-Cha$\overset{C}{-}$Val-methylamid

Analog Beispiel 1 wird aus 80 mg 2(S)-Benzyl-3-tert-butylsulfonyl-propionsäure, 80 mg H-Ser-Cha$\overset{C}{-}$Val-methylamid, 40 mg HOBt und 67 mg DCCI die Titelverbindung hergestellt und durch Flash-Chromatographie an 65 g Kieselgel 60 (Laufmittel O) gereinigt. $R_f$(O) = 0,28.

Das Ausgangsmaterial wird folgendermassen hergestellt:

a) H-Ser-Cha$\overset{C}{-}$Val-methylamid wird durch Hydrierung von 230 mg Z-Ser-Cha$\overset{C}{-}$Val-methylamid in Gegenwart von 50 mg Palladium-Kohle (10 %) analog Beispiel 1k) erhalten. $R_f$(P) = 0,21.

b) Z-Ser-Cha$\overset{C}{-}$Val-methylamid wird ausgehend von 213 mg Z-Ser-OH, 169 mg H-Cha$\overset{C}{-}$Val-methylamid, 109 mg HOBt und 184 mg DCCI analog Beispiel 1j) erhalten und durch Flash-Chromatographie an 80 g Kieselgel 60 (Laufmittel O) gereinigt. $R_f$(O) = 0,37.

Beispiel 36: N-(2(S)-Benzyl-3-tert-butylsulfonyl-propionyl)-His-Cha$\overset{C}{-}$Val-methylamid

Analog Beispiel 1 wird die Titelverbindung ausgehend von 50 mg H-His-Cha$\overset{C}{-}$Val-methylamid, 37 mg 2(S)-Benzyl-3-tert-butylsulfonyl-propionsäure (Beispiel 9a), 20 mg HOBt und 32 mg DCCI hergestellt und durch Mitteldruck-Chromatographie (1 Lobar®-Säule Grösse B, Laufmittel N) gereinigt. $R_f$(Q) = 0,30.

Beispiel 37: N-(2(S)-Benzyl-3-tert-butylsulfonyl-propionyl)-His-Cha$\overset{C}{-}$Ala-methylamid

Analog Beispiel 1 wird die Titelverbindung ausgehend von 63 mg H-His-Cha$\overset{C}{-}$Ala-methylamid, 60 mg 2(S)-Benzyl-3-tert-butylsulfonyl-propionsäure (Beispiel 9a), 32 mg HOBt und 46 mg DCCI hergestellt und durch Flash-Chromatographie an 50 g Kieselgel 60 (Laufmittel P) gereinigt. $R_f$(P) = 0,24.

Das Ausgangsmaterial wird folgendermassen hergestellt:

a) H-His-Cha$\overset{C}{-}$Ala-methylamid wird durch Hydrierung von 80 mg Z-His-Cha$\overset{C}{-}$Ala-methylamid in Gegenwart von 30 mg Palladium-Kohle (10 %) analog Beispiel 1k) erhalten. $R_f$(P) = 0,11; $R_f$(AA) = 0,22.

b) Z-His-Cha$\overset{C}{-}$Ala-methylamid wird ausgehend von 72 mg Z-His-OH, 53 mg H-Cha$\overset{C}{-}$Ala-methylamid, 35 mg HOBt und 60 mg DCCI analog Beispiel 1j) erhalten und durch Flash-Chromatographie (Laufmittel P) gereinigt. $R_f$(P) = 0,16.

c) H-Cha$\overset{c}{-}$Ala-methylamid: 220 mg BOC-Cha$\overset{cx}{-}$Ala-methylamid werden in 5 ml Trifluoressigsäure/Wasser 95:5 gelöst. Nach 30 Min. wird die Mischung am Rotationsverdampfer eingeengt und der Rückstand durch Flash-Chromatographie (Laufmittel AA) gereinigt. Durch Eindampfen der produkthaltigen Fraktionen erhält man die Titelverbindung. $R_f$(AA) = 0,33.

d) BOC-Cha$\overset{cx}{-}$Ala-methylamid: 185 mg BOC-Cha$\overset{cx}{-}$Ala-OH werden 2 Std. bei Raumtemperatur in einer gerührten Lösung in DMF mit 111 mg HOBt und 149 mg DCCI voraktiviert und anschliessend mit 1 ml einer 5 N Lösung von Methylamin in DMF versetzt. Nach 2 Std. bei Raumtemperatur wird das Lösungsmittel entfernt und die Titelverbindung durch Flash-Chromatographie in System A gereinigt. $R_f$(A) = 0,25.

e) BOC-Cha$\overset{cx}{-}$Ala-OH: 340 mg 3-tert-Butoxycarbonyl-4(S)-cyclohexylmethyl-2,2-dimethyl-5(S)-(3-hydroxy-2(R)-methyl-propyl)-1,3-oxazolidin werden in DMF gelöst und mit 1040 mg Pyridiniumchromat in DMF 16 Std. gerührt. Die Mischung wird am Rotationsverdampfer eingeengt und der Rückstand durch Flash-Chromatographie (Laufmittel B) gereinigt. $R_f$(B) = 0,12.

f) 3-tert-Butoxycarbonyl-4(S)-cyclohexylmethyl-2,2-dimethyl-5(S)-(3-hydroxy-2(R)-methyl-propyl)-1,3-oxazolidin wird durch Hydrierung von 1,90 g 5(S)-(3-Benzyloxy-2(R)-methyl-propyl)-3-tert-butoxycarbonyl-4(S)-cyclohexylmethyl-2,2-dimethyl-1,3-oxazolidin in Methanol in Gegenwart von 200 mg Pd/Kohle (10 %) erhalten. $R_f$(C) = 0,15.

g) 5(S)-(3-Benzyloxy-2(R)-methyl-propyl)-3-tert-butoxycarbonyl-4(S)-cyclohexylmethyl-2,2-dimethyl-1,3-oxazolidin wird analog Beispiel 1e) aus Benzyl-5(S)-tert-butoxycarbonylamino-6-cyclohexyl-4(S)-hydroxy-2(R)-methyl-1-hexyl-äther und 2,2-Dimethoxypropan in Gegenwart von p-Toluolsulfonsäuremonohydrat in Methylenchlorid hergestellt. $R_f$(C) = 0,45.

0236734

h) Benzyl-5(S)-tert-butoxycarbonylamino-6-cyclohexyl-4(S)-hydroxy-2(R)-methyl-1-hexyl-äther: Eine aus 3,6 g (S)-(+)-3-Benzyloxy-2-methyl-propylbromid (A. Fischli et al., Helv. Chim. Acta 60, 925 (1977)) und 360 mg Magnesium in Diäthyläther zubereitete Grignardlösung wird innerhalb von 15 Min. bei 0° zu 1,50 g 2(S)-tert-Butoxycarbonylamino-3-cyclohexyl-propionaldehyd (J. Boger et al., J. Med. Chem. 28, 1779 (1985)) in 15 ml Diäthyläther zugetropft. Nach 30 Min. bei 0° wird die Reaktionslösung auf 50 ml 1 N HCl gegossen und das Rohprodukt (Diastereomerenverhältnis (4S):(4R) = 4:1) mit Dichlormethan extrahiert. Das gewünschte Diastereomere mit der 4(S)-Konfiguration wird durch Flash-Chromatographie an 250 g Kieselgel 60 (Laufmittel Dichlormethan/Aether 20:1) in reiner Form abgetrennt. $R_f$(L) = 0,10.

Beispiel 38: N-(2(S)-Benzyl-3-sulfopropionyl)-His-Cha$^C$Val-n-butyl-amid

Eine Mischung aus 51 mg N-(2(S)-Benzyl-3-[1-methyl-2-imidazolyl-thio]-propionyl)-His-Cha$^C$Val-n-butylamid (Beispiel 30), 64 mg Oxone® (Kaliumperoxomonosulfat, 50 % $KHSO_5$, Ventron), 2 ml 0,1 N $H_2SO_4$ und 2 ml Methanol wird 20 Std. bei 25° gerührt. Das Reaktionsgemisch wird mit Chloroform extrahiert, die Extrakte mit Sole gewaschen, getrocknet und eingeengt. Der Rückstand wird aus Dioxan/Wasser 3:1 lyophilisiert. $R_f$(EE) = 0,2.

Beispiel 39: 5(S)-[N-(2(S)-Benzyl-3-tert-butylsulfonyl-propionyl)-histidinyl]-amino-6-cyclohexyl-4(R)- und 4(S)-hydroxy-2(S)-isopropyl-hexansäure-n-butylamid

Eine Lösung von 37 mg 5(S)-[N-(2(S)-Benzyl-3-tert-butylsulfonyl-propionyl)-histidinyl]-amino-6-cyclohexyl-2(S)-isopropyl-4-oxo-hexansäure-n-butylamid und 8 mg Natriumborhydrid in 3 ml Methanol wird für 6 Std. bei 25°C gerührt. Danach wird mit 2N HCl angesäuert und zwischen 50 ml $CH_2Cl_2$ und 10 ml gesättigter $NaHCO_3$-Lösung verteilt. Die organischen Extrakte werden getrocknet und eingedampft. Der Rückstand wird durch Flash-Chromatographie an 10 g Kieselgel 60 (Laufmittel M) gereinigt. $R_f$(O) = 0,35 (Diastereo-

merengemisch). Das Diastereomerengemisch enthält neben 3 Teilen der 4(R)-Verbindung 1 Teil der 4(S)-Verbindung, die mit der Verbindung von Beispiel 9 identisch ist.

Das Ausgangsmaterial wird folgendermassen hergestellt:

a) 5(S)-[N-(2(S)-Benzyl-3-tert-butylsulfonyl-propionyl)-histidinyl]-amino-6-cyclohexyl-2(S)-isopropyl-4-oxo-hexansäure-n-butylamid:
Eine Mischung aus 253 mg N-(2(S)-Benzyl-tert-butylsulfonyl-propionyl)-His-Cha$\overset{C}{-}$Val-n-butylamid (Beispiel 9), 130 mg Pyridiniumchlorchromat und 5 ml Methylenchlorid wird 10 Std. bei Raumtemperatur gerührt. Das Reaktionsgemisch wird mit 100 ml Essigester verdünnt und anschliessend mit 20 ml Wasser gewaschen. Die organische Phase wird getrocknet und eingedampft und der Rückstand mittels Flash-Chromatographie an 40 g Kieselgel 60 (Laufmittel H) gereinigt. $R_f(O)$ = 0,43.

Beispiel 40: N-(2(S)-Benzyl-3-tert-butylsulfonyl-propionyl)-His-Cha$\overset{C}{-}$Val-n-butylamid durch eine alternative Kondensationsreaktion

Eine Mischung aus 127 mg N-(2(S)-Benzyl-tert-butylsulfonyl-propionyl)-His-OH, 65 mg H-Cha$\overset{C}{-}$Val-n-butylamid, 55 mg N-Hydroxy-norbornan-endo-2,3-dicarbonsäureimid, 62 mg DCCI und 2 ml DMF wird 70 Std. bei Raumtemperatur gerührt. Der kristallisierte DCH wird abfiltriert und das Filtrat eingedampft. Der Rückstand wird in einem Gemisch von Methanol/Wasser/Eisessig 94:3:3 während 30 Min. bei 60°C gerührt und anschliessend eingeengt. Der Rückstand wird mittels Flash-Chromatographie an 40 g Kieselgel 60 (Laufmittel M) gereinigt. Die die Titelverbindung enthaltenden Fraktionen werden vereinigt und aus Dioxan/Wasser 9:1 lyophilisiert. Das Produkt ist identisch mit der Verbindung von Beispiel 9, $R_f(O)$ = 0,35.

Das Augangsmaterial wird folgendermassen hergestellt:

a) N-(2(R)- und 2(S)-Benzyl-3-tert-butylsulfonyl-propionyl)-His-OH: 284 mg 2-Benzyl-3-tert-butylsulfonyl-propionsäure (Beispiel lo), 121 mg N-Hydroxysuccinimid und 227 mg DCCI werden in 4 ml DMF gelöst und 1 Std. bei 25°C gerührt. Der kristallisierte DCH wird abfiltriert und das Filtrat mit einer Lösung von 245 mg Histidin-Natriumsalz in 6 ml DMF/Wasser 2:1 3 Std. bei Raumtemperatur gerührt. Das Reaktionsgemisch wird mit 1 ml 1N HCl verdünnt und eingeengt. Der Rückstand wird durch Flash-Chromatographie aufgetrennt (30 g Kieselgel 60, Laufmittel J). Die produkthaltigen Fraktionen der getrennten Diastereomere werden vereinigt und aus Dioxan/Wasser 5:1 lyophilisiert. 2(R)-Verbindung: $R_f$(EE) = 0,27. 2(S)-Verbindung: $R_f$(EE) = 0,32.

Alternative Darstellungsweise:

b) N-2(S)-Benzyl-3-tert-butylsulfonyl-propionyl)-His-OH wird ausgehend von 4,15 g 2(S)-Benzyl-3-tert-butylsulfonyl-propionsäure, 1,77 g N-Hydroxysuccinimid, 3,62 g DCCI und 3,55 g Histidin-Natriumsalz analog Beispiel a) erhalten. $R_f$(EE) = 0,32.

Beispiel 41: N-(2(R,S)-Benzyl-3-tert-butylsulfonyl-propionyl)-His-Cha$\stackrel{c}{-}$Val-n-butylamid durch Oxidation der Sulfinylverbindung

Eine Mischung aus 142 mg N-(2(R,S)-Benzyl-3-tert-butylsulfinyl-propionyl)-His-Cha$\stackrel{c}{-}$Val-n-butylamid (Beispiel 3), 64 mg Natrium-(meta)perjodat und 10 ml Methanol/Wasser 1:1 wird 20 Std. bei Raumtemperatur gerührt. Das Reaktionsgemisch wird mit 10 ml $NaHCO_3$-Lösung verdünnt und anschliessend mit 100 ml Methylenchlorid extrahiert. Die Methylenchloridextrakte werden mit Sole gewaschen, mit $Na_2SO_4$ getrocknet und eingeengt. Der Rückstand wird mittels Flash-Chromatographie (50 g Kieselgel 60, Laufmittel M) gereinigt. Das Produkt ist identisch mit dem Diastereomerengemisch von Beispiel 2, $R_f$(O) = 0,30 und 0,35 (2 Diastereomere).

Beispiel 42: N-(2(R,S)-Benzyl-3-tert-butylsulfonyl-propionyl)-His-Cha$^{C}$Val-methylamid durch Sulfinat-Addition

Eine Mischung aus 100 mg N-(α-Benzylacryloyl)-His-Cha$^{C}$Val-methylamid und 80 mg Natrium-tert-butylsulfinat werden in THF während 20 Std. bei 50°C gerührt. Das Reaktionsgemisch wird anschliessend teilweise eingeengt und die Titelverbindung durch Flash-Chromatographie an 80 g Kieselgel 60 (Laufmittel O) gereinigt. Das Produkt ist identisch mit dem Diastereomerengemisch von Beispiel 1, $R_f$(Q) = 0,26 und 0,30 (2 Diastereomere).

Das Ausgangsmaterial wird folgendermassen hergestellt:

a) N-(α-Benzylacryloyl)-His-Cha$^{C}$Val-methylamid: Analog Beispiel 1 wird aus 32 mg α-Benzylacrylsäure, 50 mg H-His-Cha$^{C}$Val-methylamid, 20 mg HOBt und 32 mg DCCI die Titelverbindung erhalten und durch Flash-Chromatographie im System Q gereinigt. $R_f$(Q) = 0,36.

Beispiel 43: N-(2(R,S)-Benzyl-3-tert-butylsulfonyl-propionyl)-His-Cha$^{C}$Val-n-butylamid durch Abspaltung einer Silyl-Schutzgruppe

840 mg 5(S)-[N-(2(R,S)-Benzyl-3-tert-butylsulfonyl-propionyl)-histidinyl]-amino-4(S)-tert-butyldimethylsilyloxy-6-cyclohexyl-2(S)-isopropyl-hexansäure-n-butylamid werden in 24 ml Acetonitril gelöst und bei 25° mit 1,25 ml 48 % Fluorwasserstoffsäure versetzt. Nach 30 Min. wird 2,0 g Natriumhydrogencarbonat zugegeben und das Lösungsmittel abgedampft. Der Rückstand wird in Essigester aufgenommen, mit Wasser gewaschen, mit Natriumsulfat getrocknet, wieder eingedampft und durch Flash-Chromatographie an 100 g Kieselgel (Laufmittel M) gereinigt. Das Produkt ist identisch mit dem Diastereomerengemisch von Beispiel 2, $R_f$(O) = 0,30 und 0,35 (2 Diastereomere).

Das Ausgangsmaterial wird folgendermassen hergestellt:

a) 5(S)-[N-(2(R,S)-Benzyl-3-tert-butylsulfonyl-propionyl)-histidinyl]-amino-4(S)-tert-butyldimethylsilyloxy-6-cyclohexyl-2(S)-isopropyl-hexansäure-n-butylamid wird analog Beispiel 1 hergestellt aus 2-Benzyl-3-tert-butylsulfonyl-propionsäure und dem tert-Butyldimethylsilyläther von H-His-Cha$\stackrel{C}{-}$Val-n-butylamid, der analog Beispiel 2a), b) und c) aus dem entsprechenden Aether von Z-Cha$\stackrel{C}{-}$Val-n-butylamid (Beispiel 43b) erhalten wird.

b) 5(S)-Benzyloxycarbonylamino-4(S)-tert-butyldimethylsilyloxy-6-cyclohexyl-2(S)-isopropyl-hexansäure-n-butylamid wird ausgehend von 4,6 g Z-Cha$\stackrel{C}{-}$Val-n-butylamid in Gegenwart von 1,36 g Imidazol und 2,3 g tert-Butyldimethylchlorsilan in 75 ml DMF gebildet.

Beispiel 44: N-(2(R,S)-Benzyl-3-tert-butylsulfonyl-propionyl)-His-Cha$\stackrel{C}{-}$Val-n-butylamid durch Abspaltung der 2-Chloräthoxycarbonyl-Schutzgruppe

830 mg 5(S)-[N-(2(R,S)-Benzyl-3-tert-butylsulfonyl-propionyl)-histidinyl]-amino-4(S)-(2-chloräthoxycarbonyloxy)-6-cyclohexyl-2(S)-isopropyl-hexansäure-n-butylamid werden in 20 ml DMF in eine Lösung von 0,8 g Schwefelkohlenstoff und 2,5 g Natriumsulfid-nonahydrat in DMF eingetropft. Das Gemisch wird 10 Min. auf 40° erwärmt, dann eingedampft. Der Rückstand wird in Essigester aufgenommen, mit 20 ml Wasser gewaschen, mit Natriumsulfat getrocknet, wieder eingedampft und durch Flash-Chromatographie an 100 g Kieselgel (Laufmittel M) gereinigt. Das Produkt ist identisch mit dem Diastereomerengemisch von Beispiel 2, $R_f(O) = 0,30$ und 0,35 (2 Diastereomere).

Das Ausgangsmaterial wird analog Beispiel 43a) und b) aus Z-Cha$\stackrel{C}{-}$Val-n-butylamid und 2-Chloräthoxychloroformiat und Kondensation mit Histidin und 2-Benzyl-3-tert-butylsulfonyl-propionsäure hergestellt.

Beispiel 45: Alternative Reaktionsbedingungen zur Herstellung von N-(2(R,S)-Benzyl-3-tert-butylsulfonyl-propionyl)-His-Cha$\overset{C}{-}$Val-n-butylamid durch Kondensation

a) Mit Bis-(2-oxo-3-oxazolidinyl)-phosphinsäurechlorid: 50 mg 2-Benzyl-3-tert-butylsulfonylpropionsäure, 81 mg H-His-Cha$\overset{C}{-}$Val-n-butylamid und 47 mg Bis-(2-oxo-3-oxazolidinyl)-phosphinsäurechlorid werden während 20 Std. bei Raumtemperatur in 4 ml Acetonitril gerührt. Anschliessend wird das Reaktionsgemisch eingeengt und der Rückstand durch Flash-Chromatographie an 70 g Kieselgel 60 (System Q) gereinigt. $R_f$(O) = 0,30 und 0,35 (2 Diastereomere).

b) Mit 1-Benzotriazolyloxy-tris-(dimethylamino)-phosphonium-hexafluoroborat (Castro's Reagens, BOP): 50 mg 2-Benzyl-3-tert-butylsulfonylpropionsäure, 81 mg H-His-Cha$\overset{C}{-}$Val-n-butylamid und 78 mg des Titelreagens BOP werden während 20 Std. bei Raumtemperatur in 5 ml Methylenchlorid gerührt und wie unter a) aufgearbeitet.

c) Mit 2-Aethoxy-1-äthoxycarbonyl-1,2-dihydrochinolin (EEDQ): 50 mg 2-Benzyl-3-tert-butylsulfonylpropionsäure, 81 mg H-His-Cha$\overset{C}{-}$Val-n-butylamid und 44 mg des Titelreagens EEDQ werden während 20 Std. bei Raumtemperatur in 4 ml THF gerührt und wie unter a) aufgearbeitet.

Beispiel 46: Analog der vorstehenden Beispiele werden hergestellt:

a) N-(2(R,S)-Benzyl-3-sulfamoyl-propionyl)-His-Cha$\overset{C}{-}$Val-n-butylamid.

b) N-(2(R,S)-Benzyl-3-methylaminosulfonyl-propionyl)-His-Cha$\overset{C}{-}$Val-n-butylamid.

c) N-(2(S)-Benzyl-3-tert-butylsulfonyl-propionyl)-His-Cha$\overset{C}{-}$Gly(OH)-methylamid.

d) N-(2(S)-Benzyl-3-tert-butylsulfonyl-propionyl)-His-Cha$\overset{C}{-}$Val-(2-sulfoäthyl)-amid.

e) N-(2(S)-Benzyl-3-tert-butylsulfonyl-propionyl)-His-Cha$\stackrel{c}{-}$Val-(4-aminobutyl)-amid.

<u>Beispiel 47:</u> <u>Gelatine-Lösung</u>

Eine sterilfiltrierte wässrige Lösung von N-(2(S)-Benzyl-3-tert-butylsulfonyl-propionyl)-His-Cha$\stackrel{c}{-}$Val-n-butylamid wird unter Erwärmen mit einer sterilen Gelatinelösung, welche als Konservierungsmittel Phenol enthält, unter aseptischen Bedingungen so vermischt, dass 1,0 ml Lösung folgende Zusammensetzung hat:

| | |
|---|---|
| N-(2(S)-Benzyl-3-tert-butylsulfonyl-propionyl)-His-Cha$\stackrel{c}{-}$Val-n-butylamid | 3 mg |
| Gelatine | 150,0 mg |
| Phenol | 4,7 mg |
| dest. Wasser bis zu | 1,0 ml |

Die Mischung wird unter aseptischen Bedingungen in Vialen zu 1,0 ml abgefüllt.

<u>Beispiel 48:</u> <u>Sterile Trockensubstanz zur Injektion</u>

Man löst 5 mg N-(2(S)-Benzyl-3-tert-butylsulfonyl-propionyl)-His-Cha$\stackrel{c}{-}$Val-n-butylamid in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

<u>Beispiel 49:</u> <u>Nasenspray</u>

In einer Mischung von 3,5 ml Myglyol 812® und 0,08 g Benzylalkohol werden 500 mg fein gemahlenes (<5,0 µm) N-(2(S)-Benzyl-3-tert-butylsulfonyl-propionyl)-His-Cha$\stackrel{c}{-}$Val-n-butylamid suspendiert. Diese Suspension wird in einen Behälter mit Dosierventil eingefüllt. Es werden 5,0 g Freon® 12 unter Druck durch das Ventil in den Behälter

abgefüllt. Durch Schütteln wird das Freon® in der Myglyol-Benzylalkoholmischung gelöst. Dieser Spraybehälter enthält ca. 100 Einzeldosen, die einzeln appliziert werden können.

Beispiel 50: Lacktabletten

Für die Herstellung von 10 000 Tabletten enthaltend je 100 mg Wirkstoff werden folgende Bestandteile verarbeitet:

| | |
|---|---|
| N-(2(S)-Benzyl-3-tert-butylsulfonyl-propionyl)-His-Cha$^{c}$-Val-n-butylamid | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | q.s. |

Ein Gemisch des N-(2(S)-Benzyl-3-tert-butylsulfonyl-propionyl)-His-Cha$^{c}$-Val-n-butylamids, 50 g Maisstärke und der kolloidalen Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Min. im Wirbelschichttrockner getrocknet. Das trockene Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpresst.

Die Presslinge werden in einem Dragierkessel von 45 cm Durchmesser mit einer Lösung von 20 g Schellack und 40 g Hydroxypropylmethylcellulose (niedere Viskosität) in 110 g Methanol und 1350 g Methylenchlorid durch gleichmässiges Aufsprühen während 30 Min. überzogen; dabei wird durch gleichzeitiges Einblasen von Luft von 60° getrocknet.

Anstelle des oben genannten Wirkstoffes kann man auch dieselbe Menge eines anderen Wirkstoffes der vorangehenden Beispiele verwenden.

Patentansprüche für die benannten Vertragsstaaten ausser AT, ES, GR

1. Verbindungen der Formel

$$R_1-A-\overset{R_2}{\overset{|}{N}}-\underset{R_3}{\underset{|}{CH}}-\overset{R_4}{\overset{|}{CH}}-CH_2-\overset{R_5}{\overset{|}{CH}}-\overset{O}{\overset{\|}{C}}-R_6 \qquad (I),$$

worin $R_1$ durch eine Thio-, Sulfinyl- oder Sulfonylgruppe substituiertes Acyl mit Ausnahme eines gegebenenfalls N-substituierten Acylrestes einer natürlichen Aminosäure, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, gegebenenfalls veräthertes oder verestertes Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, veräthertes oder verestertes Hydroxy, $R_5$ Niederalkyl, gegebenenfalls veräthertes oder verestertes Hydroxyniederalkyl, Cycloalkyl, Cycloalkylniederalkyl, Bicycloalkyl, Bicycloalkylniederalkyl, Tricycloalkyl, Tricycloalkylniederalkyl, Aryl, Arylniederalkyl, gegebenenfalls substituiertes Carbamoyl, gegebenenfalls substituiertes Amino, gegebenenfalls substituiertes Hydroxy oder gegebenenfalls substituiertes Mercapto, Sulfinyl oder Sulfonyl und $R_6$ substituiertes Amino mit Ausnahme eines von einer α-Aminosäure abgeleiteten Aminorestes darstellen, ferner Salze von solchen Verbindungen mit salzbildenden Gruppen.

2. Verbindungen gemäss Anspruch 1 der Formel I, worin $R_1$ eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, Hydroxyniederalkyl, Cycloalkyl,

Cycloalkylniederalkyl, Bicycloalkylniederalkyl, Tricycloalkylniederalkyl, Aryl oder Arylniederalkyl, $R_4$ Hydroxy, $R_5$ Niederalkyl mit 2 und mehr C-Atomen, Hydroxyniederalkyl, Cycloalkyl, Cycloalkylniederalkyl, Bicycloalkyl, Bicycloalkylniederalkyl, Tricycloalkyl, Tricycloalkylniederalkyl, Carbamoyl, Niederalkylcarbamoyl, Diniederalkylamino, Aryl oder Arylniederalkyl und $R_6$ eine Aminogruppe, welche durch einen oder gegebenenfalls zwei unsubstituierte oder substituierte, gesättigte oder ungesättigte, aliphatische Kohlenwasserstoffreste mit bis 18 C-Atomen oder einen unsubstituierten oder substituierten aromatischen, heteroaromatischen, aromatisch-aliphatischen oder heteroaromatisch-aliphatischen Kohlenwasserstoffrest mit bis 18 C-Atomen substituiert ist, mit Ausnahme eines von einer α-Aminosäure abgeleiteten Aminorestes, darstellen, ferner pharmazeutisch annehmbare Salze von solchen Verbindungen mit salzbildenden Gruppen.

3. Verbindungen gemäss Anspruch 1 der Formel I, worin $R_1$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{\underset{O}{\|}}{C} - \qquad \text{(Ia)},$$

worin $R^a$ unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, Hydroxy, substituiertes Hydroxy, Amino oder substituiertes Amino, $R^b$ Wasserstoff, Mono-, Bi- oder Tricycloalkyl, unsubstituiertes oder substituiertes Aryl oder unsubstituiertes oder substituiertes Heteroaryl, m 0, 1 oder 2, n 0, 1 oder 2, p 0, 1 oder 2 und q 0, 1, 2, 3 oder 4 darstellen,

A einen bivalenten Rest einer natürlichen α-Aminosäure mit der L-Konfiguration, wie sie normalerweise in Proteinen vorkommen, eines Homologen einer solchen Aminosäure, worin die Aminosäureseitenkette um eine oder zwei Methylengruppen verlängert oder verkürzt ist und/oder eine Methylgruppe durch Wasserstoff ersetzt ist, eines substituierten Phenylalanins oder Phenylglycins, worin der Substituent Niederalkyl, Halogen, Hydroxy, Niederalkoxy, Niederalkanoyloxy, Amino, Niederalkylamino, Diniederalkylamino, Niederalkanoylamino, Niederalkoxycarbonylamino, Arylmethoxycarbonylamino und/oder Nitro sein kann und ein- oder mehrfach vorkommt, eines benzannellierten Phenylalanins oder Phenylglycins, eines hydrierten Phenylalanins oder Phenylglycins, einer fünf- oder sechsgliedrigen cyclischen, benzannellierten α-Aminosäure, einer natürlichen oder homologen α-Aminosäure, in der eine Carboxygruppe der Seitenkette in veresterter oder amidierter Form, eine Aminogruppe der Seitenkette in acylierter Form oder eine Hydroxygruppe der Seitenkette in verätherter oder veresterter Form vorliegt, oder eines Epimeren einer solchen Aminosäure, d.h. mit der unnatürlichen D-Konfiguration, gegebenenfalls am N-Atom durch Niederalkyl substituiert,

$R_2$ Wasserstoff oder Niederalkyl, $R_3$ Niederalkyl, Cycloalkyl, Cycloalkylniederalkyl, Tricycloalkylniederalkyl, Phenylniederalkyl oder Phenyl, $R_4$ Hydroxy, $R_5$ Niederalkyl, Cycloalkyl, Cycloalkylniederalkyl, Bicycloalkyl, Tricycloalkyl, Phenyl, Phenylniederalkyl, Carbamoyl, Niederalkylcarbamoyl, Diniederalkylamino, Hydroxy, Niederalkoxy, Niederalkylthio oder Niederalkylsulfonyl,

und $R_6$ Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Phenoxyniederalkylamino oder Phenoxyhydroxyniederalkylamino, worin Phenoxy gegebenenfalls durch Niederalkyl, Niederalkoxy, Hydroxy, Carboxy, Niederalkoxycarbonyl oder Carbamoyl substituiert ist, Carboxyalkylamino oder Amino-carboxy-alkylamino, worin der Carboxyrest nicht in 1-Stellung des Alkylrest steht, ferner Dicarboxy-methylamino,

Niederalkoxycarbonylalkylamino oder Acylamino-niederalkoxycarbonylalkylamino, worin der Carbonylrest nicht in 1-Stellung des Alkylrests steht, ferner Diniederalkoxycarbonyl-methylamino, physiologisch spaltbares verestertes Carboxyalkylamino, worin die Esterfunktion nicht in 1-Stellung des Alkylrests steht, Carbamoyl- oder Hydroxyniederalkylcarbamoylalkylamino, worin der Carbamoylrest nicht in 1-Stellung des Alkylrests steht, ferner Dicarbamoyl-methylamino, Di-(niederalkylcarbamoyl)-methylamino, Di-(hydroxyniederalkylcarbamoyl)-methylamino oder Bis-(diniederalkylcarbamoyl)-methylamino, Aminoniederalkylamino, Niederalkylaminoniederalkylamino, Diniederalkylaminoniederalkylamino, Niederalkoxycarbonylaminoniederalkylamino, Guanidinoniederalkylamino, über ein Stickstoffatom gebundenes, gesättigtes fünf- oder sechsgliedriges Heterocyclylniederalkylamino, Niederalkenylamino, Niederalkinylamino, Cycloalkylniederalkylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino und/oder durch Nitro mono- oder mehrfach substituiert ist, Pyridylniederalkylamino, Imidazolylniederalkylamino, Indolylniederalkylamino oder Sulfoniederalkylamino, darstellen, sowie pharmazeutisch annehmbare Salze von diesen Verbindungen mit salzbildenden Gruppen.

4. Verbindungen gemäss Anspruch 1 der Formel I, worin $R_1$ einen Rest der Formel

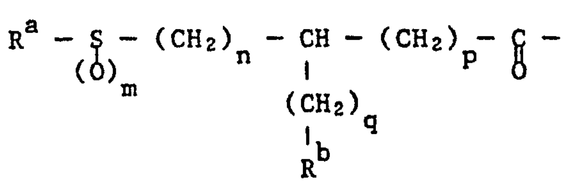

(Ia),

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, Cycloalkyl, Cycloalkylniederalkyl, unsubstituiertes oder durch Niederalkyl, Hydroxy oder Amino substituiertes Aryl, Arylniederalkyl, unsubstituiertes oder durch Oxido, Niederalkyl oder Phenyl substituiertes Heteroaryl, Heteroarylniederalkyl, Hydroxy, Niederalkoxy, Aryloxy, Amino, Niederalkylamino, Diniederalkylamino oder Amino als Teil eines fünf- oder sechsgliedrigen Ringes enthaltend ein Stickstoffatom und gewünschtenfalls ein Sauerstoffatom, $R^b$ Wasserstoff, Cycloalkyl, Aryl oder Heteroaryl, m 0, 1 oder 2, n 0, 1 oder 2, p 0, 1 oder 2 und q 0, 1, 2 oder 3 darstellen,

A den bivalenten Rest der Aminosäuren Alanin, Valin, Norvalin, Leucin, Norleucin, Serin, Prolin, Phenylalanin, β-Phenylserin, α-Naphthylalanin, Cyclohexylalanin, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutamin, Diniederalkylglutamin, Histidin, Lysin oder Ornithin, wobei die Carboxygruppe in der Seitenkette von Asparaginsäure oder Glutaminsäure mit einem Niederalkanol verestert, die Hydroxygruppe in Serin mit Niederalkyl oder mit Benzyl veräthert, die Aminogruppe in der Seitenkette von Lysin oder Ornithin durch Niederalkanoyl, durch Niederalkoxycarbonyl oder durch Arylmethoxycarbonyl acyliert und/oder das α-Stickstoffatom der Aminosäuren durch Niederalkyl substituiert sein kann,

$R_2$ Wasserstoff oder Niederalkyl, $R_3$ Niederalkyl, Cycloalkylniederalkyl oder Tricycloalkylniederalkyl, $R_4$ Hydroxy und $R_5$ Niederalkyl, Cycloalkyl, Cycloalkylniederalkyl, 1-Adamantyl, Benzyl, Carbamoyl, Niederalkylcarbamoyl, Diniederalkylamino, Hydroxy, Niederalkoxy, Niederalkylthio oder Niederalkylsulfonyl,

und $R_6$ Alkylamino mit 1 bis 10 C-Atomen, Diniederalkylamino, Hydroxyniederalkylamino, Carboxyalkylamino oder Amino-carboxy-alkylamino, worin der Carboxyrest nicht in 1-Stellung des Alkylrests steht, ferner Dicarboxy-methylamino, Niederalkoxycarbonylalkylamino oder Acylamino-niederalkoxycarbonyl-alkylamino, worin der Carbonylrest nicht in 1-Stellung des Alkylrests steht, ferner Diniederalkoxycarbonyl-methylamino, physiologisch spaltbares verestertes Carboxyalkylamino, worin die Esterfunktion nicht in 1-Stellung des Alkylrests steht, Carbamoyl- oder Hydroxyniederalkylcarbamoylalkylamino, worin der Carbamoylrest nicht in 1-Stellung des Alkylrests steht, ferner Dicarbamoyl-methylamino, Di-(niederalkylcarbamoyl)-methylamino, Di-(hydroxyniederalkylcarbamoyl)-methylamino oder Bis-(diniederalkylcarbamoyl)-methylamino, Aminoniederalkylamino, Niederalkylaminoniederalkylamino, Diniederalkylaminoniederalkylamino, Niederalkoxycarbonylaminoniederalkylamino, Guanidinoniederalkylamino, Cycloalkylniederalkylamino, Benzylamino, Pyridylniederalkylamino, Imidazolylniederalkylamino, Indolylniederalkylamino oder Sulfoniederalkylamino darstellen, sowie pharmazeutisch annehmbare Salze von diesen Verbindungen mit salzbildenden Gruppen.

5. Verbindungen gemäss Anspruch 1 der Formel I, worin $R_1$ einen Rest der Formel

$$R^a - \underset{(O)_m}{S} - (CH_2)_n - \underset{\underset{R^b}{|}}{\underset{(CH_2)_q}{\underset{|}{CH}}} - (CH_2)_p - \underset{O}{\overset{}{C}} - \qquad (Ia),$$

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, Cycloalkyl, Cycloalkylniederalkyl, unsubstituiertes oder durch Niederalkyl, Hydroxy oder Amino substituiertes Aryl, Arylniederalkyl, unsubstituiertes oder durch Oxido, Niederalkyl oder Phenyl substituiertes Heteroaryl, Heteroarylniederalkyl, Hydroxy, Niederalkoxy, Amino, Niederalkylamino, Diniederalkylamino oder Amino als Teil eines fünf- oder sechsgliedrigen Ringes enthaltend ein Stickstoffatom und gewünschtenfalls ein Sauerstoffatom, $R^b$ Wasserstoff, Cycloalkyl, Aryl oder Heteroaryl, m 0, 1 oder 2, n 0 oder 1, p 0 und q 1 oder 2 darstellen,

A den bivalenten Rest der Aminosäuren Alanin, Serin, Phenylalanin, N-Methyl-phenylalanin, Cyclohexylalanin, Histidin oder N-Methylhistidin, $R_2$ Wasserstoff, $R_3$ Niederalkyl oder Cycloalkylniederalkyl, $R_4$ Hydroxy, $R_5$ Niederalkyl, Hydroxy, Niederalkoxy, Niederalkylthio oder Niederalkylsulfonyl,

und $R_6$ Niederalkylamino mit 1 - 7 C-Atomen, Diniederalkylamino, Hydroxyniederalkylamino, Carboxyalkylamino oder Amino-carboxy-alkylamino, worin der Carboxyrest nicht in 1-Stellung des Alkylrests steht, Niederalkoxycarbonylalkylamino oder Acylamino-niederalkoxycarbonyl-alkylamino, worin der Carbonylrest nicht in 1-Stellung des Alkylrests steht, Aminoniederalkylamino, Diniederalkylaminoniederalkylamino, Niederalkoxycarbonylaminoniederalkylamino, Morpholinoniederalkylamino, Pyridylniederalkylamino, Imidazolylniederalkylamino oder Sulfoniederalkylamino, darstellen, sowie pharmazeutisch annehmbare Salze von diesen Verbindungen mit salzbildenden Gruppen.

6. Verbindungen gemäss Anspruch 1 der Formel I, worin $R_1$ einen Rest der Formel

$$R^a - \underset{(O)_m}{\underset{\|}{S}} - (CH_2)_n - \underset{\underset{R^b}{\underset{|}{(CH_2)_q}}}{\underset{|}{CH}} - (CH_2)_p - \underset{O}{\underset{\|}{C}} - \qquad (Ia),$$

worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, A den bivalenten Rest der Aminosäure Histidin, $R_2$ Wasserstoff, $R_3$ Cyclohexylmethyl, $R_4$ Hydroxy, $R_5$ Isopropyl und $R_6$ Niederalkylamino, Dimethylamino oder 2-Hydroxyäthylamino, darstellen, sowie pharmazeutisch annehmbare Salze von diesen Verbindungen.

7. Verbindungen gemäss Anspruch 1 der Formel I, worin $R_1$ einen Rest der Formel

$$R^a - \underset{(O)_m}{\underset{\|}{S}} - (CH_2)_n - \underset{\underset{R^b}{\underset{|}{(CH_2)_q}}}{\underset{|}{CH}} - (CH_2)_p - \underset{O}{\underset{\|}{C}} - \qquad (Ia),$$

worin $R^a$ Niederalkyl, Phenyl, 2-Pyridyl, Hydroxy, Niederalkylamino, Diniederalkylamino oder Pyrrolidino, $R^b$ Phenyl, m 2, n 1, p 0 und q 1 bedeuten, A den bivalenten Rest der Aminosäure Alanin, Serin oder Histidin, $R_2$ Wasserstoff, $R_3$ Cycloalkylniederalkyl, $R_4$ Hydroxy, $R_5$ Methyl, Isopropyl, Hydroxy, Methoxy, Methylthio oder Methylsulfonyl und $R_6$ Niederalkylamino, 5-Amino-5-carboxy-n-pentylamino, 4-Aminobutylamino, 2-(4-Imidazolyl)-äthylamino oder 2-Sulfoäthylamino darstellen und die die Reste $R_3$ und $R_4$ tragenden C-Atome die S-Konfiguration aufweisen, ferner pharmazeutisch annehmbare Salze von diesen Verbindungen.

8. Die Verbindung gemäss Anspruch 7 der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze.

9. Die Verbindung gemäss Anspruch 7 der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Methyl und $R_6$ Methylamino bedeuten und die die Reste $R_3$ und $R_4$ tragenden C-Atome und das Methin-C-Atom der Teilformel Ia die S-Konfiguration und das den Rest $R_5$ tragende C-Atom die R-Konfiguration aufweisen, und deren pharmazeutisch annehmbare Salze.

10. Die Verbindung gemäss Anspruch 7 der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Alanin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze.

11. Die Verbindung gemäss Anspruch 7 der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cycloheptylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze.

12. Die Verbindung gemäss Anspruch 7 der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ Methylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze.

13. Pharmazeutische Präparate enthaltend Verbindungen gemäss Anspruch 1 der Formel I oder pharmazeutisch verwendbare Salze von diesen Verbindungen mit salzbildenden Gruppen zusammen mit einem pharmazeutischen Trägermaterial.

14. Verbindungen gemäss Anspruch 1 der Formel I oder pharmazeutisch verwendbare Salze dieser Verbindungen zur Anwendung in einem Verfahren zur therapeutischen Behandlung des menschlichen oder tierischen Körpers.

15. Verwendung von Verbindungen gemäss Anspruch 1 der Formel I oder pharmazeutisch verwendbare Salze davon zur Hemmung der Wirkung des Enzyms Renin.

16. Verwendung von Verbindungen gemäss Anspruch 1 der Formel I oder von pharmazeutisch verwendbaren Salzen von Verbindungen der Formel I zur Herstellung eines Arzneimittels zur Behandlung von Bluthochdruck und Herzinsuffizienz.

17. Verfahren zur Herstellung von Verbindungen der Formel I, worin die Substituenten die in Anspruch 1 genannten Bedeutungen haben, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

$$R_1 - A - \overset{R_2}{\overset{|}{N}} - \underset{R_3}{\underset{|}{CH}} - \overset{O}{\overset{\|}{C}} - CH_2 - \overset{R_5}{\overset{|}{CH}} - \overset{O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

$$R_1 - A - \overset{R_2}{\overset{|}{N}} - \underset{R_3}{\underset{|}{CH}} - \overset{O}{\overset{\|}{C}} - H \qquad (III),$$

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

$$M - CH_2 - \overset{R_5}{\overset{|}{CH}} - \overset{O}{\overset{\|}{C}} - R_6 \qquad (IV),$$

worin die Substituenten die genannten Bedeutungen haben und M ein Metallradikal bedeutet, umsetzt und das gebildete Additionsprodukt hydrolysiert, oder

d) in einer Verbindung der Formel

$$R_1 - A - \overset{R_2}{\overset{|}{N}} - \underset{\underset{R_3}{|}}{CH} - \overset{X}{\overset{|}{CH}} - CH_2 - \overset{R_5}{\overset{|}{CH}} - \overset{O}{\overset{\|}{C}} - R_6 \quad (V),$$

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X durch ein den Substituenten $R_4$ in nukleophiler Form einführendes Reagens gegen $R_4$ austauscht, oder

e) in einer Verbindung der Formel

$$R_1 - A - \overset{R_2}{\overset{|}{N}} - \underset{\underset{R_3}{|}}{CH} - \overset{R_4}{\overset{|}{CH}} - CH_2 - \overset{R_5}{\overset{|}{CH}} - CN \quad (VI),$$

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C{=}O)R_6$ überführt, oder

f) zur Herstellung einer Verbindung der Formel I, worin $R_4$ freies Hydroxy bedeutet, ein Epoxid der Formel

$$R_1 - A - \overset{R_2}{\overset{|}{N}} - \underset{\underset{R_3}{|}}{CH} - \overset{O}{\overbrace{CH-CH}} - \overset{R_5}{\overset{|}{CH}} - \overset{O}{\overset{\|}{C}} - R_6 \quad (VII),$$

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, oder

g) zur Herstellung einer Verbindung der Formel I, worin $R_1$ einen Rest der Formel

$$R^a - \underset{(O)_m}{S} - (CH_2)_n - \underset{\substack{| \\ (CH_2)_q \\ | \\ R^b}}{CH} - (CH_2)_p - \underset{\substack{\| \\ O}}{C} - \qquad (Ia)$$

und m 0 oder 2, n 1 und p 0 bedeuten, eine Verbindung der Formel $R^a-S(O)_mH$ oder ein Salz davon an eine Verbindung der Formel

$$R^b-(CH_2)_q - \underset{\substack{\| \\ CH_2}}{C} - \overset{\substack{O \\ \|}}{C} - A - \overset{\substack{R_2 \\ |}}{N} - \underset{\substack{| \\ R_3}}{CH} - \overset{\substack{R_4 \\ |}}{CH} - CH_2 - \overset{\substack{R_5 \\ |}}{CH} - \overset{\substack{O \\ \|}}{C} - R_6 \qquad (VIII),$$

worin die Substituenten die genannten Bedeutungen haben und freie funktionelle Gruppen gegebenenfalls in geschützter Form vorliegen, addiert, oder

h) zur Herstellung einer Verbindung der Formel I, worin $R_1$ einen Rest der Formel

$$R^a - \underset{(O)_m}{S} - (CH_2)_n - \underset{\substack{| \\ (CH_2)_q \\ | \\ R^b}}{CH} - (CH_2)_p - \underset{\substack{\| \\ O}}{C} - \qquad (Ia)$$

und p 0 bedeutet, eine Verbindung der Formel

$$R^a - \underset{(O)_m}{S} - (CH_2)_n - CH_2 - \underset{\substack{\| \\ O}}{C} - A - \overset{\substack{R_2 \\ |}}{N} - \underset{\substack{| \\ R_3}}{CH} - \overset{\substack{R_4 \\ |}}{CH} - CH_2 - \overset{\substack{R_5 \\ |}}{CH} - \overset{\substack{O \\ \|}}{C} - R_6 \qquad (IX),$$

worin die Substituenten die genannten Bedeutungen haben und freie funktionelle Gruppen gegebenenfalls in geschützter Form vorliegen, mit einer den Rest $R^b-(CH_2)q-$ einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine

erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

18. Die nach einem der Verfahren des Anspruchs 17 erhältlichen Verbindungen und ihre Salze.

FO 7.4 KB/cc*

<u>Patentansprüche</u> für die Vertragsstaaten AT, ES, GR

1. Verfahren zur Herstellung von Verbindungen der Formel

$$R_1\text{-}A\text{-}\overset{R_2}{\overset{|}{N}}\text{-}\underset{R_3}{\underset{|}{CH}}\text{-}\overset{R_4}{\overset{|}{CH}}\text{-}CH_2\text{-}\overset{R_5}{\overset{|}{CH}}\text{-}\overset{O}{\overset{\|}{C}}\text{-}R_6 \qquad (I),$$

worin $R_1$ durch eine Thio-, Sulfinyl- oder Sulfonylgruppe substituiertes Acyl mit Ausnahme eines gegebenenfalls N-substituierten Acylrestes einer natürlichen Aminosäure, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, gegebenenfalls veräthertes oder verestertes Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, veräthertes oder verestertes Hydroxy, $R_5$ Niederalkyl, gegebenenfalls veräthertes oder verestertes Hydroxyniederalkyl, Cycloalkyl, Cycloalkylniederalkyl, Bicycloalkyl, Bicycloalkylniederalkyl, Tricycloalkyl, Tricycloalkylniederalkyl, Aryl, Arylniederalkyl, gegebenenfalls substituiertes Carbamoyl, gegebenenfalls substituiertes Amino, gegebenenfalls substituiertes Hydroxy oder gegebenenfalls substituiertes Mercapto, Sulfinyl oder Sulfonyl und $R_6$ substituiertes Amino mit Ausnahme eines von einer α-Aminosäure abgeleiteten Aminorestes darstellen, ferner von Salzen von solchen Verbindungen mit salzbildenden Gruppen, dadurch gekennzeichnet, dass man

a) ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert, oder

b) zur Herstellung von Verbindungen der Formel I, worin $R_4$ Hydroxy bedeutet, die Ketogruppe in einer Verbindung der Formel

$$R_1 - A - \overset{R_2}{\overset{|}{N}} - \underset{\underset{R_3}{|}}{CH} - \overset{O}{\overset{\|}{C}} - CH_2 - \overset{R_5}{\overset{|}{CH}} - \overset{O}{\overset{\|}{C}} - R_6 \qquad (II),$$

worin die Substituenten die genannten Bedeutungen haben und freie funktionelle Gruppen mit Ausnahme der an der Reaktion teilnehmenden Ketogruppe gegebenenfalls in geschützter Form vorliegen, durch Umsetzung mit einem geeigneten Reduktionsmittel zu einer Hydroxygruppe reduziert, oder

c) zur Herstellung von Verbindungen der Formel I, worin $R_4$ Hydroxy bedeutet, eine Aldehyd-Verbindung der Formel

$$R_1 - A - \overset{R_2}{\overset{|}{N}} - \underset{\underset{R_3}{|}}{CH} - \overset{O}{\overset{\|}{C}} - H \qquad (III),$$

worin die Substituenten die genannten Bedeutungen haben und freie funktionelle Gruppen mit Ausnahme der Aldehydgruppe gegebenenfalls in geschützter Form vorliegen, mit einer Organometallverbindung der Formel

$$M - CH_2 - \overset{R_5}{\overset{|}{CH}} - \overset{O}{\overset{\|}{C}} - R_6 \qquad (IV),$$

worin die Substituenten die genannten Bedeutungen haben und M ein Metallradikal bedeutet, umsetzt und das gebildete Additionsprodukt hydrolysiert, oder

d) in einer Verbindung der Formel

$$R_1 - A - \overset{R_2}{\overset{|}{N}} - \underset{\underset{R_3}{|}}{CH} - \overset{X}{\overset{|}{CH}} - CH_2 - \overset{R_5}{\overset{|}{CH}} - \overset{O}{\overset{\|}{C}} - R_6 \qquad (V),$$

worin X eine nukleofuge Abgangsgruppe ist, die übrigen Substituenten die oben genannten Bedeutungen haben und freie funktionelle Gruppen gegebenenfalls in geschützter Form vorliegen, den Substituenten X durch ein den Substituenten $R_4$ in nukleophiler Form einführendes Reagens gegen $R_4$ austauscht, oder

e) in einer Verbindung der Formel

$$R_1 - A - \overset{R_2}{\overset{|}{N}} - \underset{R_3}{\underset{|}{CH}} - \overset{R_4}{\overset{|}{CH}} - CH_2 - \overset{R_5}{\overset{|}{CH}} - CN \quad (VI),$$

worin die Substituenten die genannten Bedeutungen haben und vorhandene funktionelle Gruppen gegebenenfalls in geschützter Form vorliegen, die Cyanogruppe in eine N-substituierte Carboxamidogruppe $-(C{=}O)R_6$ überführt, oder

f) zur Herstellung einer Verbindung der Formel I, worin $R_4$ freies Hydroxy bedeutet, ein Epoxid der Formel

$$R_1 - A - \overset{R_2}{\overset{|}{N}} - \underset{R_3}{\underset{|}{CH}} - \overset{O}{\overbrace{CH-CH}} - \overset{R_5}{\overset{|}{CH}} - \overset{O}{\overset{\|}{C}} - R_6 \quad (VII),$$

worin die Substituenten die genannten Bedeutungen haben und freie funktionelle Gruppen gegebenenfalls in geschützter Form vorliegen, mit einem regio-selektiven Reduktionsmittel zum entsprechenden Alkohol reduziert, oder

g) zur Herstellung einer Verbindung der Formel I, worin $R_1$ einen Rest der Formel

$$R^a - \underset{(O)_m}{\underset{\|}{S}} - (CH_2)_n - \underset{\underset{R^b}{|}}{\underset{(CH_2)_q}{\underset{|}{CH}}} - (CH_2)_p - \underset{O}{\underset{\|}{C}} - \quad (Ia)$$

und m 0 oder 2, n 1 und p 0 bedeuten, eine Verbindung der Formel $R^a{-}S(O)_mH$ oder ein Salz davon an eine Verbindung der Formel

$$R^b-(CH_2)_q-\underset{\underset{CH_2}{\|}}{C}-\overset{\overset{O}{\|}}{C}-A-\overset{\overset{R_2}{|}}{N}-\underset{\underset{R_3}{|}}{CH}-\overset{\overset{R_4}{|}}{CH}-CH_2-\overset{\overset{R_5}{|}}{CH}-\overset{\overset{O}{\|}}{C}-R_6 \qquad \text{(VIII)},$$

worin die Substituenten die genannten Bedeutungen haben und freie funktionelle Gruppen gegebenenfalls in geschützter Form vorliegen, addiert, oder

h) zur Herstellung einer Verbindung der Formel I, worin $R_1$ einen Rest der Formel

$$R^a-\underset{\underset{(O)_m}{\|}}{S}-(CH_2)_n-\underset{\underset{\underset{R^b}{|}}{(CH_2)_q}}{\overset{}{CH}}-(CH_2)_p-\underset{\underset{O}{\|}}{C}- \qquad \text{(Ia)}$$

und p 0 bedeutet, eine Verbindung der Formel

$$R^a-\underset{\underset{(O)_m}{\|}}{S}-(CH_2)_n-CH_2-\underset{\underset{O}{\|}}{C}-A-\overset{\overset{R_2}{|}}{N}-\underset{\underset{R_3}{|}}{CH}-\overset{\overset{R_4}{|}}{CH}-CH_2-\overset{\overset{R_5}{|}}{CH}-\overset{\overset{O}{\|}}{C}-R_6 \qquad \text{(IX)},$$

worin die Substituenten die genannten Bedeutungen haben und freie funktionelle Gruppen gegebenenfalls in geschützter Form vorliegen, mit einer den Rest $R^b-(CH_2)q-$ einführenden Verbindung alkyliert, und gewünschtenfalls

i) in einer erhältlichen Verbindung vorhandene Schutzgruppen abspaltet und/oder gewünschtenfalls nach Ausführung eines der vorstehend genannten Verfahren a) - h) oder eines beliebigen anderen Verfahrens zur Herstellung einer Verbindung der Formel I eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder in einer erhältlichen Verbindung der Formel I die Konfiguration eines chiralen Kohlenstoffatoms umkehrt und/oder eine erfindungsgemässe Verbindung der Formel I in eine andere erfindungsgemässe Verbindung der Formel I umwandelt.

2. Verfahren zur Herstellung von Verbindungen der Formel I gemäss Anspruch 1, dadurch gekennzeichnet, dass man ein Fragment einer Verbindung der Formel I mit einer endständigen Carboxygruppe oder ein reaktionsfähiges Säurederivat dieses Fragments mit einem zur Verbindung der Formel I komplementären Fragment mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der Reaktion teilnehmenden Gruppen gegebenenfalls in geschützter Form vorliegen, unter Bildung einer Amidbindung kondensiert und gewünschtenfalls in einer erhältlichen Verbindung vorhandene Schutzgruppen durch Behandlung mit Säure, mit Base, durch Hydrogenolyse oder durch Behandlung mit einem Reduktionsmittel abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

3. Verfahren zur Herstellung von Verbindungen der Formel I gemäss Anspruch 1, dadurch gekennzeichnet, dass man ein Fragment einer Verbindung der Formel I mit einer endständigen Carboxygruppe oder ein davon abgeleiteter aktivierter Ester, reaktionsfähiges Anhydrid oder reaktionsfähiges cyclisches Amid mit einem zur Verbindung der Formel I komplementären Fragment mit einer freien Aminogruppe, wobei in den Reaktionskomponenten vorhandene freie funktionelle Gruppen mit Ausnahme der an der Reaktion teilnehmenden Gruppen gegebenenfalls in geschützter Form vorliegen, in Gegenwart eines Kondensationsmittels ausgewählt aus einem Carbodiimid, Carbonyldiimidazol, 1,2-Oxazoliumverbindungen, einem 1,2-Dihydrochinolin oder einer aktivierten Phosphorsäureverbindung, und gewünschtenfalls in Gegenwart einer organischen Base oder von Alkalimetallcarbonaten in einem inerten, polaren, aprotischen Lösungsmittel in einem Temperaturbereich von -40°C bis +100°C kondensiert, wobei aktivierte Ester vom Amino- oder Amidoester-Typ gewünschtenfalls in situ durch Zugabe

einer N-Hydroxyamino- oder N-Hydroxyamido-Verbindung gebildet werden, und gewünschtenfalls in einer erhältlichen Verbindung vorhandene Schutzgruppen durch Behandlung mit Säure, mit Base, durch Hydrogenolyse oder durch Behandlung mit einem Reduktionsmittel abspaltet und/oder eine erhältliche Verbindung der Formel I mit einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemässe Verbindung der Formel I in eine andere erfindungsgemässe Verbindung der Formel I umwandelt.

4. Verfahren gemäss einem der Ansprüche 1-3, dadurch gekennzeichnet, dass man solche Ausgangsstoffe wählt, dass Verbindungen der Formel I, worin $R_1$ eine durch eine Thio-, Sulfinyl- oder Sulfonylgruppe und gegebenenfalls durch weitere, Heteroatome enthaltende Gruppen substituierte Acylgruppe einer gesättigten oder ungesättigten, aliphatischen, cycloaliphatischen, cycloaliphatisch-aliphatischen, aromatischen, aromatisch-aliphatischen, heteroaromatischen oder heteroaromatisch-aliphatischen Carbonsäure mit Ausnahme der gegebenenfalls N-substituierten natürlichen Aminosäure Methionin, A einen gegebenenfalls N-alkylierten α-Aminosäurerest, der N-terminal mit $R_1$ und C-terminal mit der Gruppe $-NR_2-$ verbunden ist, $R_2$ Wasserstoff oder Niederalkyl, $R_3$ Wasserstoff, Niederalkyl, Hydroxyniederalkyl, Cycloalkyl, Cycloalkylniederalkyl, Bicycloalkylniederalkyl, Tricycloalkylniederalkyl, Aryl oder Arylniederalkyl, $R_4$ Hydroxy, $R_5$ Niederalkyl mit 2 und mehr C-Atomen, Hydroxyniederalkyl, Cycloalkyl, Cycloalkylniederalkyl, Bicycloalkyl, Bicycloalkylniederalkyl, Tricycloalkyl, Tricycloalkylniederalkyl, Carbamoyl, Niederalkylcarbamoyl, Diniederalkylamino, Aryl oder Arylniederalkyl und $R_6$ eine Aminogruppe, welche durch einen oder gegebenenfalls zwei unsubstituierte oder substituierte, gesättigte oder ungesättigte, aliphatische Kohlenwasserstoffreste mit bis 18 C-Atomen oder einen unsubstituierten oder substituierten aromatischen, heteroaromatischen, aromatisch-aliphatischen oder heteroaromatisch-aliphatischen Kohlenwasserstoffrest mit bis 18 C-Atomen substituiert ist,

mit Ausnahme eines von einer α-Aminosäure abgeleiteten Aminorestes, darstellen, ferner pharmazeutisch annehmbare Salze von solchen Verbindungen mit salzbildenden Gruppen gebildet werden.

5. Verfahren gemäss einem der Ansprüche 1-3, dadurch gekennzeichnet, dass man solche Ausgangsstoffe wählt, dass Verbindungen der Formel I, worin $R_1$ einen Rest der Formel

$$R^a - \underset{(O)_m}{\overset{}{S}} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia),$$

worin $R^a$ unsubstituiertes oder substituiertes Niederalkyl, Niederalkenyl, Niederalkinyl, Mono-, Bi- oder Tricycloalkyl, Cycloalkylniederalkyl, unsubstituiertes oder substituiertes Aryl, Arylniederalkyl, Arylniederalkenyl, unsubstituiertes oder substituiertes Heteroaryl, Heteroarylniederalkyl, Hydroxy, substituiertes Hydroxy, Amino oder substituiertes Amino, $R^b$ Wasserstoff, Mono-, Bi- oder Tricycloalkyl, unsubstituiertes oder substituiertes Aryl oder unsubstituiertes oder substituiertes Heteroaryl, m 0, 1 oder 2, n 0, 1 oder 2, p 0, 1 oder 2 und q 0, 1, 2, 3 oder 4 darstellen,

A einen bivalenten Rest einer natürlichen α-Aminosäure mit der L-Konfiguration, wie sie normalerweise in Proteinen vorkommen, eines Homologen einer solchen Aminosäure, worin die Aminosäureseitenkette um eine oder zwei Methylengruppen verlängert oder verkürzt ist und/oder eine Methylgruppe durch Wasserstoff ersetzt ist, eines substituierten Phenylalanins oder Phenylglycins, worin der Substituent Niederalkyl, Halogen, Hydroxy, Niederalkoxy, Niederalkanoyloxy, Amino, Niederalkylamino, Diniederalkylamino, Niederalkanoylamino, Niederalkoxycarbonylamino, Arylmethoxycarbonylamino und/oder Nitro sein kann und ein- oder mehrfach vorkommt, eines benzannellierten Phenylalanins oder Phenylglycins, eines hydrierten Phenylalanins oder Phenylglycins, einer fünf- oder sechsgliedrigen cyclischen, benzannellierten α-Aminosäure, einer natürlichen oder

homologen α-Aminosäure, in der eine Carboxygruppe der Seitenkette in veresterter oder amidierter Form, eine Aminogruppe der Seitenkette in acylierter Form oder eine Hydroxygruppe der Seitenkette in verätherter oder veresterter Form vorliegt, oder eines Epimeren einer solchen Aminosäure, d.h. mit der unnatürlichen D-Konfiguration, gegebenenfalls am N-Atom durch Niederalkyl substituiert,

$R_2$ Wasserstoff oder Niederalkyl, $R_3$ Niederalkyl, Cycloalkyl, Cycloalkylniederalkyl, Tricycloalkylniederalkyl, Phenylniederalkyl oder Phenyl, $R_4$ Hydroxy, $R_5$ Niederalkyl, Cycloalkyl, Cycloalkylniederalkyl, Bicycloalkyl, Tricycloalkyl, Phenyl, Phenylniederalkyl, Carbamoyl, Niederalkylcarbamoyl, Diniederalkylamino, Hydroxy, Niederalkoxy, Niederalkylthio oder Niederalkylsulfonyl,

und $R_6$ Alkylamino mit 1 bis 10 C-Atomen, Diniederalkylamino, Hydroxyniederalkylamino, Di-(hydroxyniederalkyl)-amino, Niederalkoxyniederalkylamino, Niederalkanoyloxyniederalkylamino, Phenoxyniederalkylamino oder Phenoxyhydroxyniederalkylamino, worin Phenoxy gegebenenfalls durch Niederalkyl, Niederalkoxy, Hydroxy, Carboxy, Niederalkoxycarbonyl oder Carbamoyl substituiert ist, Carboxyalkylamino oder Amino-carboxy-alkylamino, worin der Carboxyrest nicht in 1-Stellung des Alkylrest steht, ferner Dicarboxy-methylamino, Niederalkoxycarbonylalkylamino oder Acylamino-niederalkoxycarbonylalkylamino, worin der Carbonylrest nicht in 1-Stellung des Alkylrests steht, ferner Diniederalkoxycarbonyl-methylamino, physiologisch spaltbares verestertes Carboxyalkylamino, worin die Esterfunktion nicht in 1-Stellung des Alkylrests steht, Carbamoyl- oder Hydroxyniederalkylcarbamoylalkylamino, worin der Carbamoylrest nicht in 1-Stellung des Alkylrests steht, ferner Dicarbamoyl-methylamino, Di-(niederalkylcarbamoyl)-methylamino, Di-(hydroxyniederalkylcarbamoyl)-methylamino oder Bis-(diniederalkylcarbamoyl)-methylamino, Aminoniederalkylamino, Niederalkylaminoniederalkylamino, Diniederalkylaminoniederalkylamino, Niederalkoxycarbonylaminoniederalkylamino, Guanidinoniederalkylamino, über ein Stickstoffatom gebundenes, gesättigtes fünf- oder sechsgliedriges Heterocyclylniederalkylamino, Niederalkenylamino, Niederalkinylamino,

Cycloalkylniederalkylamino, Phenylamino oder Phenylniederalkylamino, worin Phenyl gegebenenfalls durch Niederalkyl, Hydroxy, Niederalkoxy, Niederalkanoyloxy, Halogen, Carboxy, Niederalkoxycarbonyl, Carbamoyl, Amino, Niederalkylamino, Diniederalkylamino, Acylamino und/oder durch Nitro mono- oder mehrfach substituiert ist, Pyridylniederalkylamino, Imidazolylniederalkylamino, Indolylniederalkylamino oder Sulfoniederalkylamino, darstellen, sowie pharmazeutisch annehmbare Salze von diesen Verbindungen mit salzbildenden Gruppen gebildet werden.

6. Verfahren gemäss einem der Ansprüche 1-3, dadurch gekennzeichnet, dass man solche Ausgangsstoffe wählt, dass Verbindungen der Formel I, worin $R_1$ einen Rest der Formel

$$R^a - \underset{(O)_m}{S} - (CH_2)_n - \underset{\substack{|\\ (CH_2)_q \\ | \\ R^b}}{CH} - (CH_2)_p - \underset{O}{\overset{\parallel}{C}} - \qquad (Ia),$$

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, Cycloalkyl, Cycloalkylniederalkyl, unsubstituiertes oder durch Niederalkyl, Hydroxy oder Amino substituiertes Aryl, Arylniederalkyl, unsubstituiertes oder durch Oxido, Niederalkyl oder Phenyl substituiertes Heteroaryl, Heteroarylniederalkyl, Hydroxy, Niederalkoxy, Aryloxy, Amino, Niederalkylamino, Diniederalkylamino oder Amino als Teil eines fünf- oder sechsgliedrigen Ringes enthaltend ein Stickstoffatom und gewünschtenfalls ein Sauerstoffatom, $R^b$ Wasserstoff, Cycloalkyl, Aryl oder Heteroaryl, m 0, 1 oder 2, n 0, 1 oder 2, p 0, 1 oder 2 und q 0, 1, 2 oder 3 darstellen,

A den bivalenten Rest der Aminosäuren Alanin, Valin, Norvalin, Leucin, Norleucin, Serin, Prolin, Phenylalanin, β-Phenylserin, α-Naphthylalanin, Cyclohexylalanin, Indolin-2-carbonsäure, 1,2,3,4-Tetrahydroisochinolin-3-carbonsäure, Asparaginsäure, Asparagin, Aminomalonsäure, Aminomalonsäuremonoamid, Glutaminsäure, Glutamin,

Diniederalkylglutamin, Histidin, Lysin oder Ornithin, wobei die Carboxygruppe in der Seitenkette von Asparaginsäure oder Glutaminsäure mit einem Niederalkanol verestert, die Hydroxygruppe in Serin mit Niederalkyl oder mit Benzyl veräthert, die Aminogruppe in der Seitenkette von Lysin oder Ornithin durch Niederalkanoyl, durch Niederalkoxycarbonyl oder durch Arylmethoxycarbonyl acyliert und/oder das α-Stickstoffatom der Aminosäuren durch Niederalkyl substituiert sein kann,

$R_2$ Wasserstoff oder Niederalkyl, $R_3$ Niederalkyl, Cycloalkylniederalkyl oder Tricycloalkylniederalkyl, $R_4$ Hydroxy und $R_5$ Niederalkyl, Cycloalkyl, Cycloalkylniederalkyl, 1-Adamantyl, Benzyl, Carbamoyl, Niederalkylcarbamoyl, Diniederalkylamino, Hydroxy, Niederalkoxy, Niederalkylthio oder Niederalkylsulfonyl,

und $R_6$ Alkylamino mit 1 bis 10 C-Atomen, Diniederalkylamino, Hydroxyniederalkylamino, Carboxyalkylamino oder Amino-carboxy-alkylamino, worin der Carboxyrest nicht in 1-Stellung des Alkylrests steht, ferner Dicarboxy-methylamino, Niederalkoxycarbonylalkylamino oder Acylamino-niederalkoxycarbonyl-alkylamino, worin der Carbonylrest nicht in 1-Stellung des Alkylrests steht, ferner Diniederalkoxycarbonyl-methylamino, physiologisch spaltbares verestertes Carboxyalkylamino, worin die Esterfunktion nicht in 1-Stellung des Alkylrests steht, Carbamoyl- oder Hydroxyniederalkylcarbamoylalkylamino, worin der Carbamoylrest nicht in 1-Stellung des Alkylrests steht, ferner Dicarbamoyl-methylamino, Di-(niederalkylcarbamoyl)-methylamino, Di-(hydroxyniederalkylcarbamoyl)-methylamino oder Bis-(diniederalkylcarbamoyl)-methylamino, Aminoniederalkylamino, Niederalkylaminoniederalkylamino, Diniederalkylaminoniederalkylamino, Niederalkoxycarbonylaminoniederalkylamino, Guanidinoniederalkylamino, Cycloalkylniederalkylamino, Benzylamino, Pyridylniederalkylamino, Imidazolylniederalkylamino, Indolylniederalkylamino oder Sulfoniederalkylamino darstellen, sowie pharmazeutisch annehmbare Salze von diesen Verbindungen mit salzbildenden Gruppen gebildet werden.

7. Verfahren gemäss einem der Ansprüche 1-3, dadurch gekennzeichnet, dass man solche Ausgangsstoffe wählt, dass Verbindungen der Formel I, worin $R_1$ einen Rest der Formel

$$R^a - \underset{(\overset{\|}{O})_m}{S} - (CH_2)_n - \underset{\underset{R^b}{|}}{\underset{(CH_2)_q}{\underset{|}{CH}}} - (CH_2)_p - \underset{O}{\overset{}{\underset{\|}{C}}} - \qquad (Ia),$$

worin $R^a$ unsubstituiertes oder durch Hydroxy, Niederalkoxy, Phenoxy, Niederalkanoyloxy, Carboxy, Niederalkoxycarbonyl oder Carbamoyl substituiertes Niederalkyl, Cycloalkyl, Cycloalkylniederalkyl, unsubstituiertes oder durch Niederalkyl, Hydroxy oder Amino substituiertes Aryl, Arylniederalkyl, unsubstituiertes oder durch Oxido, Niederalkyl oder Phenyl substituiertes Heteroaryl, Heteroarylniederalkyl, Hydroxy, Niederalkoxy, Amino, Niederalkylamino, Diniederalkylamino oder Amino als Teil eines fünf- oder sechsgliedrigen Ringes enthaltend ein Stickstoffatom und gewünschtenfalls ein Sauerstoffatom, $R^b$ Wasserstoff, Cycloalkyl, Aryl oder Heteroaryl, m 0, 1 oder 2, n 0 oder 1, p 0 und q 1 oder 2 darstellen,

A den bivalenten Rest der Aminosäuren Alanin, Serin, Phenylalanin, N-Methyl-phenylalanin, Cyclohexylalanin, Histidin oder N-Methylhistidin, $R_2$ Wasserstoff, $R_3$ Niederalkyl oder Cycloalkylniederalkyl, $R_4$ Hydroxy, $R_5$ Niederalkyl, Hydroxy, Niederalkoxy, Niederalkylthio oder Niederalkylsulfonyl,

und $R_6$ Niederalkylamino mit 1 - 7 C-Atomen, Diniederalkylamino, Hydroxyniederalkylamino, Carboxyalkylamino oder Amino-carboxy-alkylamino, worin der Carboxyrest nicht in 1-Stellung des Alkylrests steht, Niederalkoxycarbonylalkylamino oder Acylamino-niederalkoxycarbonyl-alkylamino, worin der Carbonylrest nicht in 1-Stellung des Alkylrests steht, Aminoniederalkylamino, Diniederalkylaminoniederalkylamino, Niederalkoxycarbonylaminoniederalkylamino, Morpholinoniederalkylamino, Pyridylniederalkylamino, Imidazolylniederalkylamino oder Sulfoniederalkylamino, darstellen, sowie pharmazeutisch annehmbare Salze von diesen Verbindungen mit salzbildenden Gruppen gebildet werden.

8. Verfahren gemäss einem der Ansprüche 1-3, dadurch gekennzeichnet, dass man solche Ausgangsstoffe wählt, dass Verbindungen der Formel I, worin $R_1$ einen Rest der Formel

$$R^a-\underset{(O)_m}{S}-(CH_2)_n-\underset{\substack{|\\(CH_2)_q\\|\\R^b}}{CH}-(CH_2)_p-\underset{\substack{\|\\O}}{C}- \qquad (Ia),$$

worin $R^a$ unsubstituiertes oder durch Hydroxy oder Niederalkoxy substituiertes Niederalkyl, Phenyl, Benzyl oder unsubstituiertes oder durch Oxido oder Niederalkyl substituiertes Heteroaryl mit 1 oder 2 Stickstoffatomen, $R^b$ Cyclohexyl oder Phenyl, m 0, 1 oder 2, n 1, p 0 und q 1 oder 2 darstellen, A den bivalenten Rest der Aminosäure Histidin, $R_2$ Wasserstoff, $R_3$ Cyclohexylmethyl, $R_4$ Hydroxy, $R_5$ Isopropyl und $R_6$ Niederalkylamino, Dimethylamino oder 2-Hydroxyäthylamino, darstellen, sowie pharmazeutisch annehmbare Salze von diesen Verbindungen gebildet werden.

9. Verfahren gemäss einem der Ansprüche 1-3, dadurch gekennzeichnet, dass man solche Ausgangsstoffe wählt, dass Verbindungen der Formel I, worin $R_1$ einen Rest der Formel

$$R^a-\underset{(O)_m}{S}-(CH_2)_n-\underset{\substack{|\\(CH_2)_q\\|\\R^b}}{CH}-(CH_2)_p-\underset{\substack{\|\\O}}{C}- \qquad (Ia),$$

worin $R^a$ Niederalkyl, Phenyl, 2-Pyridyl, Hydroxy, Niederalkylamino, Diniederalkylamino oder Pyrrolidino, $R^b$ Phenyl, m 2, n 1, p 0 und q 1 bedeuten, A den bivalenten Rest der Aminosäure Alanin, Serin oder Histidin, $R_2$ Wasserstoff, $R_3$ Cycloalkylniederalkyl, $R_4$ Hydroxy, $R_5$ Methyl, Isopropyl, Hydroxy, Methoxy, Methylthio oder Methylsulfonyl

und $R_6$ Niederalkylamino, 5-Amino-5-carboxy-n-pentylamino, 4-Aminobutylamino, 2-(4-Imidazolyl)-äthylamino oder 2-Sulfoäthylamino darstellen und die die Reste $R_3$ und $R_4$ tragenden C-Atome die S-Konfiguration aufweisen, ferner pharmazeutisch annehmbare Salze von diesen Verbindungen gebildet werden.

10. Verfahren gemäss Anspruch 9, dadurch gekennzeichnet, dass man solche Ausgangsstoffe wählt, dass die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze gebildet werden.

11. Verfahren gemäss Anspruch 9, dadurch gekennzeichnet, dass man solche Ausgangsstoffe wählt, dass die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Methyl und $R_6$ Methylamino bedeuten und die die Reste $R_3$ und $R_4$ tragenden C-Atome und das Methin-C-Atom der Teilformel Ia die S-Konfiguration und das den Rest $R_5$ tragende C-Atom die R-Konfiguration aufweisen, und deren pharmazeutisch annehmbare Salze gebildet werden.

12. Verfahren gemäss Anspruch 9, dadurch gekennzeichnet, dass man solche Ausgangsstoffe wählt, dass die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Alanin, $R_2$ Wasserstoff, $R_3$ Cyclohexylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze gebildet werden.

13. Verfahren gemäss Anspruch 9, dadurch gekennzeichnet, dass man solche Ausgangsstoffe wählt, dass die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cycloheptylmethyl, $R_4$ Hydroxy, $R_5$ Isopropyl und $R_6$ n-Butylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze gebildet werden.

14. Verfahren gemäss Anspruch 9, dadurch gekennzeichnet, dass man solche Ausgangsstoffe wählt, dass die Verbindung der Formel I, worin $R_1$ einen Rest der Teilformel Ia, worin $R^a$ tert-Butyl, $R^b$ Phenyl, m 2, n 1, p 0 und q 1 bedeuten, A den bivalenten Rest der Aminosäure L-Histidin, $R_2$ Wasserstoff, $R_3$ Cyclohexylmethyl, $R_4$ Hydroxy, $R_5$ Isopropyl und $R_6$ Methylamino bedeuten und die die Reste $R_3$, $R_4$ und $R_5$ tragenden C-Atome und das Methin-C-Atom der Teilformel Ia die S-Konfiguration aufweisen, und deren pharmazeutisch annehmbare Salze gebildet werden.

15. Verfahren zur Herstellung von pharmazeutischen Präparaten, dadurch gekennzeichnet, dass man Verbindungen der Formel I oder pharmazeutisch verwendbare Salze von Verbindungen der Formel I mit salzbildenden Gruppen mit einem pharmazeutischen Trägermaterial mischt.

FO 7.4/KB/cc*